# EUROPEAN PATENT APPLICATION

(11) **EP 4 737 454 A1**
(43) Date of publication of application: **06.05.2026**
(21) Application number: 24830883.5
(22) Date of filing: 27.06.2024
(51) Int. Cl.: C07D 471/04, A61P 3/04, A61P 3/06, A61P 3/10, C07D 519/00, A61K 31/437, A61K 31/444, A61K 31/4725

(54) **GLP-1 RECEPTOR AGONIST, AND PREPARATION METHOD THEREFOR AND USE THEREOF**

(30) Priority: 29.06.2023 CN 202310790587; 26.07.2023 CN 202310926049; 19.02.2024 CN 202410185415
(71) Applicant: Wayne Biotechnology Co., Ltd., Shanghai 201210 (CN)
(72) Inventor: FENG, Jianbo, Shanghai 201210 (CN); YAO, Yuanshan, Shanghai 201210 (CN); CHEN, Jiayu, Shanghai 201210 (CN)
(74) Representative: dompatent
(86) International application number: PCT/CN2024/101972
(87) International publication number: WO 2025/002250

(57) **Abstract**

Disclosed in the present invention are a GLP-1 receptor agonist, and a preparation method therefor and a use thereof. Specifically, disclosed are a compound represented by formula (I), and a use thereof in preparation of a drug for treating or preventing GLP-1 receptor-mediated diseases.

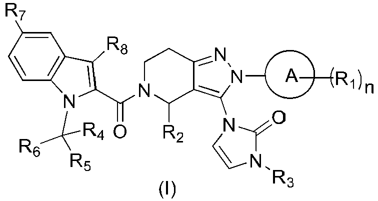

## Description

### TECHNICAL FIELD

The present invention relates to the pharmaceutical field, specifically to a GLP-1 receptor agonist compound and preparation method thereof, as well as use of thr compound in the preparation of a medicament for treating or preventing GLP-1 receptor mediated disease.

### BACKGROUND

Obesity is a prevalent chronic disease in modern society and is associated with many medical problems, including hypertension, hypercholesterolemia, and coronary heart disease. Currently, the only treatment that eliminates obesity with high efficacy is weight reduction surgery, but this treatment is expensive and carries high risks. Pharmacological interventions are generally less effective and associated with side effects. Therefore, there is a clear need for more effective pharmacological interventions with fewer side effects and convenient administration.

Glucagon-like peptide-1 (GLP-1) is an incretin secreted by intestinal epithelial L cells that exerts physiological effects by binding to its receptor. The GLP-1 receptor (GLP-1R) belongs to the G protein-coupled receptor subfamily, and when GLP-1 binds to the GLP-1 receptor, it triggers a series of biological effects. Studies have shown that GLP-1 promotes insulin secretion in a glucose-dependent manner, meaning that when blood glucose concentration rises in the human body, GLP-1 stimulates islet cells, increases insulin secretion, and lowers blood glucose. GLP-1 receptor agonists are a new type of hypoglycemic drug that can effectively control blood glucose levels without causing hypoglycemia, and can effectively reduce weight by increasing satiety, delaying gastric emptying, suppressing appetite, slowing intestinal movement to delay food absorption, and reducing fat accumulation, thereby achieving weight loss.

Peptide-based GLP-1 receptor agonist drugs such as liraglutide, exenatide, and semaglutide have been applied to obese type II diabetic patients as well as patients with simple obesity or overweight, all showing significant weight reduction effects, but are often accompanied by gastrointestinal adverse reactions such as nausea and vomiting. At the same time, injectable peptide products have poor patient compliance, while oral peptide drugs have low bioavailability, strict medication regimens, high prices, and still relatively high rates of gastrointestinal reactions. Therefore, it is necessary to develop a new compound with good biological properties, good compliance, and safety.

### SUMMARY OF THE INVENTION

The purpose of the present invention is to provide a new GLP-1 receptor agonist and preparation method therefor, pharmaceutical compositions containing it, and use thereof in medicaments, particularly for the preparation of a medicament for treating or preventing GLP-1 receptor mediated disease, with the potential to be developed into a new generation of GLP-1 receptor agonist. Specifically, the present invention provides the following technical solutions.

One aspect of the present invention provides a compound of formula (I), a stereoisomer, tautomer, or pharmaceutically acceptable salt thereof, 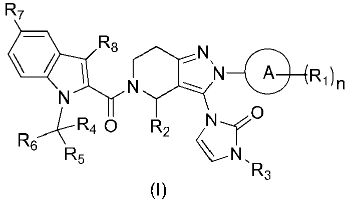
wherein, ring A is selected from a group consisting of 3-12 membered heterocyclyl, C₆₋₁₀ aryl, and 5-10 membered heteroaryl;
R₁ is selected from a group consisting of cyano, halogen, C₁₋₁₀ alkyl, C₃₋₁₂ cycloalkyl, 3-12 membered heterocyclyl, halogenated C₁₋₁₀ alkyl, halogenated C₃₋₁₂ cycloalkyl, halogenated 3-12 membered heterocyclyl, halogenated C₁₋₁₀ alkoxy, halogenated C₁₋₁₀ alkylthio, C₁₋₆ alkyl-substituted C₃₋₁₂ cycloalkyl, and C₁₋₆ alkyl-substituted 3-12 membered heterocyclyl;
R₂ is selected from a group consisting of hydrogen, cyano, C₁₋₁₀ alkyl, halogenated C₁₋₁₀ alkyl, C₁₋₁₀ alkoxy, halogenated C₁₋₁₀ alkoxy, and -NR₁₂R₁₃;
R₃ is selected from a group consisting of C₃₋₁₂ cycloalkyl, 3-12 membered heterocyclyl, C₆₋₁₀ aryl, and 5-10 membered heteroaryl, above groups are optionally further substituted with one or more substituents selected from a group consisting of halogen, cyano, nitro, azido, C₁₋₁₀ alkyl, halogenated C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, -SF₅, -S(O)ᵣR₉, -O-R₁₀, -C(O)OR₁₀, -C(O)R₁₁, -O-C(O)R₁₁, -NR₁₂R₁₃, -C(=NR₁₂)R₁₁, -N(R₁₂)-C(=NR₁₃)R₁₁, -C(O)NR₁₂R₁₃, and -N(R₁₂)-C(O)R₁₁;
R₄ and R₅ are each independently selected from a group consisting of hydrogen, halogen, hydroxyl, C₁₋₁₀ alkyl, halogenated C₁₋₁₀ alkyl, C₁₋₁₀ alkoxy, and halogenated C₁₋₁₀ alkoxy, or R₄ and R₅, together with a carbon atom to which they are directly attached, form a C₃₋₁₂ cycloalkyl, wherein said C₃₋₁₂ cycloalkyl is optionally further substituted with one or more substituents selected from a group consisting of hydrogen, halogen, amino, hydroxyl, C₁₋₁₀ alkyl, halogenated C₁₋₁₀ alkyl, C₁₋₁₀ alkoxy, and halogenated C₁₋₁₀ alkoxy;
R₆ is 5-10 membered heteroaryl;
R₇ is selected from 3-12 membered heterocyclyl, or 5-10 membered heteroaryl, wherein above groups are optionally substituted with one or more substituents selected from a group consisting of halogen, hydroxyl, cyano, nitro, C₁₋₁₀ alkyl, halogenated C₁₋₁₀ alkyl, C₁₋₁₀ alkoxy, halogenated C₁₋₁₀ alkoxy, and -NR₁₂R₁₃, or any two substituents on the same carbon atom of said 3-12 membered heterocyclyl together with a carbon atom to which they are attached form a C₃₋₆ cycloalkyl, wherein said C₃₋₆ cycloalkyl is optionally further substituted with one or more substituents selected from a group consisting of halogen, cyano, C₁₋₆ alkyl, halogenated C₁₋₆ alkyl, and C₁₋₆ alkoxy;
R₈ is selected from a group consisting of hydrogen, cyano, C₃₋₁₂ cycloalkyl, 3-12 membered heterocyclyl, C₁₋₁₀ alkyl, halogenated C₁₋₁₀ alkyl, C₁₋₁₀ alkoxy, halogenated C₁₋₁₀ alkoxy, and - NR₁₂R₁₃;
each R₉ is independently selected from a group consisting of hydrogen, deuterium, hydroxyl, C₁₋₁₀ alkyl, C₂₋₁₀ alkenyl, C₃₋₁₂ cycloalkyl, 3-12 membered heterocyclyl, C₆₋₁₀ aryl, 5-10 membered heteroaryl, and -NR₁₂R₁₃, wherein above groups are independently optionally further substituted with one or more substituents selected from a group consisting of deuterium, halogen, hydroxyl, oxo, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy, and -NR₁₂R₁₃;
each R₁₀ is independently selected from a group consisting of hydrogen, deuterium, C₁₋₁₀ alkyl, C₂₋₁₀ alkenyl, C₃₋₁₂ cycloalkyl, 3-12 membered heterocyclyl, C₆₋₁₀ aryl, and 5-10 membered heteroaryl, wherein above groups are independently optionally further substituted with one or more substituents selected from a group consisting of deuterium, halogen, hydroxyl, oxo, cyano, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy, and -NR₁₂R₁₃;
each R₁₁ is selected from a group consisting of hydrogen, deuterium, hydroxyl, C₁₋₁₀ alkyl, C₁₋₁₀ alkoxy, C₂₋₁₀ alkenyl, C₂₋₁₀ alkynyl, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy, and -NR₁₂R₁₃, wherein above groups are independently optionally further substituted with one or more substituents selected from a group consisting of deuterium, halogen, hydroxyl, cyano, C₁₋₁₀ alkyl, halogenated C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy, and -NR₁₂R₁₃;
each R₁₂ and each R₁₃ are independently selected from a group consisting of hydrogen, deuterium, hydroxyl, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, sulfinyl, sulfonyl, methanesulfonyl, isopropylsulfonyl, cyclopropylsulfonyl, p-toluenesulfonyl, aminosulfonyl, dimethylaminosulfonyl, amino, mono-C₁₋₁₀ alkylamino, di-C₁₋₁₀ alkylamino, and C₁₋₁₀ acyl, wherein above groups are independently optionally further substituted with one or more substituents selected from a group consisting of deuterium, halogen, hydroxyl, C₁₋₁₀ alkyl, C₂₋₁₀ alkenyl, C₂₋₁₀ alkynyl, halogenated C₁₋₁₀ alkyl, deuterium-substituted C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy, amino, mono-C₁₋₁₀ alkylamino, di-C₁₋₁₀ alkylamino, and C₁₋₁₀ acyl;
or, R₁₂ and R₁₃, together with a nitrogen atom to which they are directly attached, form a 4-10 membered heterocyclyl or 4-10 membered heteroaryl, wherein said 4-10 membered heterocyclyl or 4-10 membered heteroaryl is optionally further substituted with one or more substituents selected from a group consisting of deuterium, halogen, hydroxyl, C₁₋₁₀ alkyl, C₂₋₁₀ alkenyl, C₂₋₁₀ alkynyl, halogenated C₁₋₁₀ alkyl, deuterium-substituted C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy, amino, mono-C₁₋₁₀ alkylamino, di-C₁₋₁₀ alkylamino, and C₁₋₁₀ acyl;
each r is independently 0, 1, or 2;
n is 0, 1, 2, 3, or 4.

The present solution satisfies any one of the following conditions:
a. When A is selected from C₆₋₁₀ aryl and 5-10 membered heteroaryl, n is not equal to 0, at least one R₁ is selected from a group consisting of cyano, C₃₋₁₂ cycloalkyl, 3-12 membered heterocyclyl, halogenated C₃₋₁₂ cycloalkyl, halogenated 3-12 membered heterocyclyl, halogenated C₁₋₁₀ alkoxy, C₁₋₆ alkyl-substituted C₃₋₁₂ cycloalkyl, and C₁₋₆ alkyl-substituted 3-12 membered heterocyclyl,
b. When A is selected from C₆₋₁₀ aryl and 5-10 membered heteroaryl, n is not equal to 0, and R₁ is only selected from a group consisting of halogen and/or C₁₋₁₀ alkyl and/or halogenated C₁₋₁₀ alkyl, and R₃ is selected from C₆₋₈ aryl and 5-6 membered heteroaryl, wherein above groups are substituted with at least one substituent selected from a group consisting of cyano, -NR₁₂R₁₃, - C(O)NR₁₂R₁₃, and -N(R₁₂)-C(O)R₁₁, wherein in said -NR₁₂R₁₃ and -C(O)NR₁₂R₁₃, R₁₂ or R₁₃ are each independently selected from a group consisting of C₁₋₆ alkyl and C₃₋₁₂ cycloalkyl, wherein in said -N(R₁₂)-C(O)R₁₁, R₁₁ or R₁₂ is selected from C₃₋₁₂ cycloalkyl, wherein said C₃₋₁₂ cycloalkyl is independently optionally further substituted with one or more substituents selected from a group consisting of deuterium, halogen, hydroxyl, cyano, C₁₋₁₀ alkyl, halogenated C₁₋₁₀ alkyl, and C₁₋₁₀ alkoxy;

As a preferred embodiment, ring A is selected from a group consisting of 3-6 membered heterocyclyl, C₆₋₈ aryl, and 5-8 membered heteroaryl;
R₁ is selected from a group consisting of cyano, halogen, C₁₋₆ alkyl, C₃₋₆ cycloalkyl, 3-6 membered heterocyclyl, halogenated C₁₋₆ alkyl, halogenated C₃₋₆ cycloalkyl, halogenated 3-6 membered heterocyclyl, halogenated C₁₋₆ alkoxy, halogenated C₁₋₆ alkylthio, C₁₋₆ alkyl-substituted C₃₋₆ cycloalkyl, and C₁₋₆ alkyl-substituted 3-6 membered heterocyclyl;
R₂ is selected from a group consisting of hydrogen, C₁₋₆ alkyl, halogenated C₁₋₆ alkyl, C₁₋₆ alkoxy, and halogenated C₁₋₆ alkoxy;
R₃ is selected from 5-10 membered heteroaryl and C₆₋₁₀ aryl, wherein said groups are optionally further substituted with one or more substituents selected from a group consisting of halogen, cyano, nitro, hydroxyl, C₁₋₆ alkyl, halogenated C₁₋₆ alkyl, C₃₋₆ cycloalkyl, C₁₋₆ alkoxy, halogenated C₁₋₆ alkoxy, and -S(O)ᵣR₉, -C(O)NR₁₂R₁₃, -N(R₁₂)-C(O)R₁₁, and -NR₁₂R₁₃;
R₄ and R₅, together with a carbon atom to which they are directly attached, form a C₃₋₆ cycloalkyl, wherein said C₃₋₆ cycloalkyl is optionally further substituted with one or more substituents selected from a group consisting of hydrogen, halogen, amino, hydroxyl, C₁₋₆ alkyl, and halogenated C₁₋₆ alkyl;
R₆ is 5-6 membered heteroaryl;
R₇ is 3-6 membered heterocyclyl, wherein said 3-6 membered heterocyclyl is optionally substituted with one or more substituents selected from a group consisting of halogen, hydroxyl, cyano, nitro, C₁₋₆ alkyl and halogenated C₁₋₆ alkyl, or any two substituents on said 3-6 membered heterocyclyl connected to the same carbon atom together with a carbon atom to which they are attached form a C₃₋₆ cycloalkyl, wherein said C₃₋₆ cycloalkyl is optionally further substituted with one or more substituents selected from a group consisting of halogen, cyano, C₁₋₆ alkyl, halogenated C₁₋₆ alkyl and C₁₋₆ alkoxy;
R₈ is selected from a group consisting of hydrogen, cyano, C₃₋₆ cycloalkyl, 3-6 membered heterocyclyl, C₁₋₆ alkyl, halogenated C₁₋₆ alkyl, C₁₋₆ alkoxy, halogenated C₁₋₆ alkoxy and -NR₁₂R₁₃;
R₁₁, R₁₂ and R₁₃ are each independently selected from a group consisting of hydrogen, deuterium, C₁₋₆ alkyl and C₃₋₆ cycloalkyl, wherein said C₃₋₆ cycloalkyl is optionally further substituted with one or more substituents selected from a group consisting of halogen, halogenated C₁₋₆ alkyl and C₁₋₆ alkyl.

The embodiment satisfies any one of the following conditions:
a. when A is selected from C₆₋₈ aryl and 5-8 membered heteroaryl, n is not equal to 0, at least one R₁ is selected from a group consisting of cyano, C₃₋₆ cycloalkyl, 3-6 membered heterocyclyl, halogenated C₃₋₆ cycloalkyl, halogenated 3-6 membered heterocyclyl, halogenated C₁₋₆ alkoxy, C₁₋₆ alkyl-substituted C₃₋₆ cycloalkyl and C₁₋₆ alkyl-substituted 3-6 membered heterocyclyl;
b. when A is selected from C₆₋₈ aryl and 5-8 membered heteroaryl, n is not equal to 0, and R₁ is only selected from a group consisting of halogen and/or C₁₋₆ alkyl and/or halogenated C₁₋₆ alkyl, and R₃ is selected from a group consisting of C₆₋₈ aryl and 5-6 membered heteroaryl, the above groups are substituted with at least one substituent selected from a group consisting of cyano, - NR₁₂R₁₃, -C(O)NR₁₂R₁₃ and -N(R₁₂)-C(O)R₁₁, wherein in said -NR₁₂R₁₃ and -C(O)NR₁₂R₁₃, R₁₂ or R₁₃ are each independently selected from a group consisting of C₁₋₆ alkyl, C₃₋₁₂ cycloalkyl, wherein in said -N(R₁₂)-C(O)R₁₁, R₁₁ or R₁₂ is C₃₋₆ cycloalkyl, wherein said C₃₋₆ cycloalkyl is independently optionally further substituted with one or more substituents selected from a group consisting of deuterium, halogen, halogenated C₁₋₆ alkyl and C₁₋₆ alkyl;
wherein "R₁ is only selected from a group consisting of halogen and/or C₁₋₆ alkyl and/or halogenated C₁₋₆ alkyl" means that R₁ is only selected from any one of halogen, C₁₋₆ alkyl, and halogenated C₁₋₆ alkyl, or R₁ is only selected from more of halogen, C₁₋₆ alkyl, and halogenated C₁₋₆ alkyl, and R₁ is not selected from other groups except halogen, C₁₋₆ alkyl, and halogenated C₁₋₆ alkyl.

In a preferred embodiment, R₂ is selected from methyl, ethyl and isopropyl; R₄ and R₅ together with a carbon atom to which they are directly attached form a cyclopropyl or cyclobutyl, wherein said cyclopropyl or cyclobutyl is optionally further substituted with one or more substituents selected from methyl, ethyl and isopropyl;
R₆ is

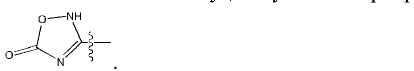

In a preferred embodiment, ring A is selected from a group consisting of phenyl, pyridinyl, pyrimidinyl.

In a preferred embodiment, it is selected from the following compound: 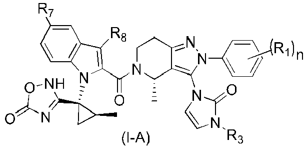
R₃ is selected from 5-10 membered heteroaryl and C₆₋₁₀ aryl, wherein the above groups are optionally further substituted with one or more substituents selected from a group consisting of halogen, cyano, nitro, hydroxyl, C₁₋₆ alkyl, halogenated C₁₋₆ alkyl, C₃₋₆ cycloalkyl, C₁₋₆ alkoxy, halogenated C₁₋₆ alkoxy, -S(O)ᵣR₉, -C(O)NR₁₂R₁₃, -N(R₁₂)-C(O)R₁₁ and -NR₁₂R₁₃;
R₁₁, R₁₂ and R₁₃ are each independently selected from a group consisting of hydrogen, deuterium, C₁₋₆ alkyl and C₃₋₆ cycloalkyl, wherein said C₃₋₆ cycloalkyl is optionally further substituted with one or more substituents selected from a group consisting of halogen, halogenated C₁₋₆ alkyl and C₁₋₆ alkyl;
n is 1, 2, 3 or 4;
R₁, R₇ and R₈ are as defined above;
the embodiment satisfies any one of the following conditions:
   a. at least one R₁ is selected from a group consisting of cyano, C₃₋₆ cycloalkyl, 3-6 membered heterocyclyl, halogenated C₃₋₆ cycloalkyl, halogenated 3-6 membered heterocyclyl, halogenated C₁₋₆ alkoxy, C₁₋₆ alkyl-substituted C₃₋₆ cycloalkyl and C₁₋₆ alkyl-substituted 3-6 membered heterocyclyl;
   b. when R₁ is only selected from a group consisting of halogen and/or C₁₋₆ alkyl and/or halogenated C₁₋₆ alkyl, and R₃ is selected from C₆₋₈ aryl and 5-6 membered heteroaryl, the above groups are substituted with at least one substituent selected from a group consisting of cyano, - NR₁₂R₁₃, -C(O)NR₁₂R₁₃ and -N(R₁₂)-C(O)R₁₁, wherein in said -NR₁₂R₁₃ and -C(O)NR₁₂R₁₃, R₁₂ or R₁₃ are each independently selected from C₁₋₆ alkyl, and C₃₋₆ cycloalkyl, wherein in said - N(R₁₂)-C(O)R₁₁, R₁₁ or R₁₂ is C₃₋₆ cycloalkyl, wherein said C₃₋₆ cycloalkyl is independently optionally further substituted with one or more substituents selected from a group consisting of deuterium, halogen, halogenated C₁₋₆ alkyl and C₁₋₆ alkyl.

In a preferred embodiment, R₃ is selected from a group consisting of phenyl, pyridinyl, pyrimidinyl, pyrazinyl, pyridazinyl or the following groups: 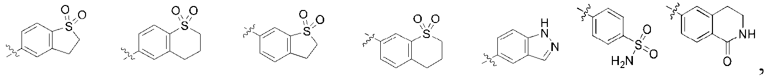
wherein the above groups are optionally further substituted with one or more substituents selected from a group consisting of cyano, nitro, hydroxyl, halogen, C₁₋₆ alkyl, halogenated C₁₆ alkyl, C₃₋₆ cycloalkyl, C₁₋₆ alkoxy, halogenated C₁₋₆ alkoxy, -C(O)NR₁₂R₁₃, -N(R₁₂)-C(O)R₁₁ and -NR₁₂R₁₃;
R₁₁, R₁₂ and R₁₃ are as defined above.

In a preferred embodiment, it is selected from the following compound: 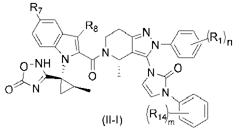
n is 1, 2, 3 or 4; m is 0, 1, 2, 3, 4, 5 or 6;
R₁₄ is selected from a group consisting of hydrogen, cyano, hydroxyl, nitro, halogen, C₁₋₆ alkyl, halogenated C₁₋₆ alkyl, C₃₋₆ cycloalkyl, C₁₋₆ alkoxy, halogenated C₁₋₆ alkoxy, -C(O)NR₁₂R₁₃, -N(R₁₂)-C(O)R₁₁ and -NR₁₂R₁₃;
R₁₁, R₁₂ and R₁₃ are each independently selected from a group consisting of hydrogen, deuterium, C₁₋₆ alkyl and C₃₋₆ cycloalkyl, wherein said C₃₋₆ cycloalkyl is optionally further substituted with one or more substituents selected from a group consisting of halogen, halogenated C₁₋₆ alkyl and C₁₋₆ alkyl;
R₁, R₇ and R₈ are as defined above;
the embodiment of formula (II-I) satisfies any one of the following conditions:
   a. at least one R₁ is selected from a group consisting of cyano, C₃₋₆ cycloalkyl, 3-6 membered heterocyclyl, halogenated C₃₋₆ cycloalkyl, halogenated 3-6 membered heterocyclyl, halogenated C₁₋₆ alkoxy, C₁₋₆ alkyl-substituted C₃₋₆ cycloalkyl and C₁₋₆ alkyl-substituted 3-6 membered heterocyclyl;
   b. R₁ is only selected from a group consisting of halogen and/or C₁₋₆ alkyl and/or halogenated C₁₋₆ alkyl, at least one R₁₄ is selected from a group consisting of cyano, -NR₁₂R₁₃, -C(O)NR₁₂R₁₃, and -N(R₁₂)-C(O)R₁₁, wherein in said -NR₁₂R₁₃ and -C(O)NR₁₂R₁₃, R₁₂ or R₁₃ is each independently selected from C₁₋₆ alkyl and C₃₋₆ cycloalkyl, wherein in said -N(R₁₂)-C(O)R₁₁, R₁₁ or R₁₂ is C₃₋₆ cycloalkyl, wherein said C₃₋₆ cycloalkyl is independently optionally further substituted by one or more substituents selected from a group consisting of deuterium, halogen, halogenated C₁₋₆ alkyl, and C₁₋₆ alkyl.

As a preferred embodiment, R₇ is 5-6 membered heterocyclyl, wherein the 5-6 membered heterocyclyl is optionally substituted by one or more substituents selected from a group consisting of halogen, hydroxy, cyano, nitro, C₁₋₆ alkyl, and halogenated C₁₋₆ alkyl, or any two substituents on said 3-6 membered heterocyclyl connected to the same carbon atom together with a carbon atom to which they are attached form a C₃₋₆ cycloalkyl, wherein said C₃₋₆ cycloalkyl is optionally further substituted by one or more substituents selected from a group consisting of halogen, cyano, C₁₋₆ alkyl, halogenated C₁₋₆ alkyl, and C₁₋₆ alkoxy.

As a preferred embodiment, R₇ is tetrahydropyranyl, wherein said tetrahydropyranyl is optionally substituted by one or more substituents selected from a group consisting of methyl, ethyl, isopropyl, fluoromethyl, difluoromethyl, and trifluoromethyl, or any two substituents on the tetrahydropyranyl connected to the same carbon atom together with the carbon atom to which they are attached form a cyclopropyl, cyclobutyl, and cyclopentyl, wherein the formed ring is optionally further substituted by one or more substituents selected from a group consisting of fluoro, chloro, bromo, cyano, methyl, ethyl, isopropyl, fluoromethyl, difluoromethyl, trifluoromethyl, methoxy, and ethoxy.

As a preferred embodiment, it is selected from the following compound: 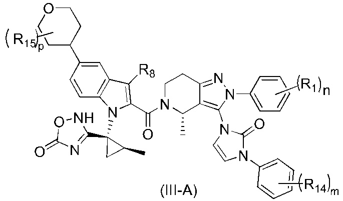
R₁₄ is selected from a group consisting of hydrogen, cyano, hydroxy, nitro, halogen, C₁₋₆ alkyl, halogenated C₁₋₆ alkyl, C₃₋₆ cycloalkyl, C₁₋₆ alkoxy, halogenated C₁₋₆ alkoxy, -C(O)NR₁₂R₁₃, - N(R₁₂)-C(O)R₁₁, and -NR₁₂R₁₃;
R₁₁, R₁₂, and R₁₃ are each independently selected from a group consisting of hydrogen, deuterium, C₁₋₆ alkyl, and C₃₋₆ cycloalkyl, wherein said C₃₋₆ cycloalkyl is optionally further substituted by one or more substituents selected from a group consisting of halogen, halogenated C₁₋₆ alkyl, and C₁₋₆ alkyl;
R₁₅ is selected from C₁₋₆ alkyl and halogenated C₁₋₆ alkyl, or any two R₁₅ connected to the same carbon atom together with a carbon atom to which they are attached form a C₃₋₆ cycloalkyl;
p is 1, 2, 3, or 4;
m is 1, 2, 3, or 4;
n, R₁, and R₈ are as defined above.

The compounds of the embodiment need to satisfy any one of the following conditions:
a. At least one R₁ is selected from a group consisting of cyano, C₃₋₆ cycloalkyl, 3-6 membered heterocyclyl, halogenated C₃₋₆ cycloalkyl, halogenated 3-6 membered heterocyclyl, halogenated C₁₋₆ alkoxy, C₁₋₆ alkyl-substituted C₃₋₆ cycloalkyl, and C₁₋₆ alkyl-substituted 3-6 membered heterocyclyl;
b. R₁ is only selected from a group consisting of halogen and/or C₁₋₆ alkyl and/or halogenated C₁₋₆ alkyl, at least one R₁₄ is selected from a group consisting of cyano, -NR₁₂R₁₃, -C(O)NR₁₂R₁₃, and -N(R₁₂)-C(O)R₁₁, wherein in said -NR₁₂R₁₃ and -C(O)NR₁₂R₁₃, R₁₂ or R₁₃ is each independently selected from C₁₋₆ alkyl and C₃₋₆ cycloalkyl, wherein in said -N(R₁₂)-C(O)R₁₁, R₁₁ or R₁₂ is C₃₋₆ cycloalkyl, wherein said C₃₋₆ cycloalkyl is independently optionally further substituted by one or more substituents selected from a group consisting of deuterium, halogen, halogenated C₁₋₆ alkyl, and C₁₋₆ alkyl.

As a preferred embodiment, it is selected from the following compounds: 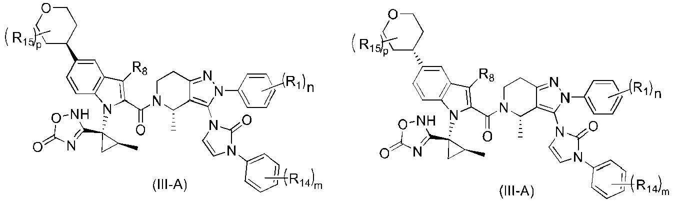
R₁, R₈, R₁₄, R₁₅, p, m, and n are as defined above;
the compounds of the embodiment need to satisfy any one of the following conditions:
   a. At least one R₁ is selected from a group consisting of cyano, C₃₋₆ cycloalkyl, 3-6 membered heterocyclyl, halogenated C₃₋₆ cycloalkyl, halogenated 3-6 membered heterocyclyl, halogenated C₁₋₆ alkoxy, C₁₋₆ alkyl-substituted C₃₋₆ cycloalkyl, and C₁₋₆ alkyl-substituted 3-6 membered heterocyclyl;
   b. R₁ is only selected from halogen and/or C₁₋₆ alkyl and/or halogenated C₁₋₆ alkyl, at least one R₁₄ is selected from a group consisting of cyano, -NR₁₂R₁₃, -C(O)NR₁₂R₁₃, and -N(R₁₂)-C(O)R₁₁, wherein in said -NR₁₂R₁₃ and -C(O)NR₁₂R₁₃, R₁₂ or R₁₃ is each independently selected from a group consisting of C₁₋₆ alkyl and C₃₋₆ cycloalkyl, wherein in said -N(R₁₂)-C(O)R₁₁, R₁₁ or R₁₂ is C₃₋₆ cycloalkyl, wherein said C₃₋₆ cycloalkyl is independently optionally further substituted by one or more substituents selected from a group consisting of deuterium, halogen, halogenated C₁₋₆ alkyl, and C₁₋₆ alkyl.
As a preferred embodiment, R₁₅ is selected from a group consisting of hydrogen, methyl, and ethyl, or any two R₁₅ connected to the same carbon atom form a cyclopropyl, cyclobutyl, or cyclopentyl with the carbon atom to which they are attached; R₁₄ is selected from a group consisting of hydrogen, cyano, nitro, hydroxy, fluoro, chloro, bromo, methyl, ethyl, propyl, isopropyl, fluoromethyl, difluoromethyl, trifluoromethyl, methoxy, ethoxy, isopropoxy, fluoromethoxy, difluoromethoxy, trifluoromethoxy, fluoroethoxy, difluoroethoxy, trifluoroethoxy, cyclopropyl, cyclobutyl, cyclopentyl, cyclohexyl, -C(O)NR₁₂R₁₃, -N(R₁₂)-C(O)R₁₁, and -NR₁₂R₁₃;
R₁₁, R₁₂, and R₁₃ are each independently selected from a group consisting of hydrogen, deuterium, methyl, ethyl, propyl, cyclopropyl, cyclobutyl, cyclopentyl, and bicyclo[1.1.1]pentyl, wherein the above groups are optionally substituted by one or more substituents selected from a group consisting of fluoro, chloro, bromo, fluoromethyl, difluoromethyl, trifluoromethyl, methyl, ethyl, propyl, and isopropyl;

As a preferred embodiment, R₁₄ is selected from a group consisting of the following groups:

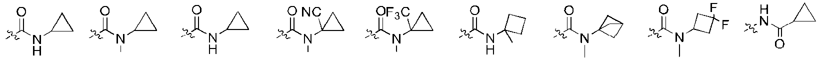

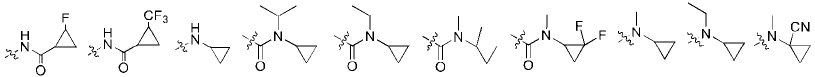

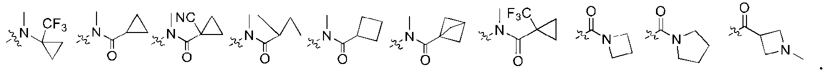

As a preferred embodiment, it is selected from the following compounds: 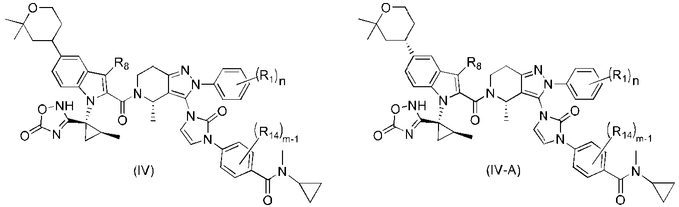
R₁₄ is selected from a group consisting of hydrogen, cyano, hydroxy, nitro, halogen, C₁₋₆ alkyl, halogenated C₁₋₆ alkyl, C₃₋₆ cycloalkyl, C₁₋₆ alkoxy, halogenated C₁₋₆ alkoxy, -C(O)NR₁₂R₁₃, - N(R₁₂)-C(O)R₁₁, and -NR₁₂R₁₃;
m is 1, 2, 3, or 4;
n is 1, 2, 3, or 4;
R₁ and R₈ are as defined above.

The compounds of the embodiment need to satisfy any one of the following conditions:
a. at least one R₁ is selected from a group consisting of cyano, C₃₋₆ cycloalkyl, 3-6 membered heterocyclyl, halogenated C₃₋₆ cycloalkyl, halogenated 3-6 membered heterocyclyl, halogenated C₁₋₆ alkoxy, C₁₋₆ alkyl-substituted C₃₋₆ cycloalkyl, and C₁₋₆ alkyl-substituted 3-6 membered heterocyclyl;
b. R₁ is only selected from halogen and/or C₁₋₆ alkyl and/or halogenated C₁₋₆ alkyl, at least one R₁₄ is selected from a group consisting of cyano, -NR₁₂R₁₃, -C(O)NR₁₂R₁₃, and -N(R₁₂)-C(O)R₁₁, wherein in said -NR₁₂R₁₃ and -C(O)NR₁₂R₁₃, R₁₂ or R₁₃ is each independently selected from C₁₋₆ alkyl and C₃₋₆ cycloalkyl, wherein in said -N(R₁₂)-C(O)R₁₁, R₁₁ or R₁₂ is C₃₋₆ cycloalkyl, wherein said C₃₋₆ cycloalkyl is independently optionally further substituted by one or more substituents selected from a group consisting of deuterium, halogen, halogenated C₁₋₆ alkyl, and C₁₋₆ alkyl.

As a further preferred embodiment, R₁₄ is selected from a group consisting of hydrogen, cyano, nitro, hydroxy, fluoro, chloro, bromo, methyl, ethyl, propyl, isopropyl, fluoromethyl, difluoromethyl, trifluoromethyl, methoxy, ethoxy, isopropoxy, fluoromethoxy, difluoromethoxy, trifluoromethoxy, fluoroethoxy, difluoroethoxy, trifluoroethoxy, cyclopropyl, cyclobutyl, cyclopentyl, cyclohexyl, -C(O)NR₁₂R₁₃, -N(R₁₂)-C(O)R₁₁, and -NR₁₂R₁₃.

As a preferred embodiment, R₁ is selected from a group consisting of cyano, halogen, C₁₋₆ alkyl, halogenated C₁₋₆ alkyl, C₃₋₆ cycloalkyl, 3-6 membered heterocyclyl, halogenated C₃₋₆ cycloalkyl, halogenated 3-6 membered heterocyclyl, halogenated C₁₋₆ alkoxy, halogenated C₁₋₆ alkylthio, C₁₋₆ alkyl-substituted C₃₋₆ cycloalkyl, and C₁₋₆ alkyl-substituted 3-6 membered heterocyclyl; provided that at least one R₁ in said compound is selected from a group consisting of cyano, C₃₋₆ cycloalkyl, 3-6 membered heterocyclyl, halogenated C₃₋₆ cycloalkyl, halogenated 3-6 membered heterocyclyl, halogenated C₁₋₆ alkoxy, C₁₋₆ alkyl-substituted C₃₋₆ cycloalkyl, and C₁₋₆ alkyl-substituted 3-6 membered heterocyclyl.

As a further preferred embodiment, R₁ is selected from a group consisting of cyano, fluoro, chloro, bromo, methyl, ethyl, isopropyl, fluoromethyl, difluoromethyl, trifluoromethyl, fluoromethoxy, difluoromethoxy, trifluoromethoxy, and trifluoromethylthio, or is selected from a group consisting of the following groups: 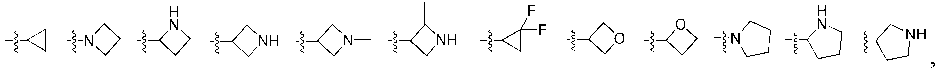 provided that at least one R₁ in said compound is selected from a group consisting of cyano, C₃₋₆ cycloalkyl, 3-6 membered heterocyclyl, halogenated C₃₋₆ cycloalkyl, halogenated 3-6 membered heterocyclyl, halogenated C₁₋₆ alkoxy, C₁₋₆ alkyl-substituted C₃₋₆ cycloalkyl, and C₁₋₆ alkyl-substituted 3-6 membered heterocyclyl.

As a preferred embodiment, 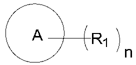 is

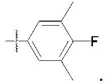

As a preferred embodiment, R₂ is methyl.

As a preferred embodiment, 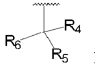 is

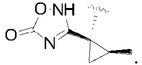

As a preferred embodiment, R₇ is

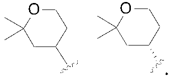

As a preferred embodiment, R₈ is H.

As a preferred embodiment, R₈ is selected from a group consisting of hydrogen, cyano, C₃₋₆ cycloalkyl, 3-6 membered heterocyclyl, C₁₋₆ alkyl, halogenated C₁₋₆ alkyl, halogenated C₁₋₆ alkoxy, and -NR₁₂R₁₃.

As a further preferred embodiment, R₈ is selected from a group consisting of hydrogen, cyano, amino, cyclopropyl, cyclobutyl, cyclopentyl, methyl, ethyl, isopropyl, fluoromethyl, difluoromethyl, trifluoromethyl, fluoromethoxy, difluoromethoxy, trifluoromethoxy, trifluoromethylthio, azacyclobutyl, and methylamino.

As a preferred embodiment, it is selected from the following compounds: 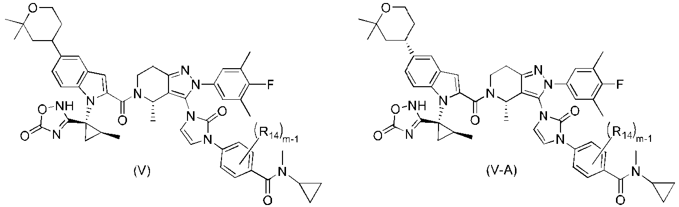
R₁₄ is selected from a group consisting of hydrogen, cyano, hydroxy, nitro, halogen, C₁₋₆ alkyl, halogenated C₁₋₆ alkyl, C₃₋₆ cycloalkyl, C₁₋₆ alkoxy, halogenated C₁₋₆ alkoxy, -C(O)NR₁₂R₁₃, - N(R₁₂)-C(O)R₁₁, and -NR₁₂R₁₃.
m is 1, 2, 3, or 4.

As a further preferred embodiment, R₁₄ is selected from a group consisting of hydrogen, cyano, nitro, hydroxy, fluoro, chloro, bromo, methyl, ethyl, propyl, isopropyl, fluoromethyl, difluoromethyl, trifluoromethyl, methoxy, ethoxy, isopropoxy, fluoromethoxy, difluoromethoxy, trifluoromethoxy, fluoroethoxy, difluoroethoxy, trifluoroethoxy, cyclopropyl, cyclobutyl, cyclopentyl, cyclohexyl, -C(O)NR₁₂R₁₃, -N(R₁₂)-C(O)R₁₁, and -NR₁₂R₁₃.

As a further preferred embodiment, R₁₄ is selected from a group consisting of hydrogen, cyano, fluoro, chloro, bromo, C₁₋₆ alkyl, halogenated C₁₋₆ alkyl, and halogenated C₁₋₆ alkoxy.

As a further preferred embodiment, R₁₄ is selected from a group consisting of hydrogen, cyano, fluoro, chloro, methyl, ethyl, propyl, trifluoromethyl, and trifluoromethoxy.

As a further preferred embodiment, 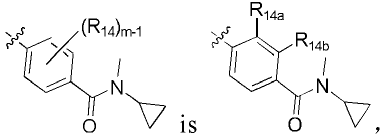 wherein R₁₄ₐ and R_{14b} are each independently selected from a group consisting of hydrogen, cyano, fluoro, chloro, methyl, ethyl, propyl, trifluoromethyl, trifluoroethyl, trifluoromethoxy, trifluoroethoxy, and when R_{14b} is hydrogen, R₁₄ₐ is also hydrogen.

As a further preferred embodiment, R_{14b} is not hydrogen.

As a further preferred embodiment, at least one of R₁₄ₐ and R_{14b} is a halogen-containing group, i.e., at least one of R₁₄ₐ and R_{14b} is fluoro, chloro, trifluoromethyl, or trifluoromethoxy.

As a further preferred embodiment, R₁₄ₐ and R_{14b} are each independently selected from a group consisting of hydrogen, fluoro, and chloro.

As a further preferred embodiment, ring A, R₁, R₂, R₃, R₄, R₅, R₆, R₇, R₈ are each independently the groups corresponding to those in the compounds in the examples.

As a preferred embodiment, it is selected from a group consisting of the following compounds:

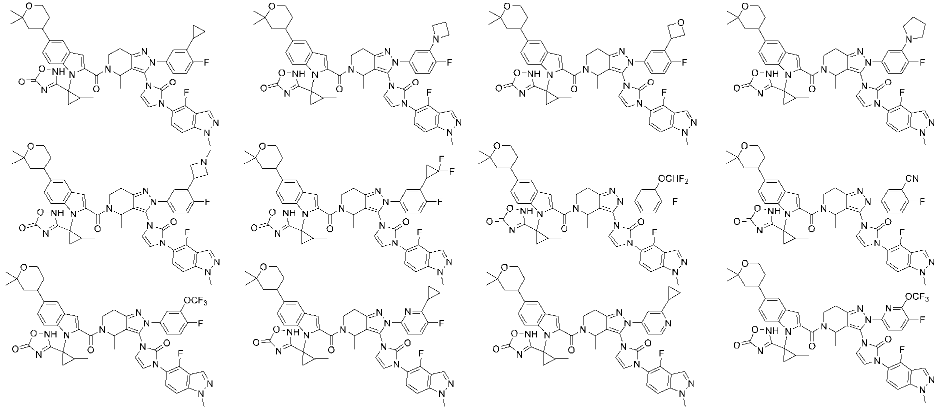

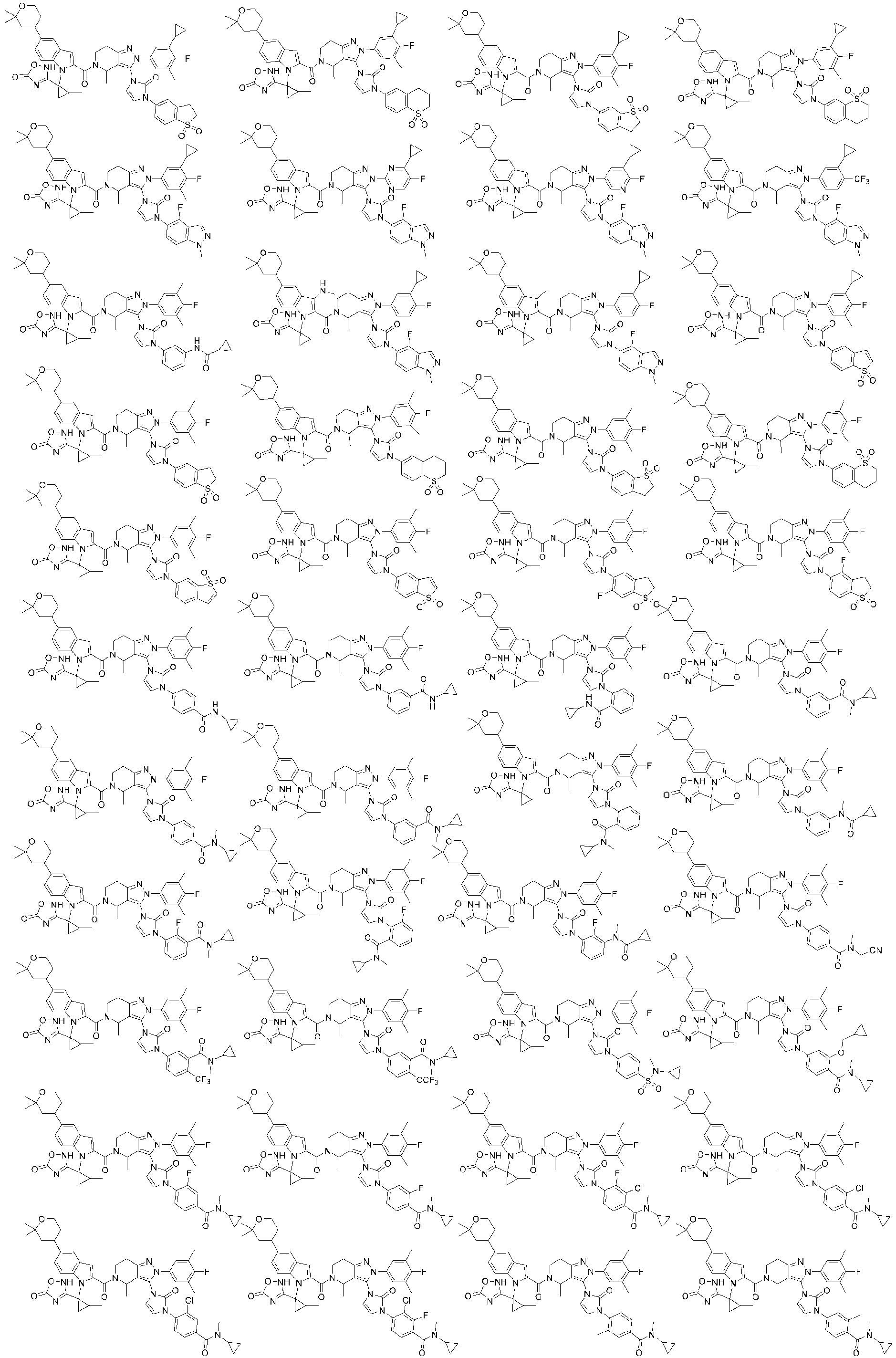

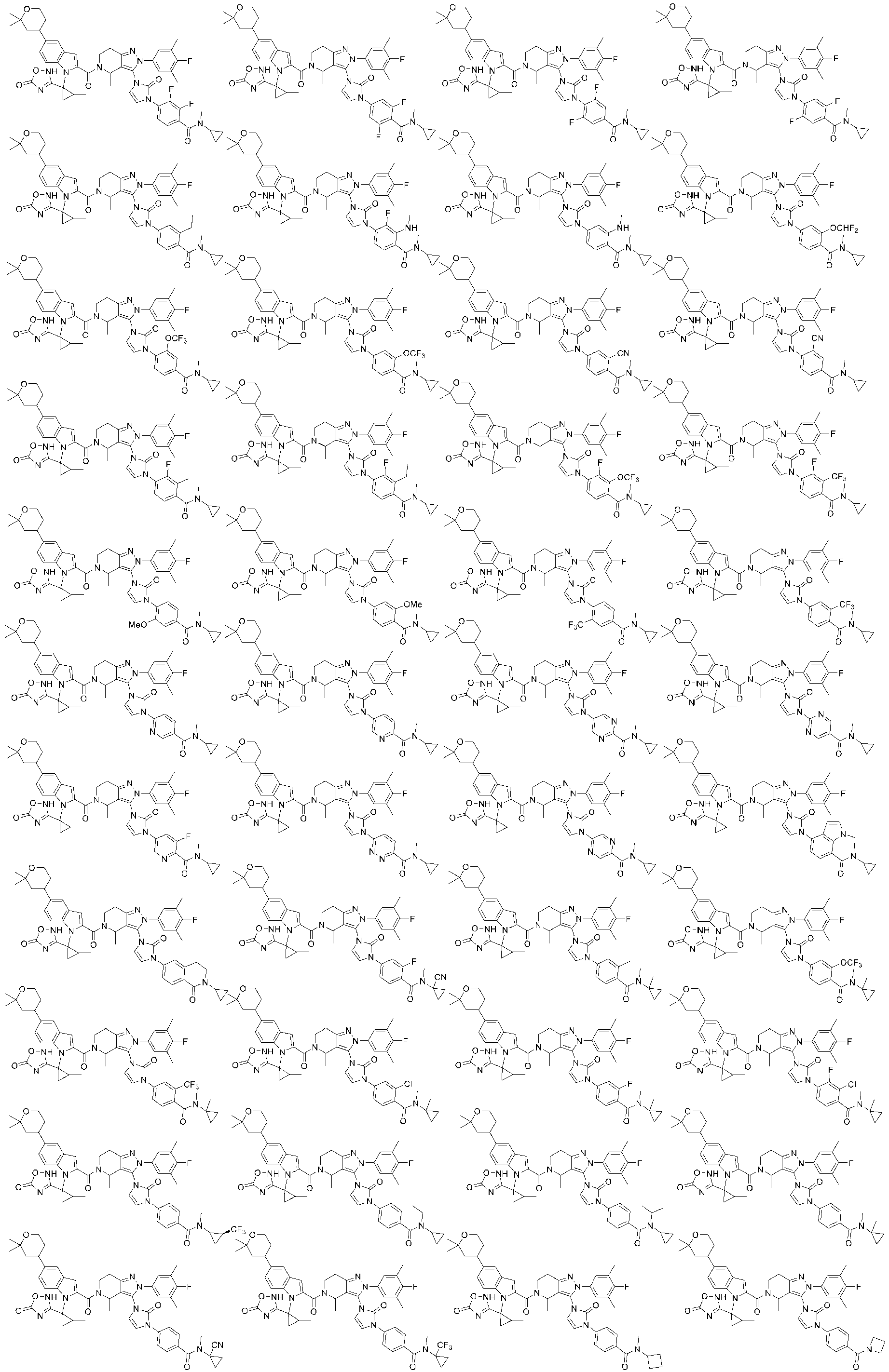

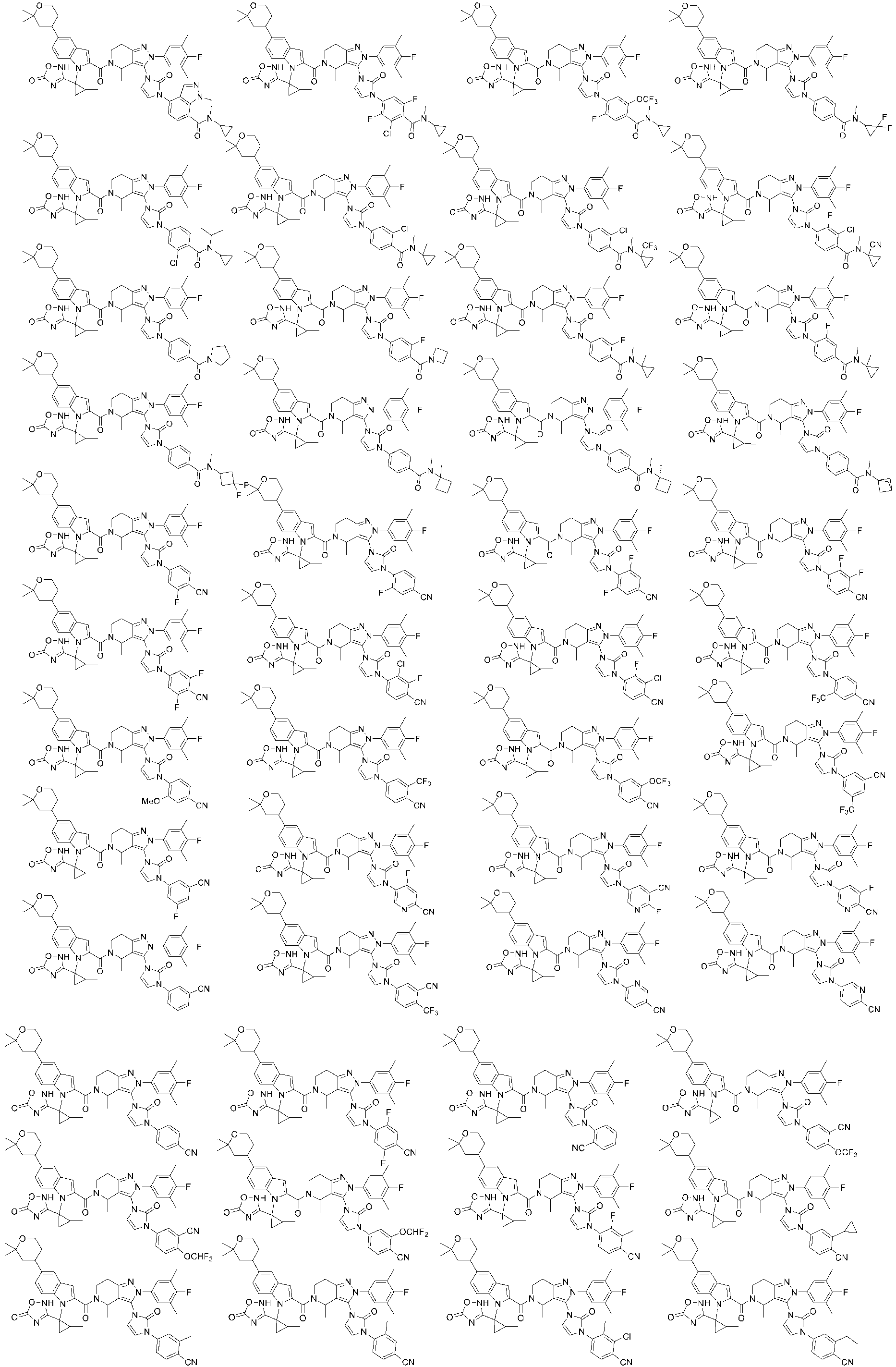

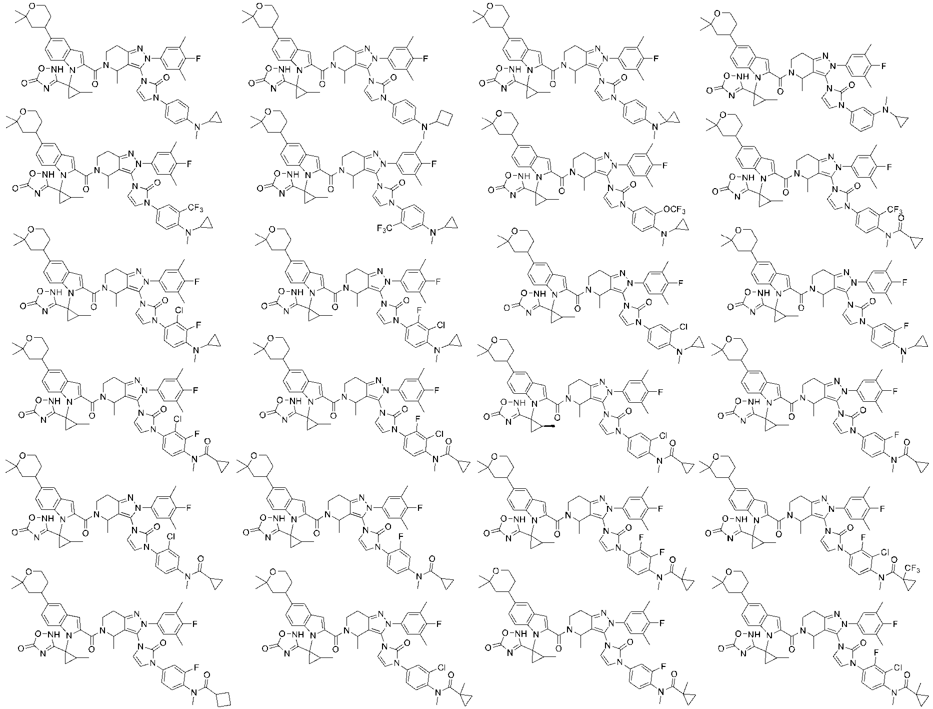

As a preferred embodiment, it is selected from a group consisting of the following compounds:

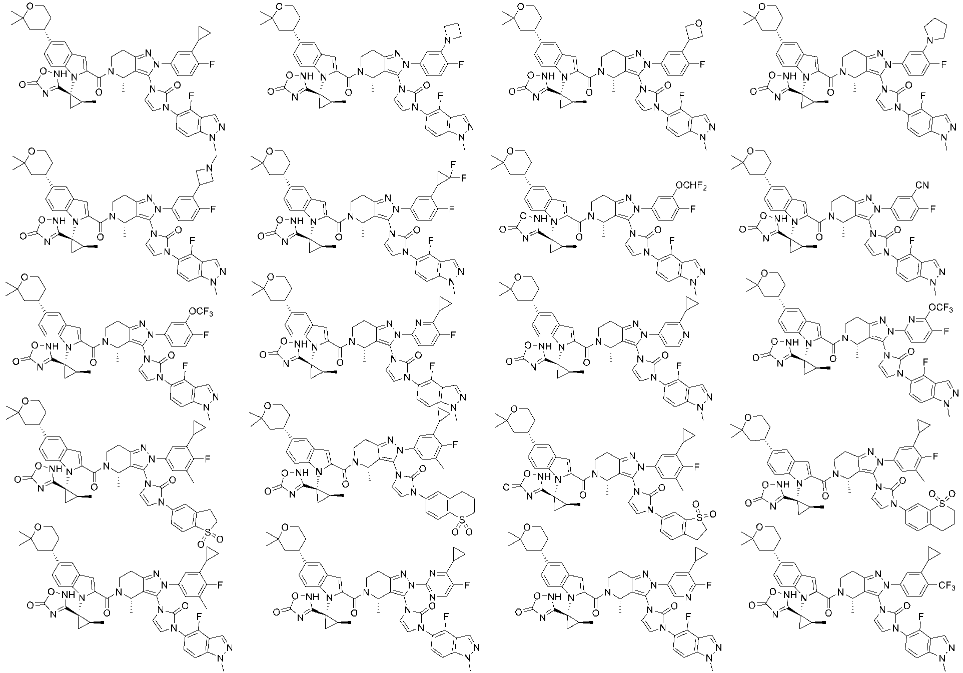

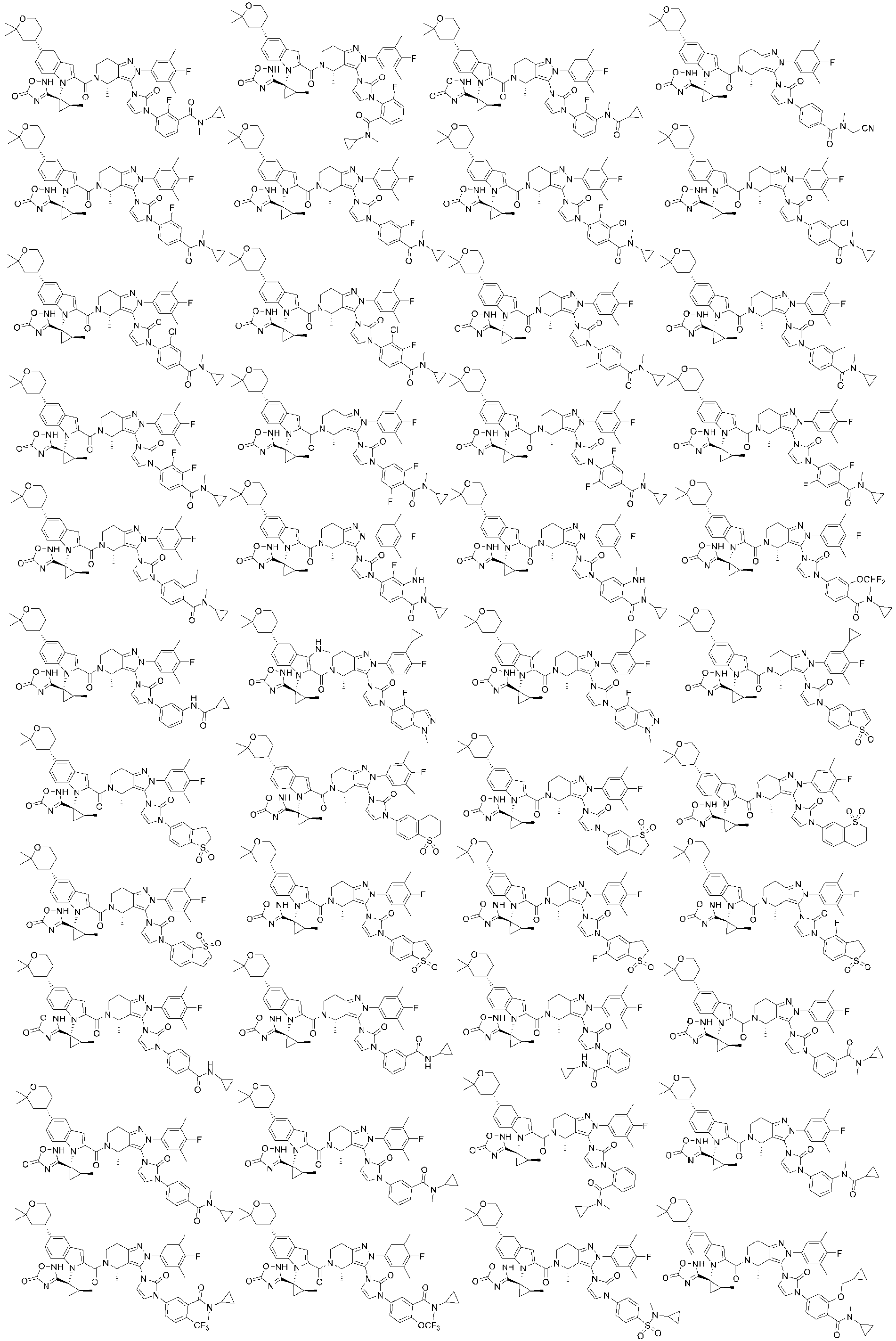

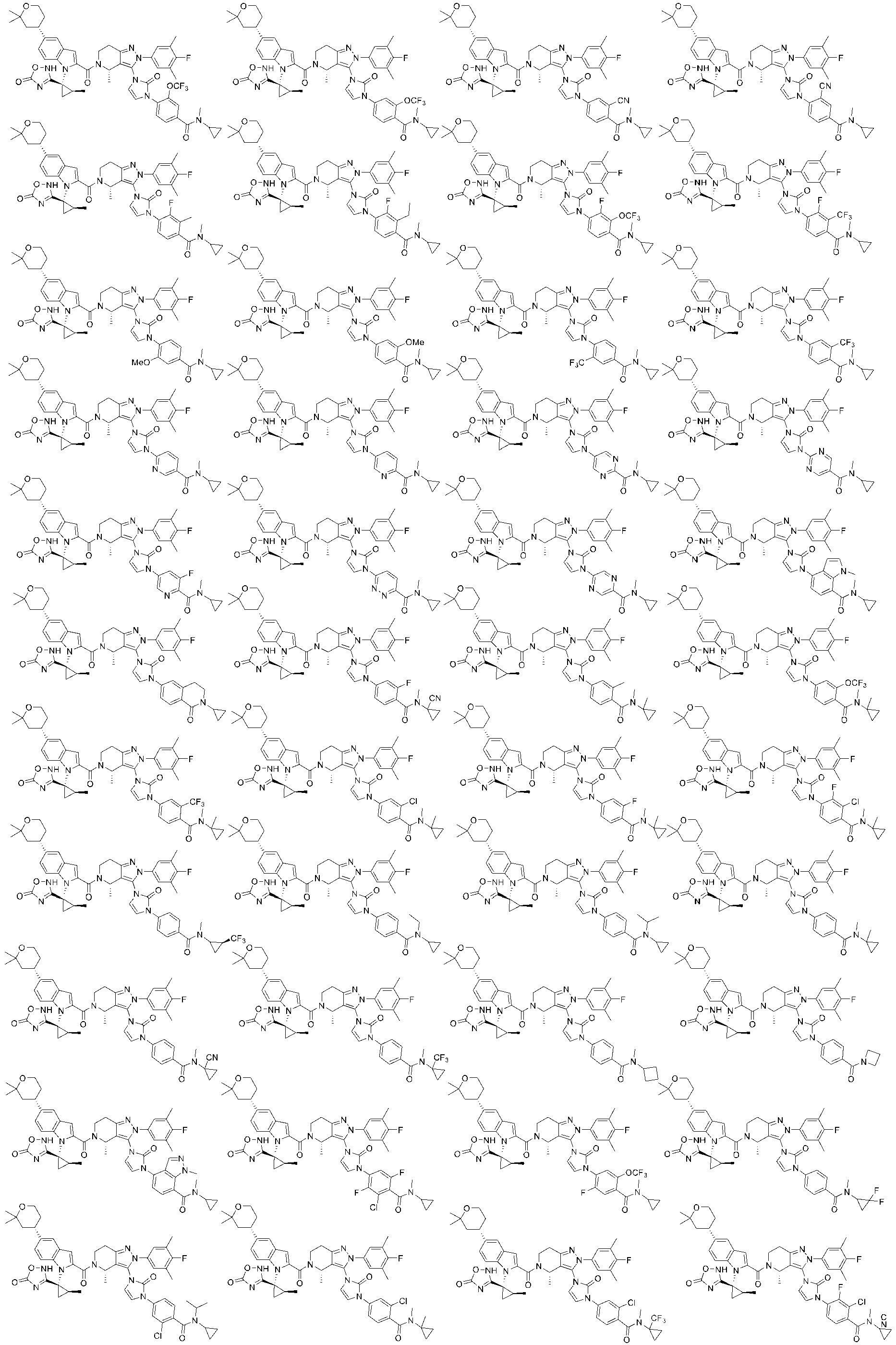

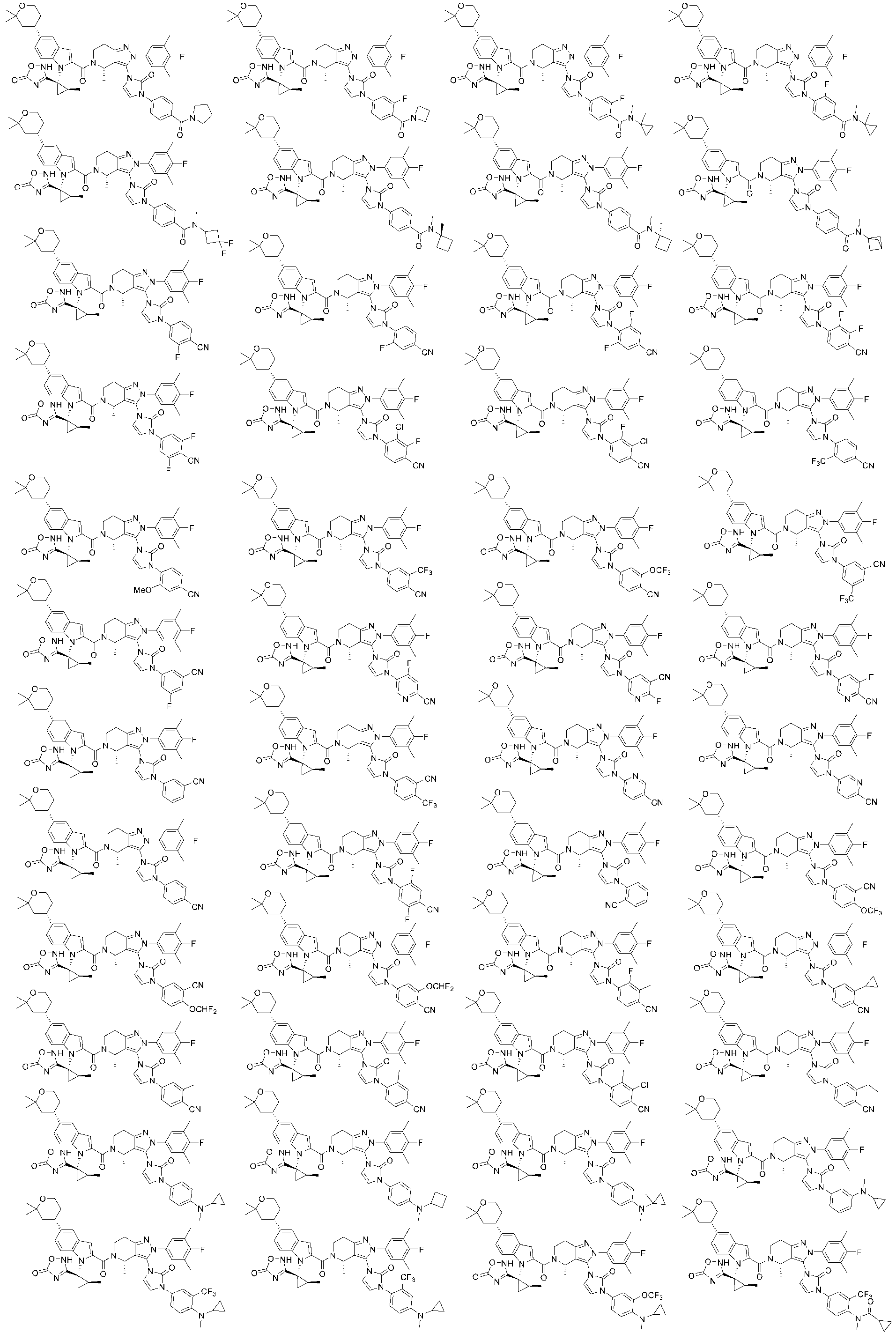

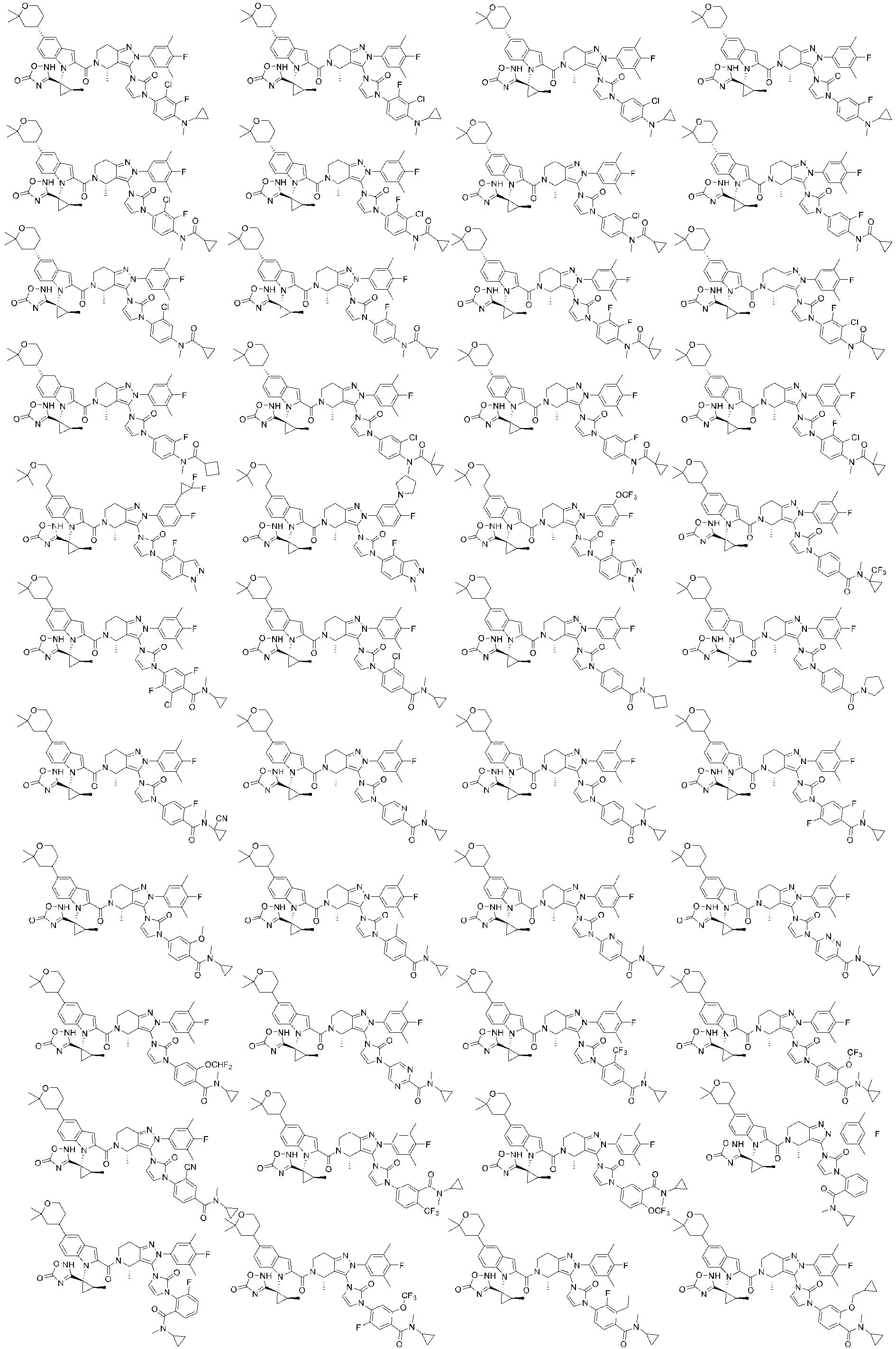

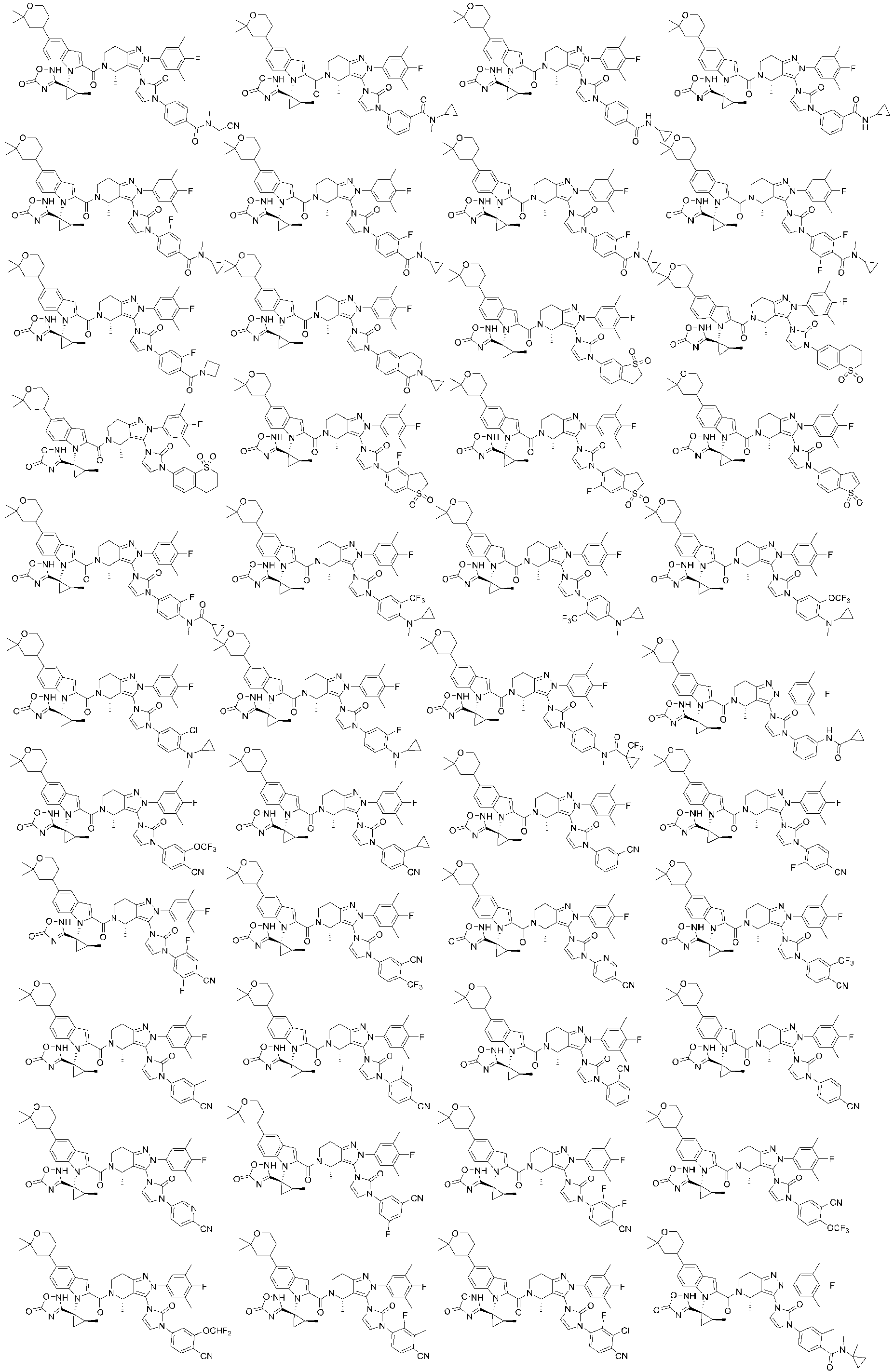

Another aspect of the present invention provides a method for preparing the above compound, the stereoisomer, tautomer, or pharmaceutically acceptable salt thereof, wherein it comprises the following step: 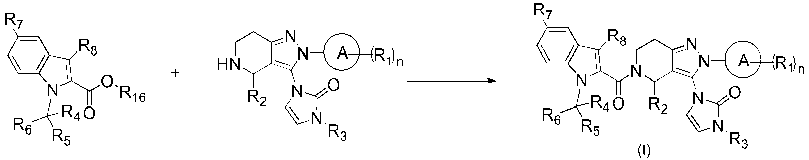 wherein R₁₆ is H.

Where appropriate, the various starting materials, intermediates, and compounds described herein can be isolated and purified using conventional techniques such as precipitation, filtration, crystallization, evaporation, distillation, and chromatography. These compounds can be characterized using conventional methods such as by melting point, mass spectrometry, nuclear magnetic resonance, and various spectroscopic analyses.

Another aspect of the present invention provides a pharmaceutical composition comprising the above compound, the stereoisomer, tautomer, or pharmaceutically acceptable salt thereof, and a pharmaceutically acceptable carrier.

Another aspect of the present invention provides use of the above compound, the stereoisomer, tautomer or pharmaceutically acceptable salt thereof, or the above pharmaceutical composition in the preparation of a medicament for treating and/or preventing a disease mediated by GLP-1 receptor agonist.

Another aspect of the present invention provides use of the above compound, the stereoisomer, tautomer or pharmaceutically acceptable salt thereof, or the above pharmaceutical composition in the preparation of a medicament for preventing and/or treating diabetes, hyperglycemia, insulin resistance, glucose intolerance, diabetic nephropathy, diabetic neuropathy, diabetic retinopathy, adipocyte dysfunction, obesity, dyslipidemia, and hyperinsulinemia.

It should be understood that, within the scope of the present invention, the above technical features of the present invention and the technical features specifically described below (such as in the examples) can be combined with each other to form new or preferred technical solutions. Due to space limitations, these combinations will not be enumerated one by one.

### DETAILED DESCRIPTION OF THE INVENTION

After extensive and in-depth research, through extensive screening and testing, the inventors of the present invention have provided a class of structurally novel GLP-1 receptor agonists. The present invention was completed on this basis.

Unless otherwise defined, all technical and scientific terms used herein have the same meaning as commonly understood by one of ordinary skill in the art to which this invention belongs.

As used herein, "containing" or "comprising (including)" can be open-ended, semi-closed, and closed. In other words, the terms also include "consisting essentially of..." or "consisting of...".

"Alkyl" refers to a straight-chain or branched-chain saturated aliphatic hydrocarbon group, preferably including straight-chain and branched-chain alkyls having 1 to 10, or 1 to 6, or 1 to 4 carbon atoms, including but not limited to methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, or various branched isomers thereof. "C₁₋₁₀ alkyl" refers to straight-chain and branched-chain alkyls having 1 to 10 carbon atoms, "C₁₋₄ alkyl" refers to straight-chain and branched-chain alkyls having 1 to 4 carbon atoms.

Alkyl can be optionally substituted or unsubstituted. When substituted, the substituents are preferably one or more (preferably 1, 2, 3, or 4) of the following groups, independently selected from a group consisting of deuterium, halogen, cyano, nitro, azido, C₁₋₁₀ alkyl, C₂₋₁₀ alkenyl, C₂₋₁₀ alkynyl, halogenated C₁₋₁₀ alkyl, deuterium-substituted C₁₋₁₀ alkyl, C₃₋₁₂ cycloalkyl, 3-12 membered heterocyclyl, C₆₋₁₀ aryl, 5-10 membered heteroaryl, =O, -SF₅, -S(O)ᵣR₉, -O-R₁₀, - C(O)OR₁₀, -C(O)R₁₁, -O-C(O)R₁₁, -NR₁₂R₁₃, -C(=NR₁₂)R₁₁, -N(R₁₂)-C(=NR₁₃)R₁₁, -C(O)NR₁₂R₁₃, and -N(R₁₂)-C(O)R₁₁.

"Cycloalkyl" or "carbocycle" refers to a saturated or partially unsaturated monocyclic or polycyclic cyclic hydrocarbon substituent, wherein the partially unsaturated cyclic hydrocarbon means that the cyclic hydrocarbon may contain one or more (preferably 1, 2, or 3) double bonds, but no ring has a fully conjugated π-electron system. Cycloalkyls are divided into monocyclic cycloalkyls and polycyclic cycloalkyls, preferably including cycloalkyls having 3 to 12, or 3 to 8, or 3 to 6 carbon atoms. For example, "C₃₋₁₂ cycloalkyl" refers to cycloalkyls having 3 to 12 carbon atoms, "C₃₋₆ cycloalkyl" refers to cycloalkyls having 3 to 6 carbon atoms, wherein:
monocyclic cycloalkyls include but are not limited to cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like.

Polycyclic cycloalkyls include spiro, fused, and bridged cycloalkyls. "Spirocycloalkyl" refers to a polycyclic group in which single rings share one carbon atom (called a spiro atom), which may contain one or more (preferably 1, 2, or 3) double bonds, but no ring has a fully conjugated π-electron system. According to the number of spiro atoms shared between rings, spirocycloalkyls are divided into monospiro cycloalkyls, dispiro cycloalkyls, or polyspiro cycloalkyls. Spirocycloalkyls include but are not limited to:

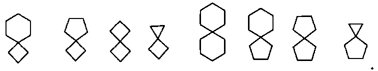

"Fused cycloalkyl" refers to an all-carbon polycyclic group in which each ring shares an adjacent pair of carbon atoms with other rings in the system, wherein one or more rings may contain one or more (preferably 1, 2, or 3) double bonds, but no ring has a fully conjugated π-electron system. According to the number of rings, they can be divided into bicyclic, tricyclic, tetracyclic, or polycyclic fused cycloalkyls. Fused cycloalkyls include but are not limited to:

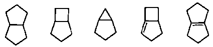

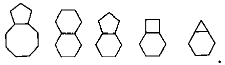

"Bridged cycloalkyl" refers to an all-carbon polycyclic group in which any two rings share two non-directly connected carbon atoms, which may contain one or more (preferably 1, 2, or 3) double bonds, but no ring has a fully conjugated π-electron system. According to the number of rings, they can be divided into bicyclic, tricyclic, tetracyclic, or polycyclic bridged cycloalkyls. Bridged cycloalkyls include but are not limited to:

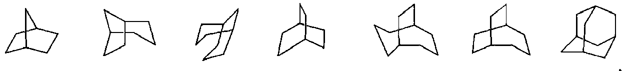

The cycloalkyl ring may be fused to an aryl, heteroaryl, or heterocycloalkyl ring, wherein the ring connected to the parent structure is a cycloalkyl, including but not limited to indanyl, tetrahydronaphthalenyl, benzocycloheptanyl, etc.

Cycloalkyl may be optionally substituted or unsubstituted, when substituted, the substituents are preferably one or more (preferably 1, 2, 3, or 4) of the following groups, independently selected from a group consisting of deuterium, halogen, cyano, nitro, azido, C₁₋₁₀ alkyl, C₂₋₁₀ alkenyl, C₂₋₁₀ alkynyl, halogenated C₁₋₁₀ alkyl, deuterium-substituted C₁₋₁₀ alkyl, C₃₋₁₂ cycloalkyl, 3-12 membered heterocyclyl, C₆₋₁₀ aryl, 5-10 membered heteroaryl, =O, -SF₅, -S(O)ᵣR₉, -O-R₁₀, - C(O)OR₁₀, -C(O)R₁₁, -O-C(O)R₁₁, -NR₁₂R₁₃, -C(=NR₁₂)R₁₁, -N(R₁₂)-C(=NR₁₃)R₁₁, -C(O)NR₁₂R₁₃, and -N(R₁₂)-C(O)R₁₁.

"Heterocyclyl" or "heterocycle" refers to a saturated or partially unsaturated monocyclic or polycyclic cyclic hydrocarbon substituent, wherein the partially unsaturated cyclic hydrocarbon refers to a cyclic hydrocarbon that may contain one or more (preferably 1, 2, or 3) double bonds, but no ring has a fully conjugated π electron system, wherein one or more (preferably 1, 2, 3, or 4) ring atoms are selected from heteroatoms such as nitrogen, oxygen, or S(O)r (wherein r is an integer 0, 1, 2), but excluding ring portions of -O-O-, -O-S-, or -S-S-, and the remaining ring atoms are carbon. Preferably, the heterocyclyl includes 3 to 12, or 3 to 8, or 3 to 6 ring atoms, for example, "3-6 membered heterocyclyl" refers to a ring group containing 3 to 6 ring atoms, "4-6 membered heterocyclyl" refers to a ring group containing 4 to 6 ring atoms, "4-10 membered heterocyclyl" refers to a ring group containing 4 to 10 ring atoms, "3-12 membered heterocyclyl" refers to a ring group containing 3 to 12 ring atoms.

Monocyclic heterocyclyl includes, but is not limited to, pyrrolidinyl, piperidinyl, piperazinyl, morpholinyl, thiomorpholinyl, homopiperazinyl, oxetanyl, tetrahydrofuranyl, and the like.

Polycyclic heterocyclyl includes spiro, fused, and bridged heterocyclyls. "Spiro heterocyclyl" refers to a polycyclic heterocyclyl wherein single rings share one atom (called the spiro atom), wherein one or more (preferably 1, 2, 3, or 4) ring atoms are selected from heteroatoms such as nitrogen, oxygen, or S(O)r (wherein r is an integer 0, 1, 2), and the remaining ring atoms are carbon. These may contain one or more double bonds (preferably 1, 2, or 3), but no ring has a fully conjugated π electron system. According to the number of spiro atoms shared between rings, spiro heterocyclyl can be classified as mono-spiro heterocyclyl, bi-spiro heterocyclyl, or poly-spiro heterocyclyl. Spiro heterocyclyl includes, but is not limited to:

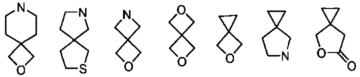

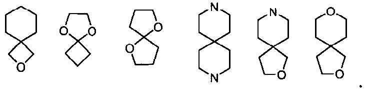

"Fused heterocyclyl" refers to a polycyclic heterocyclyl wherein each ring shares an adjacent pair of atoms with other rings in the system, one or more (preferably 1, 2, 3, or 4) rings may contain one or more (preferably 1, 2, or 3) double bonds, but no ring has a fully conjugated π electron system, wherein one or more (preferably 1, 2, 3, or 4) ring atoms are selected from heteroatoms such as nitrogen, oxygen, or S(O)r (wherein r is an integer 0, 1, 2), and the remaining ring atoms are carbon. According to the number of component rings, they can be classified as bicyclic, tricyclic, tetracyclic, or polycyclic fused heterocyclyl. Fused heterocyclyl includes, but is not limited to:

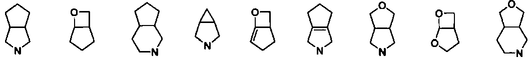

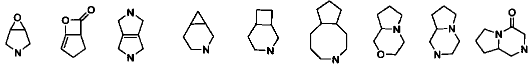

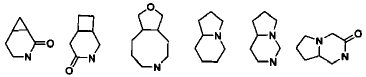

"Bridged heterocyclyl" refers to a polycyclic heterocyclyl wherein any two rings share two non-directly connected atoms, these may contain one or more (preferably 1, 2, or 3) double bonds, but no ring has a fully conjugated π electron system, wherein one or more (preferably 1, 2, 3, or 4) ring atoms are selected from a group consisting of heteroatoms such as nitrogen, oxygen, or S(O)r (wherein r is an integer 0, 1, 2), and the remaining ring atoms are carbon. According to the number of component rings, they can be classified as bicyclic, tricyclic, tetracyclic, or polycyclic bridged heterocyclyl. Bridged heterocyclyl includes, but is not limited to:

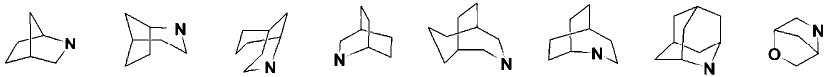

The heterocyclyl ring may be fused to an aryl, heteroaryl, or cycloalkyl ring, wherein the ring connected to the parent structure is the heterocyclyl ring, including but not limited to:

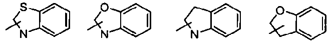

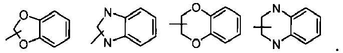

Heterocyclyl may be optionally substituted or unsubstituted, when substituted, the substituents are preferably one or more (preferably 1, 2, 3, or 4) of the following groups, independently selected from a group consisting of deuterium, halogen, cyano, nitro, azido, C₁₋₁₀ alkyl, C₂₋₁₀ alkenyl, C₂₋₁₀ alkynyl, halogenated C₁₋₁₀ alkyl, deuterium-substituted C₁₋₁₀ alkyl, C₃₋₁₂ cycloalkyl, 3-12 membered heterocyclyl, C₆₋₁₀ aryl, 5-10 membered heteroaryl, =O, -SF₅, -S(O)ᵣR₉, -O-R₁₀, -C(O)OR₁₀, -C(O)R₁₁, -O-C(O)R₁₁, -NR₁₂R₁₃, -C(=NR₁₂)R₁₁, -N(R₁₂)-C(=NR₁₃)R₁₁, - C(O)NR₁₂R₁₃, and -N(R₁₂)-C(O)R₁₁.

"Aryl" or "aromatic ring" refers to an all-carbon monocyclic or fused polycyclic group (i.e., rings which share adjacent pairs of carbon atoms) having a conjugated π electron system, preferably containing 5-10 or 5-8 carbons, for example, "C₆₋₁₀ aryl" refers to an all-carbon aryl containing 6-10 carbon atoms, including but not limited to phenyl and naphthyl, "C₆₋₈ aryl" refers to an all-carbon aryl containing 6-8 carbon atoms. The aryl ring may be fused to a heteroaryl, heterocyclyl, or cycloalkyl ring, wherein the ring connected to the parent structure is the aryl ring, including but not limited to:

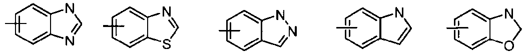

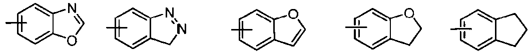

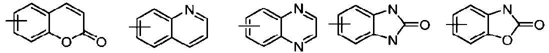

"Aryl" may be substituted or unsubstituted, when substituted, the substituents are preferably one or more (preferably 1, 2, 3, or 4) of the following groups, independently selected from a group consisting of deuterium, halogen, cyano, nitro, azido, C₁₋₁₀ alkyl, C₂₋₁₀ alkenyl, C₂₋₁₀ alkynyl, halogenated C₁₋₁₀ alkyl, deuterium-substituted C₁₋₁₀ alkyl, C₃₋₁₂ cycloalkyl, 3-12 membered heterocyclyl, C₆₋₁₀ aryl, 5-10 membered heteroaryl, =O, -SF₅, -S(O)ᵣR₉, -O-R₁₀, -C(O)OR₁₀, - C(O)R₁₁, -O-C(O)R₁₁, -NR₁₂R₁₃, -C(NR₁₂)R₁₁, -N(R₁₂)-C(=NR₁₃)R₁₁, -C(O)NR₁₂R₁₃, and - N(R₁₂)-C(O)R₁₁.

"Heteroaryl" refers to a heteroaromatic system containing one or more (preferably 1, 2, 3, or 4) heteroatoms, wherein said heteroatoms include nitrogen, oxygen, and S(O)r (wherein r is an integer 0, 1, 2), preferably a heteroaromatic system containing 5-10 or 5-8 or 5-6 ring atoms, for example, 5-6 membered heteroaryl refers to a heteroaromatic system containing 5-6 ring atoms, 5-8 membered heteroaryl refers to a heteroaromatic system containing 5-8 ring atoms, 5-10 membered heteroaryl refers to a heteroaromatic system containing 5-10 ring atoms, including but not limited to furanyl, thiophenyl, pyridinyl, pyrrolyl, N-alkylpyrrolyl, pyrimidinyl, pyrazinyl, imidazolyl, tetrazolyl, benzopyrazolyl, 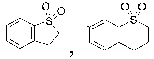 and the like. The heteroaryl ring may be fused to an aryl, heterocyclyl, or cycloalkyl ring, wherein the ring connected to the parent structure is a heteroaryl ring, including but not limited to:

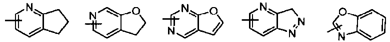

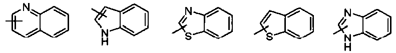

"Heteroaryl" may be optionally substituted or unsubstituted, when substituted, the substituents are preferably one or more (preferably 1, 2, 3, or 4) of the following groups, independently selected from a group consisting of deuterium, halogen, cyano, nitro, azido, C₁₋₁₀ alkyl, C₂₋₁₀ alkenyl, C₂₋₁₀ alkynyl, halogenated C₁₋₁₀ alkyl, deuterium-substituted C₁₋₁₀ alkyl, C₃₋₁₂ cycloalkyl, 3-12 membered heterocyclyl, C₆₋₁₀ aryl, 5-10 membered heteroaryl, =O, -SF₅, -S(O)ᵣR₉, -O-R₁₀, -C(O)OR₁₀, -C(O)R₁₁, -O-C(O)R₁₁, -NR₁₂R₁₃, -C(=NR₁₂)R₁₁, -N(R₁₂)-C(=NR₁₃)R₁₁, - C(O)NR₁₂R₁₃, and -N(R₁₂)-C(O)R₁₁.

"Alkenyl" refers to an alkyl as defined above consisting of at least two carbon atoms and at least one carbon-carbon double bond, preferably a straight-chain or branched-chain alkenyl containing 2-10 or 2-4 carbon atoms, for example, C₂₋₁₀ alkenyl refers to a straight-chain or branched-chain alkenyl containing 2-10 carbon atoms, C₂₋₄ alkenyl refers to a straight-chain or branched-chain alkenyl containing 2-4 carbon atoms. Alkenyl includes but not limited to ethenyl, 1-propenyl, 2-propenyl, 1-, 2-, or 3-butenyl, and the like.

"Alkenyl" may be substituted or unsubstituted, when substituted, the substituents are preferably one or more (preferably 1, 2, 3, or 4) of the following groups, independently selected from a group consisting of deuterium, halogen, cyano, nitro, azido, C₁₋₁₀ alkyl, C₂₋₁₀ alkenyl, C₂₋₁₀ alkynyl, halogenated C₁₋₁₀ alkyl, deuterium-substituted C₁₋₁₀ alkyl, C₃₋₁₂ cycloalkyl, 3-12 membered heterocyclyl, C₆₋₁₀ aryl, 5-10 membered heteroaryl, =O, -SF₅, -S(O)ᵣR₉, -O-R₁₀, - C(O)OR₁₀, -C(O)R₁₁, -O-C(O)R₁₁, -NR₁₂R₁₃, -C(=NR₁₂)R₁₁, -N(R₁₂)-C(=NR₁₃)R₁₁, -C(O)NR₁₂R₁₃, and -N(R₁₂)-C(O)R₁₁.

"Alkynyl" refers to an alkyl as defined above consisting of at least two carbon atoms and at least one carbon-carbon triple bond, preferably a straight-chain or branched-chain alkynyl containing 2-10 or 2-4 carbon atoms, for example, C₂₋₁₀ alkynyl refers to a straight-chain or branched-chain alkynyl containing 2-10 carbon atoms, C₂₋₄ alkynyl refers to a straight-chain or branched-chain alkynyl containing 2-4 carbon atoms. Alkynyl includes but not limited to ethynyl, 1-propynyl, 2-propynyl, 1-, 2-, or 3-butynyl, and the like.

"Alkynyl" may be substituted or unsubstituted, when substituted, the substituents are preferably one or more (preferably 1, 2, 3, or 4) of the following groups, independently selected from a group consisting of deuterium, halogen, cyano, nitro, azido, C₁₋₁₀ alkyl, C₂₋₁₀ alkenyl, C₂₋₁₀ alkynyl, halogenated C₁₋₁₀ alkyl, deuterium-substituted C₁₋₁₀ alkyl, C₃₋₁₂ cycloalkyl, 3-12 membered heterocyclyl, C₆₋₁₀ aryl, 5-10 membered heteroaryl, =O, -SF₅, -S(O)ᵣR₉, -O-R₁₀, - C(O)OR₁₀, -C(O)R₁₁, -O-C(O)R₁₁, -NR₁₂R₁₃, -C(=NR₁₂)R₁₁, -N(R₁₂)-C(=NR₁₃)R₁₁, -C(O)NR₁₂R₁₃, and -N(R₁₂)-C(O)R₁₁.

"Alkoxy" refers to -O-alkyl, wherein alkyl is as defined above, for example, "C₁₋₁₀ alkoxy" refers to an alkoxy containing 1-10 carbon atoms, "C₁₋₄ alkoxy" refers to an alkoxy containing 1-4 carbon atoms, including but not limited to methoxy, ethoxy, propoxy, butoxy, and the like.

"Alkoxy" may be optionally substituted or unsubstituted, when substituted, the substituents are preferably one or more (preferably 1, 2, 3, or 4) of the following groups, independently selected from a group consisting of deuterium, halogen, cyano, nitro, azido, C₁₋₁₀ alkyl, C₂₋₁₀ alkenyl, C₂₋₁₀ alkynyl, halogenated C₁₋₁₀ alkyl, deuterium-substituted C₁₋₁₀ alkyl, C₃₋₁₂ cycloalkyl, 3-12 membered heterocyclyl, C₆₋₁₀ aryl, 5-10 membered heteroaryl, =O, -SF₅, -S(O)ᵣR₉, -O-R₁₀, - C(O)OR₁₀, -C(O)R₁₁, -O-C(O)R₁₁, -NR₁₂R₁₃, -C(=NR₁₂)R₁₁, -N(R₁₂)-C(=NR₁₃)R₁₁, -C(O)NR₁₂R₁₃, and -N(R₁₂)-C(O)R₁₁.

"Cycloalkoxy" refers to -O-cycloalkyl, wherein cycloalkyl is as defined above, for example, "C₃₋₁₂ cycloalkoxy" refers to a cycloalkoxy containing 3-12 carbon atoms, "C₃₋₆ cycloalkoxy" refers to a cycloalkoxy containing 3-6 carbon atoms, including but not limited to cyclopropoxy, cyclobutoxy, cyclopentoxy, cyclohexoxy, and the like.

"Cycloalkoxy" may be optionally substituted or unsubstituted, when substituted, the substituents are preferably one or more (preferably 1, 2, 3, or 4) of the following groups, independently selected from a group consisting of deuterium, halogen, cyano, nitro, azido, C₁₋₁₀ alkyl, C₂₋₁₀ alkenyl, C₂₋₁₀ alkynyl, halogenated C₁₋₁₀ alkyl, deuterium-substituted C₁₋₁₀ alkyl, C₃₋₁₂ cycloalkyl, 3-12 membered heterocyclyl, C₆₋₁₀ aryl, 5-10 membered heteroaryl, =O, -SF₅, -S(O)ᵣR₉, -O-R₁₀, -C(O)OR₁₀, -C(O)R₁₁, -O-C(O)R₁₁, -NR₁₂R₁₃, -C(=NR₁₂)R₁₁, -N(R₁₂)-C(=NR₁₃)R₁₁, - C(O)NR₁₂R₁₃, and -N(R₁₂)-C(O)R₁₁.

"Heterocyclyloxy" refers to -O-heterocyclyl, wherein heterocyclyl is as defined above, including but not limited to azacyclobutyloxy, oxacyclobutyloxy, azacyclopentyloxy, azacyclohexyloxy, oxacyclohexyloxy, and the like.

"Heterocyclooxy" may be optionally substituted or unsubstituted, and when substituted, the substituents are preferably one or more (preferably 1, 2, 3, or 4) of the following groups, independently selected from a group consisting of deuterium, halogen, cyano, nitro, azido, C₁₋₁₀ alkyl, C₂₋₁₀ alkenyl, C₂₋₁₀ alkynyl, halogenated C₁₋₁₀ alkyl, deuterium-substituted C₁₋₁₀ alkyl, C₃₋₁₂ cycloalkyl, 3-12 membered heterocyclyl, C₆₋₁₀ aryl, 5-10 membered heteroaryl, =O, -SF₅, -S(O)ᵣR₉, -O-R₁₀, -C(O)OR₁₀, -C(O)R₁₁, -O-C(O)R₁₁, -NR₁₂R₁₃, -C(=NR₁₂)R₁₁, -N(R₁₂)-C(=NR₁₃)R₁₁, - C(O)NR₁₂R₁₃, and -N(R₁₂)-C(O)R₁₁.

The definitions of the above R₉, R₁₀, R₁₁, R₁₂, R₁₃ are as defined above.

"C₁₋₁₀ alkanoyl" refers to a monovalent group remaining after removing the hydroxyl from a C₁₋₁₀ alkyl, typically represented as "C₀₋₉ alkyl-C(O)-". For example, "C₁ alkyl-C(O)-" refers to acetyl; "C₂ alkyl-C(O)-" refers to propionyl; "C₃ alkyl-C(O)-" refers to butyryl or isobutyryl.

"Halogenated C₁₋₁₀ alkyl" refers to a C₁₋₁₀ alkyl in which hydrogen is optionally substituted by fluorine, chlorine, bromine, or iodine atoms, including but not limited to difluoromethyl, dichloromethyl, dibromomethyl, trifluoromethyl, trichloromethyl, tribromomethyl, and the like.

"Deuterium-substituted C₁₋₁₀ alkyl" refers to a C₁₋₁₀ alkyl in which hydrogen is optionally substituted by deuterium atoms, including but not limited to monodeuteromethyl, dideuteromethyl, trideuteromethyl, etc.

"Halogen" refers to fluorine, chlorine, bromine, or iodine.

"Optional" or "optionally" means that the event or circumstance subsequently described may, but not necessarily, occur, and that the description includes instances where the event or circumstance occurs or does not occur, that is, instances where substitution occurs or does not occur. For example, "heterocyclyl optionally substituted with alkyl" means that alkyl may be, but not necessarily, present, and that the description includes instances where the heterocyclyl is or is not substituted with alkyl.

"Substituted" means that one or more "hydrogen atoms" in a group are independently replaced by a corresponding number of substituents. It is self-evident that substituents are only present at chemically possible positions, consistent with chemical valence theory, and those skilled in the art can determine (through experiment or theory) possible or impossible substitution without undue effort. For example, it may be unstable when amino or hydroxy having free hydrogen is bound to a carbon atom having an unsaturated bond (such as olefin).

"Stereoisomer" refers to isomers that differ in the spatial arrangement of atoms in a molecule, which can be divided into cis-trans isomers and enantiomers, or into enantiomers and diastereomers. Stereoisomers caused by rotation around single bond are called conformational stereoisomers, sometimes also called rotamers. Stereoisomers caused by bond length, bond angle, double bonds, or rings are called configuration stereoisomers, which are further divided into two categories. Isomers caused by restricted rotation around double bonds or cyclic carbon single bond are called geometric isomers, also known as cis-trans isomers, classified as Z and E configurations. For example, cis-2-butene and trans-2-butene are a pair of geometric isomers. Stereoisomers with different optical properties due to the absence of anti-axisymmetry in the molecule are called optical isomers, classified as R and S configurations. In the present invention, unless otherwise specified, "stereoisomer" can be understood to include one or more of the above enantiomers, configurational isomers, and conformational isomers.

"Tautomer" refers to structural isomers of different energies that interconvert via low energy barriers. For example, prototropic tautomers include tautomerism via proton migration, and valence tautomers include interconversion through reorganization of some bonding electrons. For example, 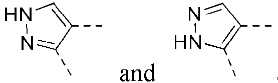 are tautomers. In the present invention, unless otherwise specified, all tautomeric forms of the compounds are within the scope of the invention.

"Pharmaceutically acceptable salt" in the present invention refers to pharmaceutically acceptable acid addition salts, including inorganic acid salts and organic acid salts, which can be prepared by methods known in the art.

"Pharmaceutical composition" refers to a mixture containing one or more compounds described herein or their physiologically/pharmaceutically acceptable salts or prodrugs with other chemical components, as well as other components such as physiologically/pharmaceutically acceptable carriers and excipients. The purpose of a pharmaceutical composition is to facilitate administration to an organism, promote absorption of the active ingredient, and thereby exert biological activity.

The compounds of the present application may contain non-natural proportions of atomic isotopes at one or more of the atoms constituting such compounds. For example, compounds may be labeled with radioactive isotopes, such as tritium (³H), iodine-125 (¹²⁵I), or C-14 (¹⁴C). Alternatively, hydrogen may be replaced with deuterium to form deuterated drug. All isotopic variations of the compounds of the present application, whether radioactive or not, are included within the scope of the present application.

The following specific embodiments further illustrate the present invention. It should be understood that these examples are used to illustrate the present invention and not to limit the scope of the present invention.

The structures of the compounds of the present invention were determined by nuclear magnetic resonance (NMR) and/or liquid chromatography-mass spectrometry (LC-MS). NMR chemical shifts (δ) are given in parts per million (ppm). NMR measurements were performed using a Bruker AVANCE-400/500 spectrometer, with deuterated dimethyl sulfoxide (DMSO-d₆), deuterated methanol (CD₃OD), and deuterated chloroform (CDCl₃) as solvents, and tetramethylsilane (TMS) as an internal standard.

LC-MS measurements were performed using an Agilent 6120 mass spectrometer. HPLC measurements were performed using an Agilent 1200DAD high-pressure liquid chromatograph (Sunfire C18 150×4.6 mm column) and a Waters 2695-2996 high-pressure liquid chromatograph (Gimini C18 150×4.6 mm column).

Thin-layer chromatography silica gel plates used were from Yantai Huanghai HSGF254 or Qingdao GF254 silica gel plates. The specification used for TLC was 0.15mm-0.20mm, and the specification used for thin-layer chromatography separation and purification of products was 0.4mm-0.5mm. Column chromatography generally used Yantai Huanghai 200-300 mesh silica gel as the carrier.

The starting materials in the examples of the present invention are known and commercially available, or can be synthesized according to methods known in the art.

Unless otherwise specified, all reactions in the present invention are carried out under continuous magnetic stirring, in a dry nitrogen or argon atmosphere, using dry solvents, with reaction temperatures in degrees Celsius (°C).

In the examples, where specific conditions are not indicated, conventional conditions or conditions recommended by the manufacturer are followed. Reagents or instruments for which manufacturers are not specified are conventional products that can be obtained commercially.

In the present invention, for all parts involving SFC chiral separation, the first eluting component is designated as a, and the later eluting component as b. For example, if a pair of enantiomeric compounds 1 undergoes SFC chiral separation to yield two enantiomers sequentially, they are recorded as compound 1a and compound 1b, respectively.

The reference compound a used in the present invention is derived from compound 67 in CN109790161, and compound b is derived from compound 146b in WO2021155841.

### Preparation of Intermediates

### Synthesis of Intermediates A-1 and A-2

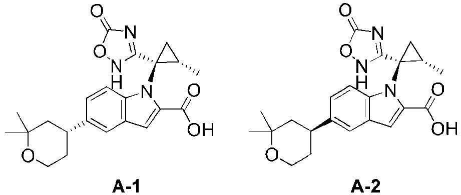

### Intermediates A-1 and A-2 were synthesized via the following route:

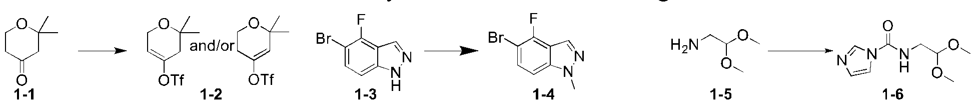

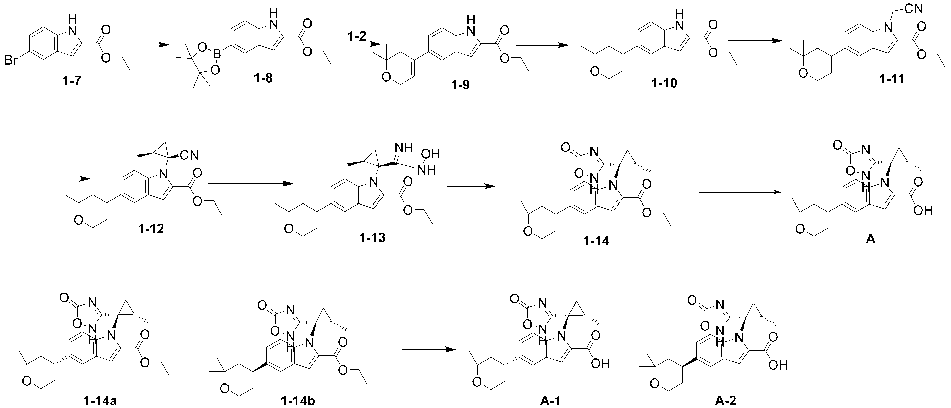

### Step 1: Synthesis of Intermediate 1-2

Under a nitrogen atmosphere at -78°C, LDA (117 mL, 2M/THF) was slowly added dropwise to a solution of compound 1-1 (25 g, 195.1 mmol) in tetrahydrofuran (100 mL), stirred for 20 minutes and maintained at -70°C. A solution of N,N-bis(trifluoromethanesulfonyl)aniline (44 g, 234 mmol) in tetrahydrofuran (100 mL) was added dropwise to the above mixture. After completion of the addition, the reaction was allowed to warm to room temperature and stirred for 16 hours. Under ice bath condition, saturated ammonium chloride solution was added dropwise to the above mixture, which was then extracted with ethyl acetate, and washed with saturated brine. The organic phase was collected and dried, concentrated under reduced pressure to obtain the crude product, which was purified by column chromatography (0-15% petroleum ether/ethyl acetate) to afford compound 1-2 (38.2 g, 75%).

### Step 2: Synthesis of Intermediate 1-4

Under ice bath condition, potassium tert-butoxide (13 g, 0.12 mol) was added to a solution of compound 1-3 (25 g, 0.12 mol) in N,N-dimethylformamide (100 mL). After stirring at room temperature for 40 minutes, iodomethane (20 g, 0.14 mol) was slowly added dropwise under ice bath condition, and the mixture was stirred at room temperature for 16 hours. After the reaction was complete, saturated ammonium chloride solution was added. The mixture was extracted with ethyl acetate, washed with saturated brine, the organic phase was collected and dried, concentrated under reduced pressure to obtain the crude product, which was purified by column chromatography (petroleum ether/ethyl acetate = 0-20%) to afford compound 1-4 (16.6 g, 70%).

LC-MS (ESI⁺) m/z: 230.1 (M+H)⁺.

### Step 3: Synthesis of Intermediate 1-6

Under ice bath condition, aminoacetaldehyde dimethanol (2 g, 19 mmol) was added to a solution of N,N'-carbonyldiimidazole (3.1 g, 19 mmol) in ethyl acetate (10 mL). After stirring at room temperature for 2 hours, the mixture was cooled to 0°C and stirred for 30 minutes and filtered. The crude product was obtained by pulping twice with methyl tert-butyl ether and further purified (methanol/dichloromethane = 0-5%) to afford compound 1-4 (3.6 g, 95%).

### Step 4: Synthesis of Intermediate 1-8

Under a nitrogen atmosphere, a mixture of compound 1-7 (28 g, 0.10 mol), bis(pinacolato)diboron (53 g, 0.20 mol), Pd(dppf)Cl₂ (3.8 g, 5.2 mmol), and potassium acetate (20.5 g, 0.21 mol) in 1,4-dioxane/water (300 mL/30 mL) was heated to 80°C and reacted for 16 hours. After the reaction was complete, the mixture was concentrated under reduced pressure to obtain the crude product, which was purified by column chromatography (petroleum ether/ethyl acetate = 0-15%) to afford compound 1-8 (32.7 g, 99%).

LC-MS (ESI⁺) m/z: 316.2 (M+H)⁺.

### Step 5: Synthesis of Intermediate 1-9

Under a nitrogen atmosphere, a mixture of compound 1-8 (20 g, 63 mmol), 1-2 (20 g, 76 mmol), Pd(dppf)Cl₂ (2.32 g, 3.20 mmol), and potassium carbonate (26.30 g, 190 mol) in 1,4-dioxane/water (250 mL/50 mL) was heated to 90°C and reacted for 16 hours. After the reaction was complete, the mixture was concentrated under reduced pressure to obtain the crude product, which was purified by column chromatography (petroleum ether/ethyl acetate = 0-10%) to afford compound 1-9 (9 g, 54%).

LC-MS (ESI⁺) m/z: 300.2 (M+H)⁺.

### Step 6: Synthesis of Intermediate 1-10

At room temperature, Pd/C (500 mg, 10%) was added to a solution of compound 1-9 (10.1 g, 33.67 mmol) in methanol (150 mL). After purging with nitrogen three times, the mixture was stirred at room temperature for 16 hours. After the reaction was complete, the mixture was filtered and concentrated under reduced pressure to obtain solid 1-10 (8.8 g), which was used in the next step without further purification.

LC-MS (ESI⁺) m/z: 302.2 (M+H)⁺.

### Step 7: Synthesis of Intermediate 1-11

Under ice bath condition, a solution of compound 1-10 (9.60 g, 31.8 mmol) in N,N-dimethylformamide (25 mL) was slowly added dropwise to a solution of NaH (1.70 g, 35 mmol) in N,N-dimethylformamide (100 mL) under a nitrogen atmosphere and stirred for 0.5 hours. Chloroacetonitrile (4.8 g, 64 mmol) was then added to the above mixture, and the mixture was stirred at room temperature for 2 hours. After LCMS indicated the reaction was complete, saturated ammonium chloride solution was added to quench the reaction, and the mixture was extracted with ethyl acetate. The organic phase was collected, washed with saturated brine, dried over anhydrous sodium sulfate, filtered, concentrated under reduced pressure to obtain the crude product, which was purified by column chromatography (petroleum ether/ethyl acetate = 0-15%) to afford compound 1-11 (9.1 g, 84%).

LC-MS (ESI⁺) m/z: 341.1 (M+H)⁺.

### Step 8: Synthesis of Intermediate 1-12

Under ice bath condition, lithium bis(trimethylsilyl)amide (45 mL, 1 M) was slowly added dropwise to a tetrahydrofuran (40 mL) solution containing compound 1-11 (3.80 g, 11.16 mmol) and (R)-4-methyl-1,3,2-dioxathiolane-2,2-dioxide (3.86 g, 27.91 mmol). The reaction was allowed to warm to room temperature and stirred for 2 hours. The reaction was quenched with saturated ammonium chloride solution, extracted with ethyl acetate, and the organic phase was collected, washed with saturated brine, dried over anhydrous sodium sulfate, filtered, and concentrated under reduced pressure. The crude product was purified by column chromatography (petroleum ether/ethyl acetate = 0-20%) to afford compound 1-12 (1.39 g, 33%).

LC-MS (ESI⁺) m/z: 381.3 (M+H)⁺.

### Step 9: Synthesis of intermediate 1-13

A solution of compound 1-12 (1.39 g, 3.65 mmol), hydroxylamine hydrochloride (1.27 g, 18.27 mmol), and potassium carbonate (2.78 g, 20 mmol) in ethanol was heated to reflux for 2 hours. The mixture was filtered and concentrated under reduced pressure to afford compound 1-13 (1.83 g), which was used in the next step without further purification.

LC-MS (ESI⁺) m/z: 414.3 (M+H)⁺.

### Step 10: Synthesis of intermediate 1-14

At room temperature, N,N'-carbonyldiimidazole (1.44 g, 8.85 mmol) and 1,8-diazabicyclo[5.4.0]undec-7-ene (1.7 g, 11.1 mmol) were sequentially added to a solution of compound 1-13 (1.83 g, 4.40 mmol) in dimethyl sulfoxide (8 mL). The mixture was heated to 80°C and stirred for 2 hours. After the reaction was complete, water was added, and the mixture was extracted with dichloromethane. The organic phase was collected, washed with saturated brine, dried over anhydrous sodium sulfate, filtered, and concentrated under reduced pressure. The crude product was purified by column chromatography (methanol/dichloromethane = 0-10%) to afford compound 1-14 (1.89 g, 97%).

LC-MS (ESI⁺) m/z: 440.3 (M+H)⁺.

Intermediate 1-14 was subjected to chiral separation to obtain compound 1-14a (retention time 6.61 min) and 1-14b (retention time 7.5 min).

### Chiral separation method:

Chromatographic conditions: Column: CHIRALPAK^{®} IB, 10µm, 30*250 mm; Mobile phase A: HEX+0.2% FA; Mobile phase B: ETOH+0.2% FA; Detection wavelength: 214nm/254nm; Flow rate: 25 mL/min; Column temperature: RT; Isocratic elution program: Mobile phase A: Mobile phase B = 85:15 (V/V).

### Step 11: Synthesis of intermediate A

Under ice bath condition, sodium hydroxide (400 mg, 10 mmol) was added to a mixed solution of compound 1-14 (1.0 g, 2.28 mmol) in tetrahydrofuran (12 mL) and water (3 mL). The mixture was heated to 60°C and stirred for 2 hours. After LCMS monitoring indicated the reaction was complete, 2N dilute hydrochloric acid was added to adjust the pH to 6-7. The mixture was extracted with ethyl acetate (80 mL × 3), and the organic phase was collected, washed with saturated brine (100 mL × 1), dried over anhydrous sodium sulfate, filtered, and concentrated under reduced pressure to afford crude compound A, which was used in the next step without further purification.

LC-MS (ESI⁺) m/z: 412.3 (M+H)⁺.

### Step 12: Synthesis of intermediates A-1 and A-2

Under ice bath condition, sodium hydroxide (280 mg, 7 mmol) was added to a mixed solution of compound 1-14a (294 mg, 0.67 mmol) in tetrahydrofuran (8 mL) and water (2 mL). The mixture was heated to 60°C and stirred for 2 hours. After the reaction was complete, 2N dilute hydrochloric acid was added to adjust the pH to 6-7. The mixture was extracted with ethyl acetate, and the organic phase was collected, washed with saturated brine, dried over anhydrous sodium sulfate, and filtered to afford compound A-1, which was used in the next step without further purification.

LC-MS (ESI⁺) m/z: 412.3 (M+H)⁺.

Under ice bath condition, sodium hydroxide (280 mg, 7 mmol) was added to a mixed solution of compound 1-14b (305 mg, 0.70 mmol) in tetrahydrofuran (8 mL) and water (2 mL). The mixture was heated to 60°C and stirred for 1 hour. After the reaction was complete, 2N dilute hydrochloric acid was added to adjust the pH to 6-7. The mixture was extracted with ethyl acetate, and the organic phase was collected, washed with saturated brine, dried over anhydrous sodium sulfate, and filtered to afford compound A-2, which was used in the next step without further purification.

LC-MS (ESI⁺) m/z: 412.3 (M+H)⁺.

### Synthesis of Intermediate B

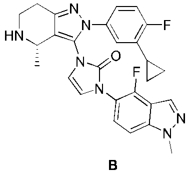

### Intermediate B was synthesized according to the following route:

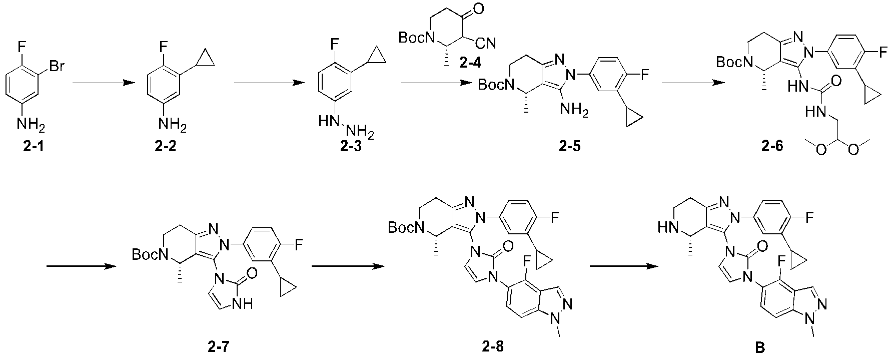

### Step 1: Synthesis of intermediate 2-2

Under nitrogen atmosphere, a mixed solution of compound 2-1 (15 g, 78.9 mmol), cyclopropylboronic acid (8.15 g, 94.7 mmol), Pd(dppf)Cl₂ (5.78 g, 7.9 mmol), and potassium carbonate (32.7 g, 0.24 mol) in 1,4-dioxane (150 mL) and water (30 mL) was heated to 100°C and stirred for 16 hours. The mixture was filtered and concentrated under reduced pressure. The crude product was purified by column chromatography (petroleum ether/ethyl acetate = 0-5%) to afford compound 2-2 (10.8 g, 85%).

LC-MS (ESI⁺) m/z: 152.1 (M+H)⁺.

### Step 2: Synthesis of intermediate 2-3

At room temperature, a mixed solution of compound 2-2 (8.8 g, 58.21 mmol) in concentrated hydrochloric acid (50 mL) and water (50 mL) was stirred for 1 hour and then filtered to obtain a solid. The solid was mixed with methyl tert-butyl ether and filtered to obtain a white solid. Under ice bath condition, the white solid was dissolved in concentrated hydrochloric acid (60 mL), and a solution of sodium nitrite (5.10 g, 74.12 mmol) in water (20 mL) was added in portions. The mixture was stirred for an additional 0.5 hour. Finally, a solution of stannous chloride (21.62 g, 114 mmol) in water (20 mL) was added, and the mixture was stirred for 2 hours. After the reaction was complete, the mixture was filtered directly and dried to afford compound 2-3 (3.9 g, 40%).

LC-MS (ESI⁺) m/z: 167.1 (M+H)⁺.

### Step 3: Synthesis of intermediate 2-5

At room temperature, pyridine (796 mg, 10.07 mmol) and compound 2-4 (2 g, 8.39 mmol) were added sequentially to a solution of compound 2-3 (2.03 g, 10.07 mmol) in ethanol (25 mL), , and then the mixture was heated to 80°C and stirred for 2 hours under nitrogen atmosphere. After the reaction was completed, the mixture was cooled to room temperature, and sodium hydroxide solution was added to adjust pH to 7-8. The mixture was extracted with dichloromethane, washed with saturated brine, dried over anhydrous sodium sulfate, filtered, and concentrated under reduced pressure. The crude product was purified by column chromatography (petroleum ether/ethyl acetate = 0-25%) to obtain compound 2-5 (3.09 g, 79%).

LC-MS (ESI⁺) m/z: 387.3 (M+H)⁺.

### Step 4: Synthesis of intermediate 2-6

At room temperature, compound 1-6 (6.36 g, 32 mmol) was added to a solution of compound 2-5 (3.09 g, 8 mmol) in N,N-dimethylformamide (40 mL), followed by the addition of potassium tert-butoxide (4.50 g, 40 mmol) under ice bath condition, and the mixture was stirred continuously for 16 hours. After the reaction was completed, the mixture was washed with water, extracted with ethyl acetate, the organic phase was collected, washed with saturated brine, dried over anhydrous sodium sulfate, filtered, and concentrated under reduced pressure to obtain crude compound 2-6 (4.89 g), which was used directly for the next step without column chromatography purification.

LC-MS (ESI⁺) m/z: 518.4 (M+H)⁺.

### Step 5: Synthesis of intermediate 2-7

Under ice bath condition, trifluoromethanesulfonic acid (3.50 g, 23.32 mmol) was added to a solution of crude compound 2-6 (4.89 g) in tetrahydrofuran (20 mL), and then the temperature was raised to 60°C for 1 hour. After cooling to room temperature, triethylamine (4.36 g, 43.08 mmol) and di-tert-butyl dicarbonate (3.53 g, 16.20 mmol) were added sequentially under ice bath condition and stirred for 1 hour. After the reaction was completed, dilute hydrochloric acid was added to adjust pH to 5-6. The mixture was washed with water, extracted with ethyl acetate, the organic phase was collected, washed with saturated brine, dried over anhydrous sodium sulfate, filtered, and concentrated under reduced pressure. The crude product was purified by column chromatography (petroleum ether/ethyl acetate = 0-50%) to obtain compound 2-7 (2.22 g, 61% yield over two steps).

LC-MS (ESI⁺) m/z: 454.3 (M+H)⁺.

### Step 6: Synthesis of intermediate 2-8

Under nitrogen atmosphere, a solution of compound 2-7 (400 mg, 0.90 mmol), compound 1-4 (310 mg, 1.40 mmol), copper(I) iodide (35 mg, 0.18 mmol), trans-(1R,2R)-N,N-dimethylcyclohexanediamine (51 mg, 0.36 mmol), and potassium carbonate (375 mg, 2.70 mmol) in N-methylpyrrolidone (8 mL) was heated to 130°C for 3 hours. After the reaction was completed, the mixture was cooled to room temperature, washed with water, extracted with ethyl acetate, the organic phase was collected, washed with saturated brine, dried over anhydrous sodium sulfate, filtered, and concentrated under reduced pressure. The crude product was purified by column chromatography (methanol/dichloromethane = 0-5%) to obtain compound 2-8 (102 mg, 19%).

LC-MS (ESI⁺) m/z: 602.4 (M+H)⁺.

### Step 7: Synthesis of intermediate B

Under ice bath condition, hydrochloric acid/dioxane solution (2 mL, 4 N) was added to a solution of compound 2-8 (102 mg, 0.17 mmol) in dichloromethane (6 mL) and reacted for 1 hour. After the reaction was completed, the mixture was concentrated under reduced pressure to obtain crude intermediate B (120 mg), which was used directly for the next step without further purification.

LC-MS (ESI⁺) m/z: 502.2 (M+H)⁺.

### Synthesis of intermediate C:

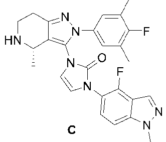

### Intermediate C was synthesized via the following route:

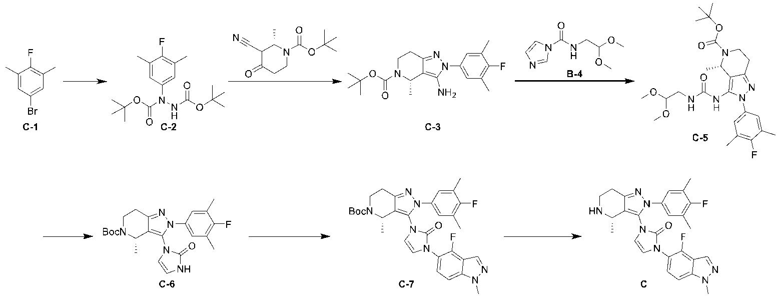

### Step 1: Synthesis of intermediate C-2

Compound 3,5-dimethyl-4-fluorobromobenzene (15 g, 73.89 mmol) was added to a 1000 mL three-necked flask, purged with N₂ three times, dissolved in tetrahydrofuran (150 mL), and cooled to -78°. Then n-butyllithium (2.5M, 29.5 mL, 73.89 mmol) was added to the reaction system and stirred for 1 h. Finally, a solution of di-tert-butyl azodicarboxylate (16.99 g, 73.89 mmol) dissolved in tetrahydrofuran (150 mL) was added dropwise to the above reaction solution, and after completion of the addition, the mixture was moved to -40°C and stirred for an additional 0.5 h. The mixture was slowly warmed to room temperature and reacted for 2 h. After the reaction was completed, ammonium chloride solution was added. The mixture was extracted with ethyl acetate (200 mL × 3), the combined organic phases were washed with saturated brine (200 mL × 1), dried over Na₂SO₄, filtered, and concentrated under reduced pressure. The crude product was purified by column chromatography (petroleum ether:ethyl acetate = 10:1) to obtain compound C-2 (11.7 g, 45%).

¹H NMR (400 MHz, CDCl₃) δ 6.98 (s, 2H), 6.65 (s, 1H), 2.16 (d, J = 2.0 Hz, 6H), 1.42 (s, 18H).

### Step 2: Synthesis of intermediate C-3

Compound C-2 (2.02 g, 8.46 mmol) and methanesulfonic acid (1.63 g, 16.92 mmol, 2.0 eq) were dissolved in NMP (20 mL), and the reaction mixture was stirred at 80°C for 12 h. After cooling to room temperature, toluene (10 mL) was added, and the pH was adjusted to 9 with potassium carbonate solution to obtain the organic phase. Tert-butyl (2S)-3-cyano-2-methyl-4-oxopiperidine-1-carboxylate (3 g, 8.46 mmol) and pyridine hydrochloride (98 mg, 0.85 mmol) were sequentially added to the above solution and heated to 90°C with stirring for 1 h. After LCMS detection showed the reaction was complete, water (50 mL) was added, the pH was adjusted to 9 with NaOH solution. The mixture was extracted with ethyl acetate (30 mL × 3), washed with saturated brine (20 mL × 3), dried over anhydrous sodium sulfate, filtered, and concentrated under reduced pressure. The crude product was purified by silica gel column chromatography (petroleum ether:ethyl acetate = 4:1) to obtain compound C-3 (2.2 g, 69%).

LC-MS (ESI⁺) m/z: 375.2 (M+H)⁺.

### Step 3: Synthesis of intermediate C-4

Compound CDI (38.56 g, 237.78 mmol) was added to a 2 L three-necked flask, purged with N2, and dissolved in ethyl acetate (800 mL). The reaction mixture was then cooled to 0°C, and aminoacetaldehyde dimethanol (25 g, 237.78 mmol) was slowly added dropwise. After completion of addition, the mixture was warmed to room temperature and stirred for 3 h. After LCMS monitoring indicated completion of the reaction, the mixture was washed with water (200 mL), followed by saturated brine (100 mL), dried over anhydrous sodium sulfate, filtered, and concentrated under reduced pressure to obtain compound C-4 (23.5 g, 49.6%).

¹H NMR (400 MHz, DMSO-d₆) δ 8.70 (t, J = 5.5 Hz, 1H), 8.28 (s, 1H), 7.74 - 7.70 (m, 1H), 7.06 - 7.04 (m, 1H), 4.52 (t, J = 5.4 Hz, 1H), 3.36 (t, J = 5.6 Hz, 2H), 3.32 (s, 6H).

### Step 4: Synthesis of intermediate C-5

Compound C-3 (1.5 g, 4.01 mmol) and C-4 (1.04 g, 5.21 mmol) were dissolved in DMA (20 mL), then potassium tert-butoxide (1.35 g, 12.03 mmol) was added to the reaction mixture and stirred at room temperature for 2 h. After LCMS monitoring indicated completion of the reaction, water was added to dilute the mixture, followed by extraction with ethyl acetate (50 mL × 3). The combined organic phases were washed with saturated brine (50 mL × 2), dried over anhydrous Na2SO4, filtered, and concentrated under reduced pressure to obtain compound C-5 (2 g, 99%).

LC-MS (ESI⁺) m/z: 506.0 (M+H)⁺.

### Step 5: Synthesis of intermediate C-6

To a solution of compound C-5 (2 g, 3.96 mmol) in toluene (20 mL), methanesulfonic acid (300 mg, 3.17 mmol, 0.8 eq) was added and the mixture was reacted at 60°C for 2 h. After completion of the reaction, saturated sodium bicarbonate solution (30 mL) was added to the reaction mixture, followed by extraction with ethyl acetate (30 mL × 3). The organic phase was washed with saturated brine (30 mL), dried over anhydrous sodium sulfate, filtered and concentrated. The crude product was purified by normal phase chromatography (petroleum ether:ethyl acetate = 1:1) to obtain compound C-6 (1.12 g, 64%).

LC-MS (ESI⁺) m/z: 442.0 (M+H)⁺;
¹H NMR (400 MHz, DMSO-d₆) δ 10.34 (s, 1H), 7.07 (d, J = 6.3 Hz, 2H), 6.58 (dd, J = 11.1, 8.6 Hz, 2H), 5.05 (br.s, 1H), 4.20 (br.s, 1H), 3.30 (d, J = 2.6 Hz, 1H), 3.10 (br.s, 2H), 2.70 - 2.60 (m, 2H), 2.19 (d, J = 1.6 Hz, 6H), 1.43 (s, 9H), 1.13 (d, J = 6.3 Hz, 3H).

### Step 6: Synthesis of intermediate C-7

To a solution of compound C-6 (1.12 g, 2.54 mmol) in NMP (10 mL), 5-bromo-4-fluoro-1-methyl-1H-indazole (1.16 g, 5.08 mmol), trans-N,N'-dimethyl-1,2-cyclohexanediamine (180 mg, 1.27 mmol), copper(I) iodide (97 mg, 0.51 mmol), and potassium carbonate (1.05 g, 7.62 mmol) were added. The mixture was reacted at 130°C under nitrogen protection for 3 h. After completion of the reaction, water (50 mL) was added to the reaction mixture. The mixture was extracted with ethyl acetate (30 mL × 3), washed with saturated brine (50 mL), dried over anhydrous sodium sulfate, filtered and concentrated. The crude product was purified by normal phase chromatography (petroleum ether:ethyl acetate = 2:1) to obtain compound C-7 (1.28 g, 86%).

LC-MS (ESI⁺) m/z: 590.0 (M+H)⁺.

### Step 7: Synthesis of intermediate C

To a solution of compound C-7 (1.18 g, 2.00 mmol) in ethyl acetate (10 mL), hydrogen chloride in ethyl acetate (20 mL) was added and the mixture was reacted at room temperature for 12 h. After completion of the reaction, the reaction mixture was concentrated, saturated sodium bicarbonate solution (20 mL) was added, and the pH was adjusted to alkaline. The mixture was then extracted with ethyl acetate (20 mL × 3). The combined organic phases were washed with saturated brine (30 mL), dried over anhydrous sodium sulfate, filtered and concentrated to obtain compound C (960 mg, 98%).

LC-MS (ESI⁺) m/z: 490.0 (M+H)⁺.

### Preparation of Specific Compounds

### Example 1: Synthesis of compounds 1a and 1b

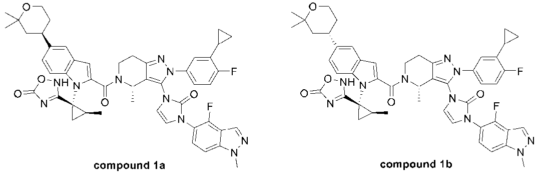

Compounds 1a and 1b were synthesized according to the following route: 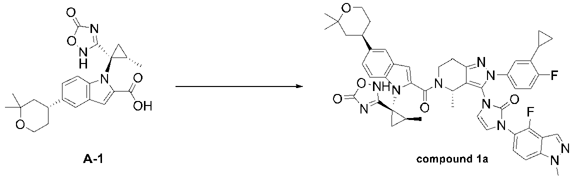 and

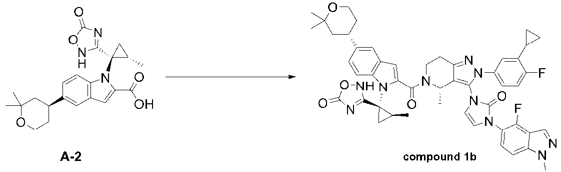

Under ice bath condition, HATU (57 mg, 0.15 mmol) was added to a solution of intermediate A-1 (41 mg, 0.10 mmol) and DIPEA (40 mg, 0.30 mmol) in N,N-dimethylformamide and stirred for 30 minutes, followed by the addition of intermediate B (50 mg, 0.1 mmol). The mixture was stirred at room temperature for 1 hour. After completion of the reaction, the mixture was washed with water, extracted with dichloromethane, and the organic phase was collected, dried over anhydrous sodium sulfate, and filtered. The crude product was purified by preparative HPLC to obtain compound 1a (36 mg, 40%).

LC-MS (ESI⁺) m/z: 895.53 (M+H)⁺.

Compound 1b was synthesized from intermediate A-2 and intermediate B following the procedure for 1a, yielding compound 1b (25 mg, 28%).

LC-MS (ESI⁺) m/z: 895.44 (M+H)⁺.

### Compound 1a:

¹H NMR (400 MHz, DMSO-d₆) δ 11.77 (s, 1H), 8.30 (s, 1H), 7.64 (d, J = 8.8 Hz, 1H), 7.53 (s, 1H), 7.46 (t, J = 7.8 Hz, 1H), 7.40 (d, J = 8.6 Hz, 1H), 7.31 (d, J = 8.5 Hz, 2H), 7.26 (d, J = 8.4 Hz, 2H), 7.10 (d, J = 3.0 Hz, 1H), 7.03-6.82 (m, 3H), 5.57 (d, J = 7.1 Hz, 1H), 4.37 (d, J = 13.6 Hz, 1H), 4.11 (s, 3H), 3.71 (d, J = 8.4 Hz, 2H), 3.02 (d, J = 12.3 Hz, 1H), 2.89 (d, J = 15.0 Hz, 1H), 2.08 (d, J = 5.7 Hz, 1H), 1.79 (s, 1H), 1.64 (d, J = 23.9 Hz, 5H), 1.43 (d, J = 6.5 Hz, 2H), 1.27 (s, 6H), 1.17 (d, J = 6.8 Hz, 6H), 1.00 (s, 2H), 0.65 (s, 2H).

### Compound 1b:

¹H NMR (400 MHz, DMSO-d₆) δ 11.75 (s, 1H), 8.30 (s, 1H), 7.64 (d, J = 8.9 Hz, 1H), 7.53 (s, 1H), 7.46 (t, J = 7.9 Hz, 1H), 7.40 (d, J = 8.6 Hz, 1H), 7.31 (d, J = 8.5 Hz, 2H), 7.26 (d, J = 8.4 Hz, 2H), 7.10 (s, 1H), 6.95 (d, J = 18.1 Hz, 3H), 5.57 (d, J = 7.3 Hz, 1H), 4.38 (d, J = 13.8 Hz, 1H), 4.10 (d, J = 12.2 Hz, 3H), 3.71 (d, J = 8.5 Hz, 2H), 3.10-2.97 (m, 1H), 2.89 (t, J = 7.5 Hz, 1H), 2.09 (s, 1H), 1.78 (s, 1H), 1.65 (dd, J = 14.5, 8.4 Hz, 5H), 1.43 (d, J = 6.5 Hz, 2H), 1.27 (s, 6H), 1.18 (s, 6H), 1.01 (d, J = 8.9 Hz, 2H), 0.65 (s, 2H).

### Compounds 2 to 4 were prepared according to the preparation method of Example 1.

| **Compound No.** | **Structure** | LC-MS (ESI⁺) m/z: (M+H)⁺ | **¹H NMR (400 MHz)** |
|---|---|---|---|
| **2** | 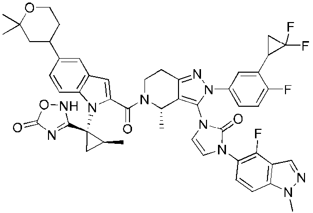 | 931.1 | ¹H NMR (400 MHz, DMSO-d₆) δ 11.74 (s, 1H), 8.30 (s, 1H), 7.63 (d, J = 8.9 Hz, 1H), 7.53 (s, 1H), 7.42 (dd, J = 17.8, 8.4 Hz, 5H), 7.26 (d, J = 8.7 Hz, 1H), 7.09 (s, 1H), 6.97 (d, J = 22.3 Hz, 2H), 5.58 (s, 1H), 4.39 (d, J = 12.1 Hz, 1H), 4.11 (s, 3H), 3.71 (d, J = 7.6 Hz, 2H), 3.19-3.00 (m, 3H), 2.91 (d, J = 15.3 Hz, 1H), 2.07 (s, 1H), 1.84 (s, 2H), 1.64 (d, J = 15.1 Hz, 5H), 1.43 (s, 2H), 1.29 (d, J = 14.5 Hz, 6H), 1.18 (s, 6H). |
| **3** | 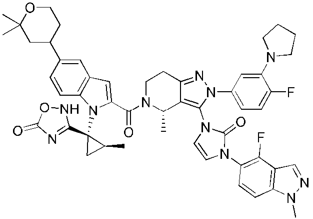 | 924.1 | ¹H NMR (400 MHz, DMSO-d₆) δ 12.04 (d, J = 228.3 Hz, 1H), 8.30 (s, 1H), 7.68-7.50 (m, 2H), 7.49-7.36 (m, 2H), 7.19 (dd, J = 52.7, 21.9 Hz, 3H), 6.83 (d, J = 86.4 Hz, 4H), 5.66 (d, J = 72.8 Hz, 1H), 4.35 (s, 1H), 4.11 (s, 3H), 3.67 (d, J = 26.6 Hz, 3H), 3.03 (s, 3H), 2.89 (s, 1H), 2.73 (s, 1H), 1.87 (s, 6H), 1.62 (dd, J = 69.9, 26.1 Hz, 7H), 1.44 (s, 2H), 1.27 (s, 5H), 1.11 (d, J = 51.1 Hz, 6H). |
| **4** | 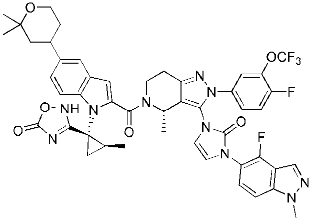 | 939.3 | ¹H NMR (400 MHz, DMSO-d₆) δ 11.74 (s, 1H), 8.29 (s, 1H), 7.77-7.70 (m, 1H), 7.64 (d, J = 8.8Hz, 1H), 7.61-7.57 (m, 2H), 7.53 (s, 1H), 7.47-7.43 (m, 1H), 7.40 (d, J = 8.8 Hz, 1H), 7.26 (d, J = 8.8 Hz, 1H), 7.14 (s, 1H), 7.07 (s, 1H), 6.94 (s, 1H), 5.60 (d, J = 6.8 Hz, 1H), 4.39 (d, J = 12.0 Hz, 1H), 4.11 (s, 3H), 3.71 (d, J = 8.0 Hz, 2H), 3.03 (t, J = 12.4 Hz, 2H), 2.93 (d, J = 15.2 Hz, 2H), 1.83-1.76 (m, 1H), 1.73-1.61 (m, 6H), 1.57-1.47 (m, 3H), 1.42 (d, J = 5.6 Hz, 3H), 1.27 (s, 3H), 1.18 (s, 3H). |

### Example 2: Synthesis of Compound 5

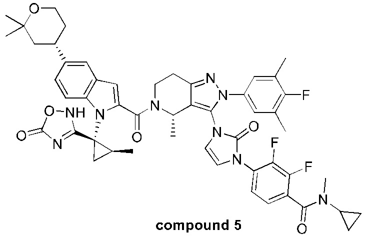

### Compound 5 was synthesized via the following route:

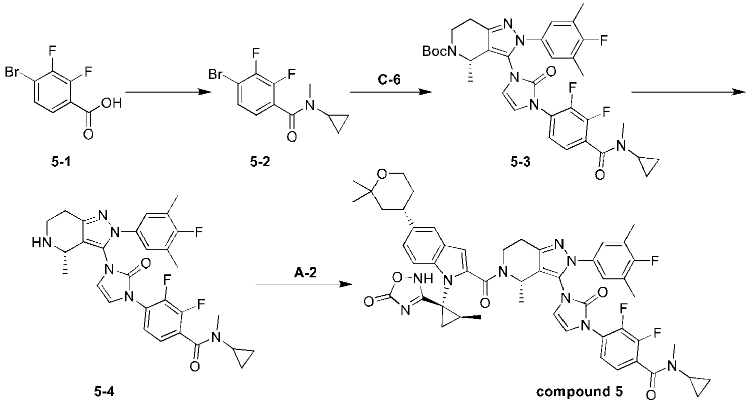

### Step 1: Synthesis of Compound 5-2

Compound 5-1 (10.0 g, 42.4 mmol) and N-methylcyclopropylamine (3.14 g, 44.3 mmol) were dissolved in N,N-dimethylformamide (150 mL), followed by sequential addition of 2-(7-azobenzotriazole)-N,N,N',N'-tetramethyluronium hexafluorophosphate (20.7 g, 54.2 mmol) and N,N-diisopropylethylamine (16.4 g, 127.2 mmol). The reaction mixture was stirred at room temperature for 1 hour. Water (200 mL) was added to dilute the reaction mixture, which was then extracted with ethyl acetate (150 mL × 3). The organic phase was washed with saturated brine (150 mL × 3), dried over anhydrous sodium sulfate, filtered, and concentrated. The crude product was purified by silica gel column chromatography (petroleum ether/ethyl acetate = 4/1) to afford the target compound 5-2 (8.1 g, yield 66%).

LC-MS (ESI⁺) m/z: 290.1 (M+H)⁺;
¹H NMR (400 MHz, CDCl₃) δ 7.42 - 7.37 (m, 1H), 7.10 - 7.04 (m, 1H), 3.14 (s, 3H), 2.81 (s, 1H), 0.67 - 0.58 (m, 2H), 0.54 - 0.43 (m, 2H).

### Step 2: Synthesis of Compound 5-3

Compound C-6 (5.0 g, 11.32 mmol) and 5-2 (3.3 g, 11.32 mmol) were dissolved in N-methylpyrrolidone (50 mL), followed by sequential addition of N,N'-dimethyl-1,2-cyclohexanediamine (0.8 g, 5.62 mmol), copper(I) iodide (0.4 g, 2.25 mmol), and potassium carbonate (4.7 g, 34.01 mmol). The reaction mixture was stirred at 130°C under nitrogen atmosphere for 3 hours. After cooling to room temperature, water (100 mL) was added to dilute the reaction mixture, which was then extracted with ethyl acetate (150 mL × 3). The combined organic phases were washed with saturated brine (100 mL × 3), dried over anhydrous sodium sulfate, filtered, and concentrated to obtain the crude product. The crude product was purified by silica gel column chromatography (petroleum ether: ethyl acetate = 2:1~9:1) followed by reverse-phase chromatography (acetonitrile/0.05% formic acid containing water= 80%-100%) to afford the target compound 5-3 (5.0 g, yield 68%).

LC-MS (ESI⁺) m/z: 651.4 (M+H)⁺.

### Step 3: Synthesis of Compound 5-4

Compound 5-3 (5.0 g, 8.45 mmol) was dissolved in a solution of hydrogen chloride in 1,4-dioxane (4M, 30 mL), and the reaction mixture was stirred at room temperature for 30 minutes. The reaction mixture was concentrated to remove most of the solvent, and saturated sodium bicarbonate solution (50 mL) was added to the residue to adjust the pH to 9. The mixture was extracted with ethyl acetate (100 mL × 3), and the combined organic phases were washed with saturated brine (100 mL × 3), dried over anhydrous sodium sulfate, filtered, and concentrated to obtain the crude product 5-4 (4.4 g), which was used in the next step without further purification.

LC-MS (ESI⁺) m/z: 551.3 (M+H)⁺.

### Step 4: Synthesis of Compound 5

Compound 5-4 (3.2 g, 5.81 mmol) and compound A-2 (2.4 g, 5.4 mmol) were dissolved in N,N-dimethylformamide (20 mL), followed by sequential addition of 2-(7-azobenzotriazole)-N,N,N',N'-tetramethyluronium hexafluorophosphate (2.7 g, 6.99 mmol) and N,N-diisopropylethylamine (2.3 g, 17.48 mmol). The reaction mixture was stirred at room temperature for 16 hours. The reaction mixture was purified by reverse-phase chromatography (acetonitrile/ 0.05% formic acid containing water = 65%-80%) to afford the target compound 5 (3.15 g, yield 57%).

LC-MS (ESI⁺) m/z: 944.4 (M+H)⁺.

¹H NMR (400 MHz, CDCl₃) δ 11.38 - 11.18 (m, 1H), 7.63 - 7.41 (m, 3H), 7.30 - 7.27 (m, 1H), 7.18 - 7.06 (m, 2H), 6.72 - 6.48 (m, 2H), 6.38 - 6.09 (m, 1H), 5.81 - 5.18 (m, 1H), 4.89 - 4.44 (m, 1H), 3.91 - 3.80 (m, 2H), 3.65 - 3.34 (m, 1H), 3.29 - 3.05 (m, 4H), 3.04 - 2.92 (m, 2H), 2.88 - 2.75 (m, 1H), 2.29 - 2.22 (m, 6H), 1.95 - 1.81 (m, 1H), 1.78 - 1.63 (m, 8H), 1.54 - 1.44 (m, 2H), 1.35 - 1.33 (m, 3H), 1.29 - 1.26 (m, 3H), 1.21 - 1.04 (m, 3H), 0.84 - 0.43 (m, 4H).

### Compounds 6 to 55 were prepared according to the preparation method of Example 2:

| **Compound No.** | **Structure** | **LC-MS (ESI⁺) m/z: (M+H)⁺** | **¹H NMR (400 MHz)** |
|---|---|---|---|
| **6** | 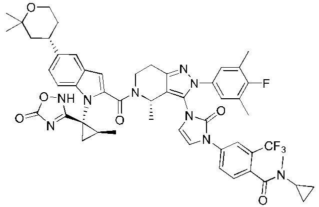 | 977.0 | ¹H NMR (400 MHz, CDCl₃) δ 11.30 - 11.23 (m, 1H), 8.01 - 7.63 (m, 2H), 7.60 - 7.45 (m, 3H), 7.25 - 7.21 (m, 1H), 7.12 - 7.00 (m, 2H), 6.81 - 6.65 (m, 2H), 6.38 - 6.15 (m, 1H), 5.78 - 5.22 (m, 1H), 4.94 - 4.35 (m, 1H), 3.89 - 3.57 (m, 3H), 3.16 - 2.97 (m, 6H), 2.89 - 2.58 (m, 2H), 2.27 - 2.21 (m, 6H), 1.91 - 1.78 (m, 2H), 1.54 - 1.52 (m, 2H), 1.35 - 1.33 (m, 3H), 1.30 - 1.23 (m, 6H), 1.21 - 1.14 (m, 3H), 1.07 - 0.75 (m, 3H), 0.59 - 0.49 (m, 3H). |
| **7** | 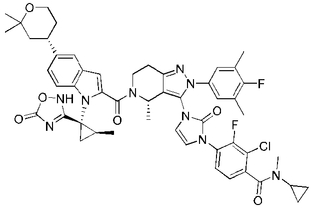 | 960.4 | ¹H NMR (400 MHz, CDCl₃) δ 11.33 - 11.21 (m, 1H), 7.78 - 7.37 (m, 3H), 7.24 - 7.20 (m, 1H), 7.13 - 7.01 (m, 2H), 6.70 - 6.50 (m, 2H), 6.33 - 6.11 (m, 1H), 5.81 - 5.21 (m, 1H), 4.90 - 4.43 (m, 1H), 3.88 - 3.38 (m, 3H), 3.15 - 2.99 (m, 5H), 2.85 - 2.72 (m, 1H), 2.28 - 2.23 (m, 6H), 1.96 - 1.71 (m, 5H), 1.65 - 1.61 (m, 3H), 1.57 - 1.50 (m, 4H), 1.35 - 1.27 (m, 6H), 1.20 - 1.03 (m, 3H), 0.95 - 0.77 (m, 1H), 0.62 - 0.52 (m, 3H). |
| **8** | 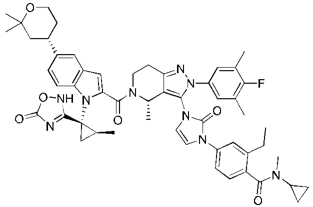 | 936.5 | ¹H NMR (400 MHz, CDCl₃) δ 11.30 - 11.28 (m, 1H), 7.59 - 7.57 (m, 1H), 7.53 - 7.51 (m, 2H), 7.48 - 7.47 (m, 1H), 7.32 - 7.31 (m, 1H), 7.14 - 7.12 (m, 2H), 6.74 - 6.69 (m, 2H), 6.31 - 6.09 (m, 1H), 5.78 - 5.27 (m, 1H), 4.96 - 4.23 (m, 1H), 3.88 - 3.85 (m, 2H), 3.61 - 3.57 (m, 1H), 3.12 - 3.07 (m, 5H), 2.70 - 2.65 (m, 3H), 2.25 - 2.21 (m, 6H), 1.77 - 1.74 (m, 4H), 1.64 - 1.62 (m, 5H), 1.58 - 1.57 (m, 4H), 1.55 - 1.54 (m, 3H), 1.35 - 1.34 (m, 3H), 1.27 - 1.27 (m, 2H), 1.19 - 1.17 (m, 3H), 0.91 - 0.83 (m, 2H), 0.54 - 0.52 (m, 2H). |
| **9** | 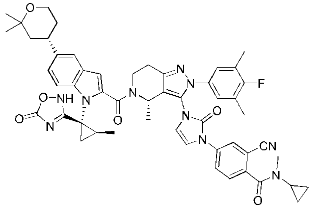 | 933.5 | ¹H NMR (400 MHz, CDCl₃) δ 11.31 - 11.20 (m, 1H), 8.08 - 7.93 (m, 2H), 7.68 - 7.46 (m, 4H), 7.12 - 7.00 (m, 2H), 6.85 - 6.72 (m, 1H), 6.71 - 6.65 (m, 1H), 6.64 - 6.17 (m, 1H), 5.83 - 5.20 (m, 1H), 4.84 - 4.44 (m, 1H), 3.90 - 3.82 (m, 2H), 3.63 - 3.54 (m, 1H), 3.20 - 3.01 (m, 6H), 2.92 - 2.88 (m, 1H), 2.28 - 2.22 (m, 6H), 1.95 - 1.87 (m, 2H), 1.76 - 1.73 (m, 2H), 1.67 - 1.65 (m, 2H), 1.60 - 1.58 (m, 2H), 1.55 - 1.49 (m, 5H), 1.34 - 1.27 (m, 6H), 1.18 - 1.07 (m, 2H), 0.65 - 0.53 (m, 2H). |
| **10** | 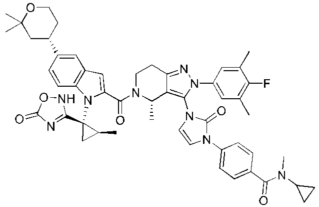 | 908.7 | ¹H NMR (400 MHz, CDCl₃) δ 11.31 (s, 1H), 7.66 (s, 3H), 7.58 (t, J = 7.6 Hz, 1H), 7.50 (d, J = 14.3 Hz, 2H), 7.11 (s, 2H), 6.77 (s, 1H), 6.69 (d, J = 9.5 Hz, 1H), 6.32 (d, J = 3.0 Hz, 1H), 5.78 (d, J = 6.2 Hz, 1H), 4.46 (d, J = 12.9 Hz, 1H), 3.87 (s, 2H), 3.09 (d, J = 13.8 Hz, 3H), 3.03 (s, 3H), 2.86 (s, 1H), 2.25 (d, J = 1.7 Hz, 6H), 2.21 (s, 2H), 1.81 - 1.70 (m, 4H), 1.70 - 1.60 (m, 3H), 1.34 (s, 3H), 1.33 (s, 1H), 1.28 (s, 3H), 1.26 (s, 2H), 1.17 (s, 3H), 0.68 (s, 2H), 0.53 (s, 2H). |
| **11** | 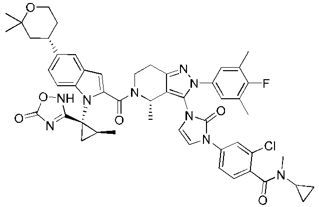 | 942.4 | ¹H NMR (400 MHz, CDCl₃) δ 11.30 - 11.22 (m, 1H), 7.75 - 7.58 (m, 2H), 7.58 - 7.45 (m, 2H), 7.42 - 7.30 (m, 1H), 7.28 - 7.26 (m, 1H), 7.12 - 6.99 (m, 2H), 6.77 - 6.60 (m, 2H), 6.36 - 6.12 (m, 1H), 5.79 - 5.24 (m, 1H), 4.90 - 4.44 (m, 1H), 3.93 - 3.53 (m, 3H), 3.16 - 3.09 (m, 3H), 3.05 - 2.96 (m, 2H), 2.79 - 2.66 (m, 1H), 2.27 - 2.21 (m, 6H), 1.91 - 1.82 (m, 1H), 1.80 - 1.78 (m, 2H), 1.75 - 1.71 (m, 2H), 1.68 - 1.59 (m, 2H), 1.57 - 1.49 (m, 4H), 1.35 - 1.32 (m, 3H), 1.29 - 1.26 (m, 3H), 1.20 - 1.05 (m, 3H), 0.98 - 0.75 (m, 1H), 0.60 - 0.49 (m, 3H). |
| **12** | 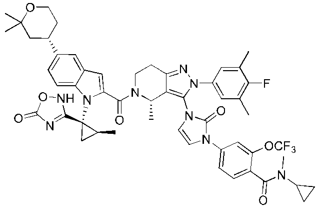 | 992.4 | ¹H NMR (400 MHz, CDCl₃) δ 7.71 (s, 1H), 7.58 (q, J = 8.2 Hz, 2H), 7.55 - 7.45 (m, 2H), 7.11 (d, J = 5.8 Hz, 2H), 6.77 (d, J = 3.0 Hz, 1H), 6.69 - 6.59 (m, 1H), 6.37 (d, J = 3.1 Hz, 1H), 5.76 (d, J = 6.3 Hz, 1H), 4.43 (d, J = 9.1 Hz, 1H), 3.86 (s, 3H), 3.57 (t, J = 11.6 Hz, 1H), 3.11 (d, J = 12.0 Hz, 3H), 2.98 (d, J = 16.2 Hz, 2H), 2.77 (d, J = 4.1 Hz, 1H), 2.25 (s, 6H), 2.20 (s, 2H), 1.76 (s, 2H), 1.73 (s, 1H), 1.63 (dd, J = 15.9, 9.9 Hz, 2H), 1.50 (dd, *J* = 19.4, 8.0 Hz, 2H), 1.34 (s, 3H), 1.28 (s, 3H), 1.25 (s, 2H), 1.18 (d, J = 6.0 Hz, 2H), 0.96 - 0.86 (m, 1H), 0.58 (d, J = 6.8 Hz, 1H), 0.48 (s, 3H). |
| **13** | 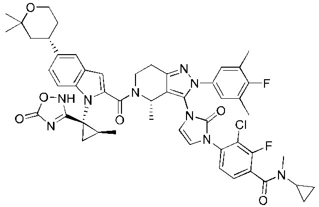 | 960.3 | ¹H NMR (400 MHz, CDCl₃) δ 11.36 - 11.23 (m, 1H), 7.62 - 7.34 (m, 4H), 7.22 - 7.12 (m, 2H), 7.11 - 7.01 (m, 1H), 6.72 - 6.66 (m, 1H), 6.57 - 6.40 (m, 1H), 6.36 - 6.14 (m, 1H), 5.82 - 5.24 (m, 1H), 5.01 - 4.32 (m, 1H), 3.90 - 3.81 (m, 2H), 3.67 - 3.42 (m, 1H), 3.15 - 3.12 (m, 2H), 3.03 - 2.80 (m, 3H), 2.32 - 2.24 (m, 6H), 1.95 - 1.88 (m, 1H), 1.84 - 1.72 (m, 4H), 1.64 - 1.54 (m, 6H), 1.36 - 1.33 (m, 3H), 1.30 - 1.28 (m, 3H), 1.26 - 1.24 (m, 2H), 1.21 - 1.19 (m, 2H), 1.08 - 0.91 (m, 2H), 0.68 - 0.55 (m, 2H). |
| **14** | 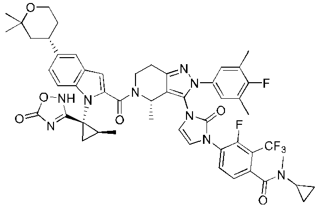 | 994.2 | ¹H NMR (400 MHz, CDCl₃) δ 11.35 - 11.23 (m, 1H), 8.04 - 7.44 (m, 3H), 7.29 - 7.26 (m, 1H), 7.25 - 7.19 (m, 1H), 7.12 - 7.00 (m, 2H), 6.71 - 6.55 (m, 2H), 6.36 - 6.13 (m, 1H), 5.82 - 5.22 (m, 1H), 4.90 - 4.43 (m, 1H), 3.91 - 3.78 (m, 2H), 3.69 - 3.24 (m, 1H), 3.19 - 2.99 (m, 5H), 2.98 - 2.79 (m, 1H), 2.76 - 2.36 (m, 1H), 2.29 - 2.21 (m, 6H), 2.02 - 1.81 (m, 1H), 1.80 - 1.72 (m, 3H), 1.68 - 1.60 (m, 3H), 1.54 - 1.51 (m, 2H), 1.35 - 1.32 (m, 3H), 1.30 - 1.24 (m, 4H), 1.21 - 1.18 (m, 2H), 1.07 - 0.77 (m, 2H), 0.66 - 0.49 (m, 3H). |
| **15** | 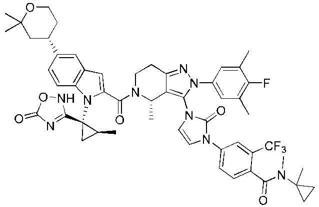 | 990.4 | ¹H NMR (400 MHz, CDCl₃) δ 11.30 - 11.22 (m, 1H), 8.04 - 7.69 (m, 2H), 7.60 - 7.56 (m, 1H), 7.54 - 7.37 (m, 2H), 7.12 - 6.99 (m, 2H), 6.81 - 6.62 (m, 2H), 6.39 - 6.14 (m, 1H), 5.77 - 5.23 (m, 1H), 4.89 - 4.45 (m, 1H), 3.89 - 3.55 (m, 3H), 3.42 - 2.98 (m, 4H), 2.77 - 2.69 (m, 2H), 2.26 - 2.21 (m, 6H), 2.09 - 1.91 (m, 1H), 1.78 - 1.71 (m, 3H), 1.55 - 1.50 (m, 4H), 1.43 - 1.34 (m, 6H), 1.30 - 1.24 (m, 6H), 1.18 - 1.05 (m, 3H), 0.96 - 0.75 (m, 4H). |
| **16** | 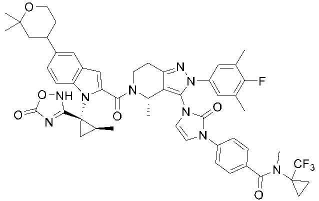 | 976.6 | ¹H NMR (400 MHz, CDCl₃) δ 11.36 - 11.20 (m, 1H), 7.70 - 7.67 (m, 1H), 7.60 - 7.51 (m, 3H), 7.45 - 7.34 (m, 1H), 7.17 - 7.01 (m, 2H), 6.80 - 6.61 (m, 2H), 6.41 - 6.12 (m, 1H), 5.83 - 5.22 (m, 1H), 5.01 - 4.42 (m, 1H), 3.91 - 3.80 (m, 2H), 3.66 - 3.38 (m, 1H), 3.20 - 2.99 (m, 6H), 2.28 - 2.19 (m, 6H), 1.95 - 1.87 (m, 1H), 1.80 - 1.70 (m, 8H), 1.60 - 1.47 (m, 5H), 1.37 - 1.33 (m, 3H), 1.32 - 1.27 (m, 3H), 1.27 - 1.23 (m, 1H), 1.22 - 1.10 (m, 4H). |
| **17** | 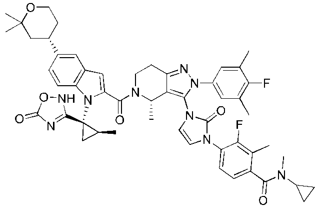 | 940.4 | ¹H NMR (400 MHz, CDCl₃) δ 11.35 - 11.26 (m, 1H), 7.61 - 7.50 (m, 3H), 7.47 - 7.42 (m, 1H), 7.15 - 7.04 (m, 3H), 6.71 - 6.69 (m, 1H), 6.61 - 6.45 (m, 1H), 6.32 - 6.07 (m, 1H), 5.80 - 4.45 (m, 1H), 3.90 - 3.83 (m, 2H), 3.67 - 3.55 (m, 1H), 3.12 - 3.01 (m, 5H), 2.82 - 2.59 (m, 2H), 2.29 - 2.23 (m, 9H), 1.92 - 1.89 (m, 1H), 1.79 - 1.76 (m, 2H), 1.74 - 1.72 (m, 1H), 1.67 - 1.63 (m, 4H), 1.56 - 1.53 (m, 4H), 1.35 - 1.33 (m, 3H), 1.29 - 1.27 (m, 3H), 1.20 - 1.18 (m, 2H), 1.07 - 1.04 (m, 1H), 0.58 - 0.50 (m, 3H). |
| 18 | 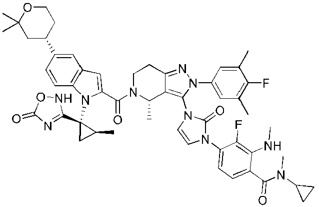 | 955.4 | ¹H NMR (400 MHz, CDCl₃) δ 11.43 - 11.18 (m, 1H), 7.64 - 7.55 (m, 1H), 7.55 - 7.50 (m, 1H), 7.26 - 7.20 (m, 1H), 7.18 - 6.99 (m, 3H), 6.88 - 6.79 (m, 1H), 6.72 - 6.65 (m, 1H), 6.58 - 6.45 (m, 1H), 6.29 - 6.03 (m, 1H), 5.78 - 5.23 (m, 1H), 4.85 - 4.45 (m, 1H), 3.91 - 3.81 (m, 2H), 3.65 - 3.53 (m, 1H), 3.10 - 3.04 (m, 3H), 3.03 - 2.98 (m, 4H), 2.96 - 2.90 (m, 1H), 2.86 - 2.79 (m, 1H), 2.31 - 2.21 (m, 6H), 2.12 - 1.99 (m, 1H), 1.93 - 1.88 (m, 1H), 1.82 - 1.71 (m, 4H), 1.69 - 1.63 (m, 2H), 1.59 - 1.51 (m, 4H), 1.36 - 1.33 (m, 3H), 1.31 - 1.27 (m, 3H), 1.21 - 1.17 (m, 2H), 1.11 - 1.03 (m, 1H), 0.74 - 0.63 (m, 2H), 0.62 - 0.51 (m, 2H). |
| **19** | 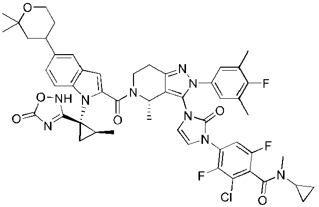 | 978.3 | ¹H NMR (400 MHz, CDCl₃) δ 11.34 - 11.19 (m, 1H), 7.63 - 7.29 (m, 3H), 7.25 - 7.21 (m, 1H), 7.12 - 7.00 (m, 2H), 6.72 - 6.54 (m, 2H), 6.37 - 6.11 (m, 1H), 5.80 - 5.18 (m, 1H), 4.90 - 4.41 (m, 1H), 3.92 - 3.76 (m, 2H), 3.65 - 3.34 (m, 1H), 3.17 - 2.87 (m, 6H), 2.84 - 2.63 (m, 1H), 2.31 - 2.20 (m, 6H), 1.93 - 1.66 (m, 5H), 1.66 - 1.64 (m, 1H), 1.55 - 1.52 (m, 3H), 1.35 - 1.27 (m, 6H), 1.21 - 1.05 (m, 3H), 0.98 - 0.42 (m, 5H). |
| **20** | 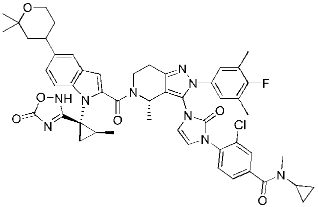 | 942.4 | ¹H NMR (400 MHz, CDCl₃) δ 11.74 - 10.87 (m, 1H), 8.02 - 7.61 (m, 1H), 7.61 - 7.56 (m, 1H), 7.55 - 7.34 (m, 3H), 7.21 - 6.55 (m, 4H), 6.45 - 6.18 (m, 1H), 6.13 - 4.43 (m, 2H), 3.90 - 3.36 (m, 3H), 3.20 - 2.97 (m, 6H), 2.87 - 2.76 (m, 1H), 2.29 - 2.23 (m, 6H), 2.19 - 2.09 (m, 3H), 1.98 - 1.82 (m, 1H), 1.80 - 1.72 (m, 3H), 1.68 - 1.64 (m, 1H), 1.57 - 1.53 (m, 3H), 1.36 - 1.33 (m, 3H), 1.29 - 1.25 (m, 3H), 1.21 - 1.04 (m, 3H), 0.79 - 0.63 (m, 2H), 0.62 - 0.44 (m, 2H). |
| **21** | 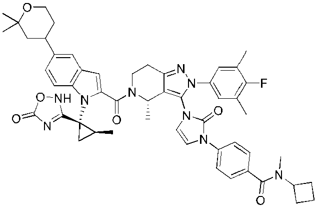 | 922.7 | ¹H NMR (400 MHz, CDCl₃) δ 11.30 (s, 1H), 7.67 (s, 1H), 7.61 - 7.56 (m, 1H), 7.54 - 7.48 (m, 2H), 7.48 - 7.30 (m, 1H), 7.15 - 7.01 (m, 2H), 6.86 - 6.55 (m, 2H), 6.24 - 5.56 (m, 1H), 4.90 - 4.45 (m, 1H), 3.90 - 3.64 (m, 2H), 3.12 - 2.95 (m, 5H), 2.39 - 2.19 (m, 8H), 2.14 - 1.92 (m, 2H), 1.91 - 1.69 (m, 6H), 1.63 - 1.56 (m, 11H), 1.36 - 1.25 (m, 7H), 1.20 - 1.05 (m, 3H). |
| **22** | 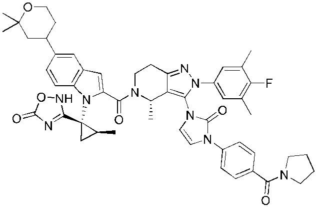 | 908.6 | ¹H NMR (400 MHz, CDCl₃) δ 11.28 (d, *J =* 24.3 Hz, 1H), 7.74 - 7.62 (m, 3H), 7.59 (d, *J* = 8.5 Hz, 1H), 7.53 - 7.51 (m, 1H), 7.13 - 7.01 (m, 2H), 6.83 - 6.64 (m, 2H), 6.22 (d, *J =* 85.9 Hz, 1H), 5.80 - 5.22 (m, 1H), 4.90 - 4.43 (m, 1H), 3.88 - 3.80 (m, 2H), 3.66 - 3.47 (m, 5H), 3.22 - 3.07 (m, 1H), 3.05 - 2.98 (m, 2H), 2.25 (s, 5H), 2.21 (s, 1H), 1.99 - 1.89 (m, 5H), 1.81 - 1.74 (m, 3H), 1.73 - 1.72 (m, 1H), 1.65 - 1.60 (m, 3H), 1.55 - 1.49 (m, 4H), 1.35 - 1.32 (m, 3H), 1.29 - 1.26 (m, 3H), 1.18 (d, *J* = 5.5 Hz, 2H), 1.08 - 0.86 (m, 1H). |
| **23** | 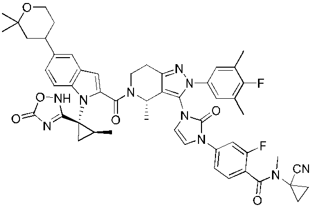 | 951.5 | ¹H NMR (400 MHz, CDCl₃) δ 11.36 - 11.15 (m, 1H), 7.79 - 7.27 (m, 6H), 7.12 - 7.00 (m, 2H), 6.81 - 6.63 (m, 2H), 6.40 - 6.14 (m, 1H), 5.81 - 5.21 (m, 1H), 4.88 - 4.45 (m, 1H), 3.99 - 3.72 (m, 2H), 3.69 - 3.35 (m, 1H), 3.24 - 2.92 (m, 6H), 2.28 - 2.19 (m, 6H), 1.91 - 1.60 (m, 10H), 1.40 - 1.25 (m, 9H), 1.21 - 0.96 (m, 4H). |
| **24** | 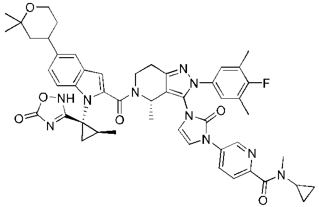 | 909.6 | ¹H NMR (400 MHz, CDCl₃) δ 11.33 - 11.15 (m, 1H), 9.08 - 8.37 (m, 1H), 8.33 - 7.77 (m, 1H), 7.60 - 7.48 (m, 2H), 7.26 - 7.23 (m, 1H), 7.14 - 7.01 (m, 2H), 7.00 - 6.73 (m, 1H), 6.71 - 6.66 (m, 1H), 6.45 - 6.15 (m, 1H), 5.82 - 5.19 (m, 1H), 4.92 - 4.44 (m, 1H), 3.91 - 3.79 (m, 2H), 3.78 - 3.32 (m, 1H), 3.22 - 2.96 (m, 7H), 2.29 - 2.20 (m, 6H), 1.92 - 1.89 (m, 1H), 1.87 - 1.68 (m, 4H), 1.68 - 1.58 (m, 2H), 1.56 - 1.45 (m, 4H), 1.35 - 1.32 (m, 3H), 1.29 - 1.25 (m, 3H), 1.19 - 1.05 (m, 3H), 0.85 - 0.33 (m, 4H). |
| **25** | 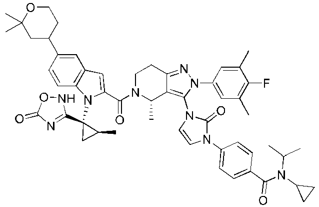 | 936.3 | ¹H NMR (400 MHz, CDCl₃) δ 11.40 - 11.01 (m, 1H), 7.60 - 7.27 (m, 6H), 7.08 - 6.92 (m, 2H), 6.73 - 6.52 (m, 2H), 6.28 - 6.01 (m, 1H), 5.72 - 5.14 (m, 1H), 4.83 - 4.35 (m, 2H), 4.05 - 3.26 (m, 4H), 3.12 (s, 3H), 2.46 (s, 1H), 2.21 - 2.09 (m, 6H), 1.85 - 1.66 (m, 4H), 1.58 - 1.53 (m, 2H), 1.50 - 1.41 (m, 4H), 1.36 - 1.28 (m, 6H), 1.27 (s, 3H), 1.21 (s, 3H), 1.12 - 1.09 (m, 2H), 1.02 - 0.77 (m, 1H), 0.64 - 0.37 (m, 4H). |
| **26** | 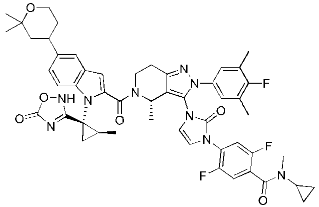 | 944.6 | ¹H NMR (400 MHz, CDCl₃) δ 11.32 - 11.25 (m, 1H), 7.61 - 7.57 (m, 1H), 7.55 - 7.49 (m, 2H), 7.30 - 7.27 (m, 1H), 7.26 - 7.21 (m, 1H), 7.12 - 7.00 (m, 2H), 6.72 - 6.57 (m, 2H), 6.33 - 6.11 (m, 1H), 5.77 - 5.25 (m, 1H), 4.85 - 4.48 (m, 1H), 3.89 - 3.82 (m, 2H), 3.64 - 3.54 (m, 1H), 3.14 - 3.09 (m, 3H), 3.04 - 2.84 (m, 4H), 2.28 - 2.22 (m, 6H), 1.93 - 1.89 (m, 1H), 1.79 - 1.72 (m, 4H), 1.67 - 1.63 (m, 2H), 1.55 - 1.52 (m, 3H), 1.34 (s, 3H), 1.28 (s, 3H), 1.20 - 1.05 (m, 3H), 0.88 - 0.53 (m, 4H). |
| **27** | 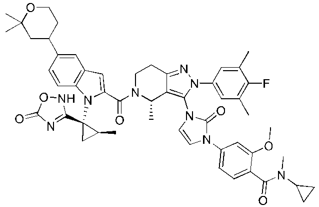 | 938.3 | ¹H NMR (400 MHz, CDCl₃) δ 11.43 - 11.15 (m, 1H), 7.68 - 7.41 (m, 3H), 7.28 (s, 1H), 7.25 - 6.98 (m, 4H), 6.87 - 6.59 (m, 2H), 6.42 - 6.00 (m, 1H), 5.86 - 5.22 (m, 1H), 4.95 - 4.37 (m, 1H), 3.88 (d, *J =* 14.0 Hz, 4H), 3.74 - 3.31 (m, 2H), 3.16 - 2.95 (m, 5H), 2.83 - 2.66 (m, 1H), 2.36 - 2.14 (m, 6H), 1.90 (s, 1H), 1.85 - 1.67 (m, 8H), 1.65 - 1.59 (m, 2H), 1.34 (s, 3H), 1.28 (s, 3H), 1.20 - 1.02 (m, 3H), 0.94 - 0.70 (m, 1H), 0.47 (d, *J* = 27.7 Hz, 3H). |
| **28** | 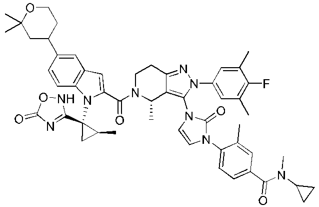 | 923.1 | ¹H NMR (400 MHz, CDCl₃) δ 11.38 - 11.21 (m, 1H), 7.97 - 7.44 (m, 4H), 7.37 - 7.27 (m, 1H), 7.24 - 7.00 (m, 3H), 6.77 - 6.52 (m, 2H), 6.40 - 6.01 (m, 1H), 5.84 - 5.20 (m, 1H), 4.89 - 4.43 (m, 1H), 3.93 - 3.78 (m, 2H), 3.74 - 3.21 (m, 1H), 3.16 - 2.96 (m, 5H), 2.40 - 2.34 (m, 2H), 2.31 - 2.16 (m, 7H), 1.95 - 1.86 (m, 1H), 1.83 - 1.61 (m, 8H), 1.53 (d, *J =* 6.4 Hz, 3H), 1.36 - 1.32 (m, 3H), 1.30 - 1.26 (m, 3H), 1.20 - 1.04 (m, 3H), 0.94 - 0.73 (m, 1H), 0.60 - 0.42 (m, 3H). |
| **29** | 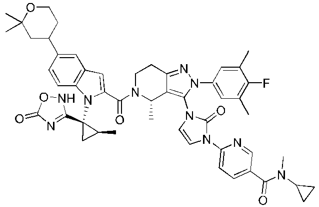 | 909.3 | ¹H NMR (400 MHz, CDCl₃) δ 11.29 (d, *J =* 24.8 Hz, 1H), 8.68 - 8.45 (m, 2H), 8.04 - 7.77 (m, 1H), 7.61 - 7.41 (m, 3H), 7.26 - 7.22 (m, 1H), 7.06 (dd, *J = 37.6,* 6.1 Hz, 2H), 6.67 (d, *J =* 19.6 Hz, 1H), 6.21 (dd, *J* = 76.4, 3.1 Hz, 1H), 5.54 (dd, *J =* 209.8, 6.6 Hz, 1H), 4.92 - 4.40 (m, 1H), 3.93 - 3.78 (m, 2H), 3.71 - 3.32 (m, 1H), 3.20 - 2.97 (m, 6H), 2.92 - 2.81 (m, 1H), 2.21 (d, *J =* 17.1 Hz, 6H), 1.93 - 1.71 (m, 4H), 1.69 - 1.64 (m, 1H), 1.55 - 1.47 (m, 4H), 1.37 - 1.21 (m, 7H), 1.11 (dd, *J =* 41.0, 5.9 Hz, 3H), 0.77 - 0.47 (m, 4H). |
| **30** | 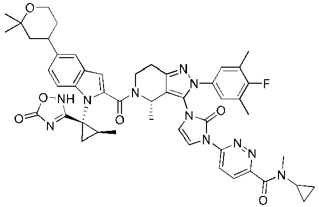 | 910.6 | ¹H NMR (400 MHz, CDCl₃) δ 11.31 - 11.21 (m, 1H), 8.89 - 7.88 (m, 2H), 7.60 - 7.57 (m, 1H), 7.52 - 7.45 (m, 1H), 7.26 - 7.25 (m, 1H), 7.11 - 6.99 (m, 2H), 6.71 - 6.64 (m, 1H), 6.40 - 6.19 (m, 1H), 5.83 - 5.26 (m, 1H), 4.90 - 4.45 (m, 1H), 3.90 - 3.80 (m, 2H), 3.63 - 3.36 (m, 1H), 3.25 - 3.19 (m, 3H), 3.19 - 3.08 (m, 2H), 3.06 - 2.99 (m, 2H), 2.26 - 2.20 (m, 6H), 1.92 - 1.83 (m, 1H), 1.80 - 1.73 (m, 3H), 1.67 - 1.59 (m, 7H), 1.54 - 1.47 (m, 4H), 1.35 - 1.33 (m, 3H), 1.29 - 1.25 (m, 3H), 1.18 - 1.06 (m, 3H), 0.69 - 0.54 (m, 2H), 0.51 - 0.31 (m, 2H). |
| **31** | 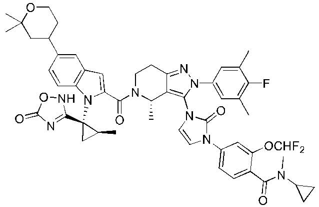 | 974.7 | ¹H NMR (400 MHz, CDCl₃) δ 11.33 - 11.20 (m, 1H), 7.63 - 7.46 (m, 4H), 7.13 - 7.00 (m, 2H), 6.81 - 6.42 (m, 3H), 6.37 - 6.13 (m, 1H), 5.79 - 5.24 (m, 1H), 4.90 - 4.43 (m, 1H), 3.93 - 3.79 (m, 2H), 3.64 - 3.36 (m, 1H), 3.16 - 2.72 (m, 7H), 2.27 - 2.20 (m, 6H), 1.95 - 1.83 (m, 1H), 1.83 - 1.70 (m, 4H), 1.68 - 1.62 (m, 3H), 1.56 - 1.50 (m, 4H), 1.35 - 1.32 (m, 3H), 1.29 - 1.26 (m, 3H), 1.19 - 1.04 (m, 3H), 0.95 - 0.70 (m, 1H), 0.59 - 0.46 (m, 3H). |
| **32** | 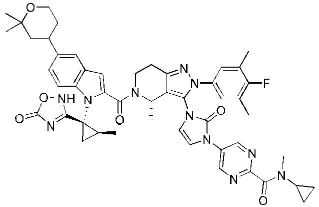 | 910.3 | ¹H NMR (400 MHz, CDCl₃) δ 11.23 (d, *J* = 16.3 Hz, 1H), 9.07 (d, *J =* 106.8 Hz, 2H), 7.57 (t, *J* = 9.4 Hz, 1H), 7.49 (d, *J* = 19.8 Hz, 1H), 7.28 - 7.26 (m, 1H), 7.15 - 6.99 (m, 2H), 6.89 - 6.65 (m, 2H), 6.36 (d, *J =* 85.4 Hz, 1H), 5.59 (dd, *J =* 107.5 Hz, 1H), 4.96 - 4.35 (m, 1H), 3.94 - 3.75 (m, 2H), 3.59 (t, *J =* 11.2 Hz, 1H), 3.20 - 3.12 (m, 3H), 3.01 (d, *J =* 13.2 Hz, 2H), 2.90 *(d, J= 26.7* Hz, 1H), 2.25 (t, *J =* 8.2 Hz, 6H), 1.98 - 1.86 (m, 1H), 1.77 (dd, *J =* 14.2, 10.0 Hz, 3H), 1.64 (dd, *J* = 15.0, 9.9 Hz, 3H), 1.53 (d, *J =* 6.4 Hz, 4H), 1.33 (d, *J =* 7.3 Hz, 3H), 1.29 - 1.25 (m, 3H), 1.12 (dd, *J=* 47.1, 5.9 Hz, 3H), 0.49 (dd, *J* = 30.8, 6.1 Hz, 4H). |
| **33** | 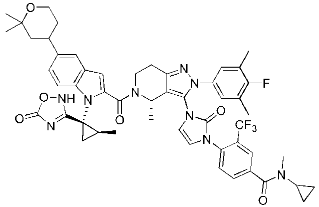 | 976.0 | ¹H NMR (400 MHz, CDCl₃) δ 11.38 - 11.06 (m, 1H), 7.99 - 7.54 (m, 2H), 7.54 - 7.33 (m, 3H), 7.18 - 6.96 (m, 3H), 6.62 (s, 1H), 6.43 (s, 1H), 6.26 - 6.02 (m, 1H), 5.73 - 5.12 (m, 1H), 4.84 - 4.29 (m, 1H), 3.84 - 3.73 (m, 2H), 3.57 - 3.26 (m, 1H), 3.09 - 2.68 (m, 7H), 2.24 - 2.17 (m, 6H), 1.88 - 1.63 (m, 5H), 1.57 (s, 2H), 1.49 - 1.44 (m, 4H), 1.27 (s, 3H), 1.21 (s, 3H), 1.15 - 1.13 (m, 2H), 0.72 - 0.40 (m, 4H). |
| **34** | 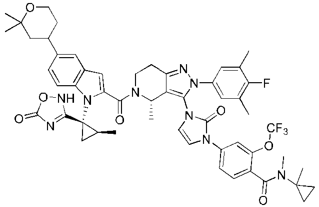 | 1006.3 | ¹H NMR (400 MHz, CDCl₃) δ 11.32 - 11.18 (m, 1H), 7.70 - 7.30 (m, 5H), 7.26 - 7.21 (m, 1H), 7.15 - 6.97 (m, 2H), 6.78 - 6.60 (m, 2H), 6.39 - 6.13 (m, 1H), 5.80 - 5.22 (m, 1H), 4.93 - 4.37 (m, 1H), 3.91 - 3.78 (m, 2H), 3.65 - 3.35 (m, 1H), 3.17 - 2.76 (m, 6H), 2.26 - 2.19 (m, 6H), 1.92 - 1.60 (m, 8H), 1.54 - 1.47 (m, 3H), 1.43 - 1.40 (m, 2H), 1.35 - 1.32 (m, 3H), 1.29 - 1.26 (m, 3H), 1.20 - 1.04 (m, 3H), 0.95 - 0.41 (m, 4H). |
| **35** | 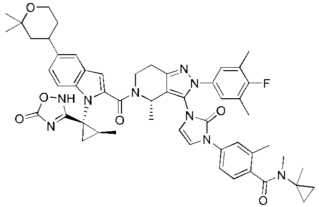 | 936.5 | ¹H NMR (400 MHz, CDCl₃) δ 11.46 - 11.02 (m, 1H), 7.62 - 7.52 (m, 1H), 7.52 - 7.27 (m, 3H), 7.24 - 7.18 (m, 1H), 7.13 - 7.01 (m, 2H), 6.75 - 6.49 (m, 2H), 6.33 - 6.06 (m, 1H), 5.79 - 5.23 (m, 1H), 4.96 - 4.33 (m, 1H), 4.05 - 3.27 (m, 3H), 3.23 - 2.89 (m, 4H), 2.84 - 2.68 (m, 2H), 2.32 (s, 2H), 2.28 - 2.14 (m, 7H), 1.91 - 1.72 (m, 5H), 1.64 (t, *J =* 11.2 Hz, 3H), 1.56 - 1.47 (m, 4H), 1.45 - 1.41 (m, 2H), 1.34 - 1.27 (m, 6H), 1.20 - 1.04 (m, 3H), 0.97 - 0.46 (m, 4H). |
| **36** | 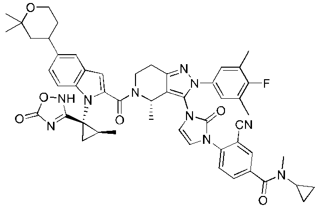 | 933.5 | ¹H NMR (400 MHz, CDCl₃) δ 11.41 - 11.14 (m, 1H), 8.09 - 7.27 (m, 6H), 7.16 - 7.00 (m, 2H), 6.85 - 6.65 (m, 2H), 6.42 - 6.14 (m, 1H), 5.85 - 5.30 (m, 1H), 5.17 - 4.29 (m, 1H), 3.93 - 3.73 (m, 2H), 3.67 - 3.34 (m, 1H), 3.27 - 2.96 (m, 6H), 2.91 - 2.79 (m, 1H), 2.33 - 2.18 (m, 6H), 1.92 - 1.61 (m, 10H), 1.37 - 1.27 (m, 6H), 1.21 - 1.05 (m, 3H), 0.80 - 0.46 (m, 4H). |
| **37** | 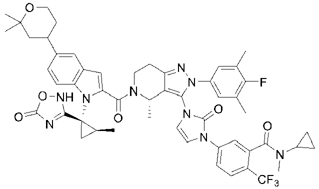 | 976.2 | ¹H NMR (400 MHz, CDCl₃) δ 11.26 (d, *J =* 8.6 Hz, 1H), 7.86 - 7.71 (m, 2H), 7.58 (d, *J* = 8.5 Hz, 1H), 7.49 (d, *J =* 17.4 Hz, 1H), 7.26 (d, *J=* 3.4 Hz, 2H), 7.05 (dd, *J =* 40.3, 6.1 Hz, 2H), 6.83 - 6.62 (m, 2H), 6.31 (t, *J =* 43.1 Hz, 1H), 5.50 (dd, *J =* 204.1, 6.5 Hz, 1H), 4.87 - 4.44 (m, 1H), 3.92 - 3.77 (m, 2H), 3.64 - 3.34 (m, 1H), 3.16 - 2.97 (m, 5H), 2.91 - 2.39 (m, 2H), 2.22 (d, *J=* 17.2 Hz, 6H), 2.03 - 1.86 (m, 1H), 1.91 (d, *J =* 4.6 Hz, 1H), 1.90 - 1.88 (m, 1H), 1.82 - 1.66 (m, 4H), 1.61 *(dd, J* = 20.2, 13.8 Hz, 4H), 1.50 *(d, J* = 6.3 Hz, 2H), 1.35 - 1.25 (m, 6H), 1.19 - 1.04 (m, 3H), 0.86 (d, *J* = 56.3 Hz, 1H), 0.61 - 0.55 (m, 2H). |
| **38** | 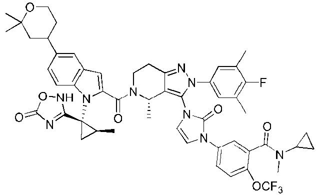 | 992.1 | ¹H NMR (400 MHz, CDCl₃) δ 11.26 (d, *J* = 16.4 Hz, 1H), 7.81 (d, *J =* 8.9 Hz, 1H), 7.60 - 7.56 (m, 2H), 7.50 (d, *J =* 12.2 Hz, 1H), 7.39 (d, *J* = 8.8 Hz, 1H), 7.10 (d, *J =* 6.3 Hz, 2H), 7.04 - 6.70 (m, 2H), 6.69 - 6.59 (m, 1H), 6.34 (s, 1H), 6.13 - 4.83 (m, 2H), 3.88 - 3.82 (m, 2H), 3.58 (t, *J =* 11.0 Hz, 1H), 3.13 (s, 2H), 3.09 (s, 1H), 3.03 - 2.97 (m, 2H), 2.90 - 2.83 (m, 1H), 2.82 - 2.76 (m, 1H), 2.26 - 2.21 (m, 6H), 1.90 (s, 1H), 1.75 *(d, J* = 13.5 Hz, 3H), 1.52 - 1.50 (m, 3H), 1.33 (d, *J =* 6.0 Hz, 3H), 1.29 - 1.27 (m, 3H), 1.26 - 1.25 (m, 1H), 1.18 (d, *J =* 5.6 Hz, 3H), 1.06 (d, *J =* 6.1 Hz, 1H), 0.85 (d, *J =* 58.9 Hz, 1H), 0.59 - 0.49 (m, 3H). |
| **39** | 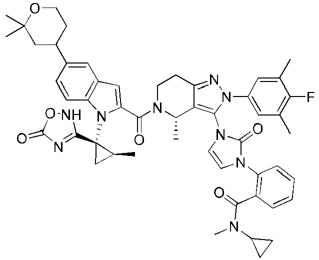 | 908.8 | ¹H NMR (400 MHz, CDCl₃) δ 11.47 - 11.10 (m, 1H), 8.03 (s, 1H), 7.77 - 7.54 (m, 2H), 7.52 (s, 2H), 7.27 (s, 1H), 7.14 (s, 2H), 7.11 - 6.79 (m, 1H), 6.71 (s, 1H), 6.51 - 5.82 (m, 1H), 5.38 - 4.29 (m, 1H), 4.23 (s, 2H), 4.06 - 3.84 (m, 2H), 3.61 (s, 1H), 3.17 (s, 3H), 3.08 - 2.97 (m, 3H), 2.26 (s, 5H), 2.22 (s, 1H), 1.80 - 1.71 (m, 4H), 1.70 - 1.60 (m, 2H), 1.55 (s, 4H), 1.41 - 1.32 (m, 4H), 1.29 - 1.25 (m, 4H), 1.19 (s, 3H), 1.09 - 0.88 (m, 1H), 0.76 - 0.09 (m, 4H). |
| **40** | 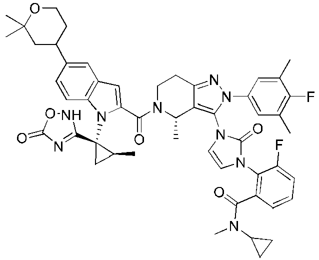 | 926.8 | ¹H NMR (400 MHz, CDCl₃) δ 11.58 - 11.04 (m, 1H), 8.05 - 7.46 (m, 2H), 7.45 - 7.28 (m, 2H), 7.20 - 6.95 (m, 3H), 6.68 (s, 1H), 6.60 - 6.26 (m, 1H), 6.23 - 5.79 (m, 1H), 5.75 (d, *J* = 7.1 Hz, 1H), 4.47 - 3.82 (m, 2H), 3.55 (s, 1H), 3.14 (s, 3H), 3.02 - 2.92 (m, 2H), 2.70 (s, 1H), 2.34 - 2.23 (m, 6H), 2.04 - 1.90 (m, 1H), 1.80 - 1.68 (m, 4H), 1.57 (s, 3H), 1.48 - 1.45 (m, 2H), 1.34 (s, 3H), 1.28 (s, 3H), 1.25 (s, 3H), 1.20 - 1.17 (m, 2H), 1.08 - 0.81 (m, 3H), 0.72 - 0.42 (m, 2H). |
| **41** | 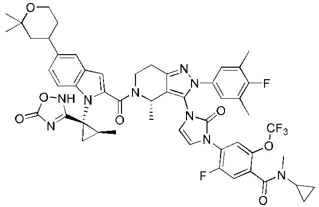 | 1010.4 | ¹H NMR (400 MHz, CDCl₃) δ 11.37 - 11.16 (m, 1H), 7.70 - 7.60 (m, 1H), 7.59 - 7.46 (m, 2H), 7.38 - 7.28 (m, 1H), 7.26 - 7.15 (m, 1H), 7.13 - 6.99 (m, 2H), 6.73 - 6.55 (m, 2H), 6.37 - 6.11 (m, 1H), 5.82 - 5.21 (m, 1H), 4.93 - 4.43 (m, 1H), 3.92 - 3.77 (m, 2H), 3.64 - 3.37 (m, 1H), 3.19 - 3.09 (m, 3H), 3.07 - 2.98 (m, 2H), 2.96 - 2.62 (m, 2H), 2.31 - 2.20 (m, 6H), 2.04 - 1.96 (m, 1H), 1.94 - 1.88 (m, 1H), 1.81 - 1.72 (m, 3H), 1.70 - 1.61 (m, 2H), 1.60 - 1.49 (m, 4H), 1.36 - 1.32 (m, 3H), 1.29 - 1.27 (m, 2H), 1.21 - 1.18 (m, 2H), 1.07 - 0.93 (m, 1H), 0.88 - 0.37 (m, 4H). |
| **42** | 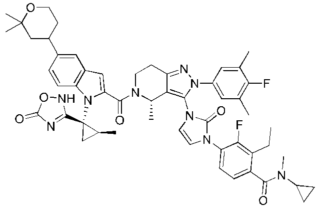 | 954.8 | ¹H NMR (400 MHz, CDCl₃) δ 11.28 (d, *J* = 22.5 Hz, 1H), 7.60 - 7.54 (m, 1H), 7.53 - 7.41 (m, 2H), 7.27 - 7.26 (m, 1H), 7.17 - 7.03 (m, 3H), 6.70 (s, 1H), 6.64 - 6.46 (m, 1H), 6.32 - 6.06 (m, 1H), 5.89 - 5.14 (m, 1H), 4.96 - 4.39 (m, 1H), 3.91 - 3.82 (m, 2H), 3.66 - 3.39 (m, 1H), 3.17 - 3.07 (m, 3H), 3.05 - 2.98 (m, 2H), 2.85 - 2.75 (m, 1H), 2.73 - 2.61 (m, 2H), 2.25 (d, *J =* 16.4 Hz, 6H), 1.96 - 1.85 (m, 1H), 1.80 - 1.76 (m, 3H), 1.73 - 1.70 (m, 2H), 1.68 - 1.61 (m, 2H), 1.57 - 1.53 (m, 3H), 1.34 (d, 3H), 1.28 (d, 3H), 1.27 - 1.23 (m, 2H), 1.21 - 1.05 (m, 4H), 0.95 - 0.76 (m, 1H), 0.65 - 0.48 (m, 3H). |
| **43** | 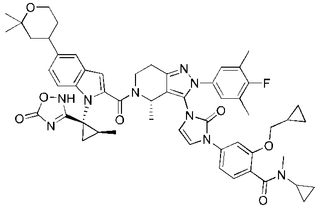 | 978.7 | ¹H NMR (400 MHz, CDCl₃) δ 11.35 - 11.21 (m, 1H), 7.64 - 7.38 (m, 3H), 7.18 - 7.06 (m, 2H), 7.05 - 6.99 (m, 1H), 6.86 - 6.73 (m, 1H), 6.72 - 6.67 (m, 1H), 6.38 - 6.11 (m, 1H), 5.80 - 5.24 (m, 1H), 5.23 - 4.37 (m, 1H), 3.95 - 3.74 (m, 4H), 3.71 - 3.50 (m, 1H), 3.25 - 3.08 (m, 3H), 3.08 - 2.88 (m, 3H), 2.88 - 2.30 (m, 2H), 2.28 - 2.20 (m, 6H), 1.92 - 1.88 (m, 1H), 1.80 - 1.72 (m, 3H), 1.68 - 1.60 (m, 2H), 1.57 - 1.49 (m, 4H), 1.36 - 1.32 (m, 3H), 1.32 - 1.28 (m, 3H), 1.28 - 1.24 (m, 2H), 1.21 - 1.11 (m, 3H), 1.10 - 0.94 (m, 1H), 0.64 - 0.58 (m, 2H), 0.56 - 0.49 (m, 2H), 0.49 - 0.38 (m, 1H), 0.36 - 0.27 (m, 2H). |
| **44** | 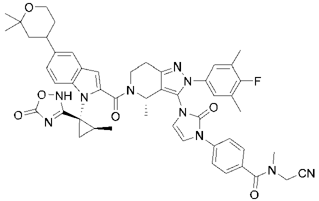 | 907.5 | ¹H NMR (400 MHz, CDCl₃) δ 11.33 - 11.23 (m, 1H), 7.76 - 7.73 (m, 1H), 7.65 - 7.44 (m, 5H), 7.13 - 7.01 (m, 2H), 6.80 - 6.64 (m, 2H), 6.36 - 6.11 (m, 1H), 5.79 - 4.90 (m, 1H), 4.50 - 4.41 (m, 2H), 3.92 - 3.81 (m, 2H), 3.64 - 3.40 (m, 1H), 3.25 - 3.02 (m, 6H), 2.27 - 2.20 (m, 6H), 2.06 - 1.90 (m, 1H), 1.83 - 1.71 (m, 4H), 1.69 - 1.66 (m, 1H), 1.64 - 1.57 (m, 6H), 1.36 - 1.33 (m, 3H), 1.30 - 1.26 (m, 3H), 1.19 - 1.06 (m, 3H). |
| **45** | 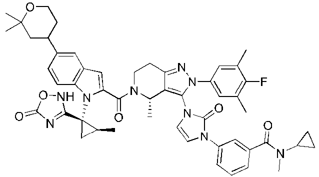 | 908.4 | ¹H NMR (400 MHz, CDCl₃) δ 11.27 (d, *J =* 14.5 Hz, 1H), 7.76 - 7.67 (m, 1H), 7.63 - 7.27 (m, 5H), 7.24 (d, *J =* 7.7 Hz, 1H), 7.13 - 6.99 (m, 2H), 6.80 - 6.59 (m, 2H), 6.21 (d, *J =* 90.3 Hz, 1H), 5.51 (dd, *J =* 205.6, 6.7 Hz, 1H), 4.67 (dd, *J =* 164.3, 9.8 Hz, 1H), 3.92 - 3.79 (m, 2H), 3.58 (t, *J =* 12.1 Hz, 1H), 3.14 - 2.97 (m, 5H), 2.81 (d, *J =* 43.9 Hz, 1H), 2.23 (t, *J =* 8.7 Hz, 6H), 1.94 - 1.86 (m, 1H), 1.81 - 1.71 (m, 3H), 1.67 - 1.62 (m, 3H), 1.56 - 1.49 (m, 4H), 1.35 - 1.25 (m, 6H), 1.12 (dd, *J =* 46.9, 5.7 Hz, 3H), 0.79 - 0.37 (m, 4H). |
| **46** | 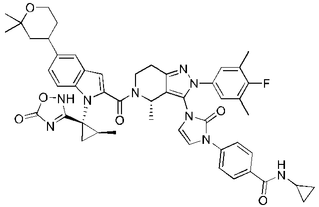 | 894.4 | ¹H NMR (400 MHz, CDCl₃) δ 11.29 (d, *J =* 23.0 Hz, 1H), 8.09 - 7.33 (m, 6H), 7.28 - 7.26 (m, 1H), 7.13 - 6.99 (m, 2H), 6.88 - 6.60 (m, 2H), 6.50 - 6.04 (m, 2H), 5.80 - 5.24 (m, 1H), 4.89 - 4.45 (m, 1H), 3.96 - 3.75 (m, 2H), 3.63 - 3.36 (m, 1H), 3.21 - 2.81 (m, 4H), 2.30 - 2.17 (m, 6H), 2.07 - 1.88 (m, 1H), 1.80 - 1.72 (m, 3H), 1.68 - 1.61 (m, 3H), 1.53 - 1.50 (m, 3H), 1.36 - 1.32 (m, 3H), 1.29 - 1.25 (m, 3H), 1.19 - 1.04 (m, 3H), 0.95 - 0.85 (m, 2H), 0.71 - 0.58 (m, 2H). |
| **47** | 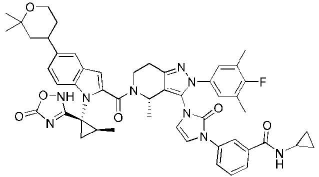 | 894.4 | ¹H NMR (400 MHz, CDCl₃) δ 11.28 (d, *J* = 16.3 Hz, 1H), 8.09 - 7.71 (m, 2H), 7.68 - 7.47 (m, 4H), 7.23 - 6.94 (m, 2H), 6.89 - 6.60 (m, 2H), 6.56 - 6.07 (m, 2H), 5.87 - 5.21 (m, 1H), 4.93 - 4.41 (m, 1H), 3.95 - 3.77 (m, 2H), 3.65 - 3.32 (m, 1H), 3.26 - 2.84 (m, 4H), 2.28 - 2.18 (m, 6H), 1.94 - 1.74 (m, 4H), 1.56 - 1.48 (m, 4H), 1.35 - 1.25 (m, 7H), 1.19 - 1.06 (m, 3H), 0.95 - 0.78 (m, 3H), 0.68 - 0.47 (m, 3H). |
| **48** | 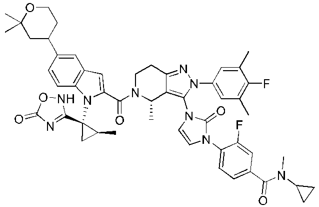 | 926.5 | ¹H NMR (400 MHz, CDCl₃) δ 11.29 (d, *J =* 23.0 Hz, 1H), 7.75 - 7.64 (m, 1H), 7.62 - 7.48 (m, 2H), 7.47 - 7.31 (m, 2H), 7.17 - 6.98 (m, 2H), 6.93 - 6.61 (m, 2H), 6.56 - 6.03 (m, 1H), 5.84 - 5.17 (m, 1H), 4.95 - 4.38 (m, 1H), 3.94 - 3.76 (m, 2H), 3.67 - 3.30 (m, 1H), 3.23 - 2.94 (m, 6H), 2.91 - 2.70 (m, 1H), 2.25 (d, *J =* 16.1 Hz, 6H), 1.91 (s, 1H), 1.83 - 1.68 (m, 4H), 1.68 - 1.64 (m, 2H), 1.58 - 1.48 (m, 4H), 1.31 (d, *J* = 23.5 Hz, 6H), 1.22 - 1.16 (m, 2H), 1.10 - 0.86 (m, 1H), 0.63 (d, *J =* 68.5 Hz, 4H). |
| **49** | 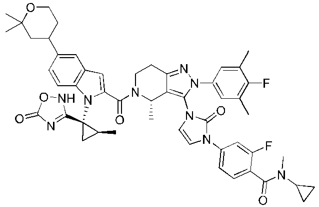 | 926.6 | ¹H NMR (400 MHz, CDCl₃) δ 11.27 (d, *J =* 15.0 Hz, 1H), 7.61 - 7.44 (m, 4H), 7.14 - 7.00 (m, 2H), 6.82 - 6.61 (m, 2H), 6.24 (d, *J =* 88.9 Hz, 1H), 5.52 (d, *J =* 203.2 Hz, 1H), 5.00 - 4.29 (m, 1H), 4.01 - 3.75 (m, 2H), 3.67 - 3.33 (m, 1H), 3.19 - 3.09 (m, 3H), 3.09 - 2.74 (m, 4H), 2.23 (d, *J =* 16.7 Hz, 6H), 1.95 - 1.75 (m, 4H), 1.71 - 1.61 (m, 4H), 1.57 - 1.46 (m, 4H), 1.36 - 1.26 (m, 6H), 1.20 - 1.03 (m, 3H), 0.91 - 0.45 (m, 4H). |
| **50** | 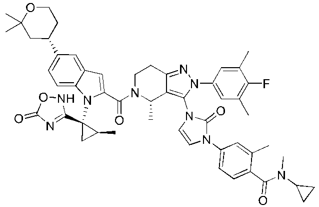 | 922.5 | ¹H NMR (400 MHz, CDCl₃) δ 11.37 - 11.21 (m, 1H), 7.64 - 7.32 (m, 4H), 7.25 - 7.02 (m, 3H), 6.75 - 6.53 (m, 2H), 6.36 - 6.07 (m, 1H), 5.82 - 5.19 (m, 1H), 4.92 - 4.41 (m, 1H), 3.93 - 3.79 (m, 2H), 3.64 - 3.33 (m, 1H), 3.19 - 2.98 (m, 5H), 2.98 - 2.55 (m, 2H), 2.39 - 2.34 (m, 2H), 2.26 - 2.21 (m, 6H), 2.01 - 1.82 (m, 1H), 1.80 - 1.71 (m, 3H), 1.70 - 1.58 (m, 4H), 1.57 - 1.47 (m, 4H), 1.36 - 1.32 (m, 3H), 1.29 - 1.26 (m, 3H), 1.19 - 1.04 (m, 3H), 0.93 - 0.74 (m, 1H), 0.61 - 0.41 (m, 3H). |
| **51** | 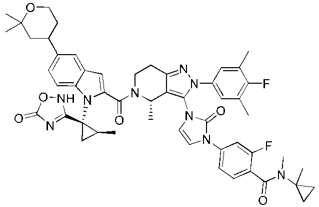 | 940.6 | ¹H NMR (400 MHz, CDCl₃) δ 11.28 (s, 1H), 7.57 (t, *J* = 7.7 Hz, 1H), 7.53 - 7.46 (m, 2H), 7.40 (s, 1H), 7.25 (s, 1H), 7.10 (d, *J* = 5.5 Hz, 2H), 6.76 (s, 1H), 6.68 (d, *J =* 9.3 Hz, 1H), 6.37 - 6.32 (m, 1H), 5.75 (d, *J =* 6.5 Hz, 1H), 4.47 (dd, *J =* 13.6, 5.0 Hz, 1H), 3.86 (td, *J =* 12.3, 11.6, 5.6 Hz, 2H), 3.12 (d, *J =* 11.9 Hz, 2H), 3.02 (s, 2H), 2.90 (s, 1H), 2.27 - 2.22 (m, 6H), 1.94 - 1.86 (m, 1H), 1.82 - 1.70 (m, 7H), 1.69 - 1.59 (m, 2H), 1.55 - 1.48 (m, 4H), 1.41 (d, *J = 10.2* Hz, 2H), 1.34 (s, 3H), 1.28 (s, 3H), 1.26 (s, 1H), 1.18 (d, *J =* 5.3 Hz, 2H), 1.06 (d, *J =* 6.0 Hz, 1H), 0.94 (d, *J =* 14.7 Hz, 1H), 0.80 (d, *J =* 13.2 Hz, 2H). |
| **52** | 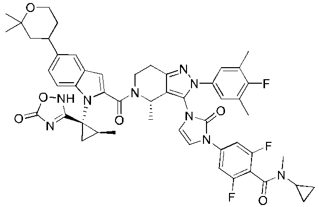 | 944.6 | ¹H NMR (400 MHz, CDCl₃) δ 11.27 (s, 1H), 7.58 (d, *J =* 8.6 Hz, 1H), 7.51 (s, 1H), 7.37 (d, *J* = 9.6 Hz, 2H), 7.25 (s, 1H), 7.10 (d, *J =* 5.8 Hz, 3H), 6.68 (d, *J* = 9.7 Hz, 1H), 6.37 (s, 1H), 5.75 (d, *J =* 6.8 Hz, 1H), 4.47 (d, *J* = 12.6 Hz, 1H), 3.85 (d, *J =* 11.1 Hz, 3H), 3.59 (s, 1H), 3.13 (s, 3H), 3.02 (s, 4H), 2.76 (q, *J* = 5.4 Hz, 1H), 2.25 (d, *J=* 2.2 Hz, 6H), 1.90 (d, *J* = 5.3 Hz, 1H), 1.75 (dt, *J =* 17.1, 6.1 Hz, 3H), 1.62 (d, *J* = 12.5 Hz, 1H), 1.52 (d, *J =* 6.8 Hz, 5H), 1.34 (s, 3H), 1.28 (s, 3H), 1.26 (s, 1H), 1.18 (d, *J =* 5.2 Hz, 3H), 1.06 (d, *J =* 5.3 Hz, 1H), 0.61 (d, *J =* 5.4 Hz, 4H), 0.54 (d, *J =* 7.1 Hz, 1H). |
| **53** | 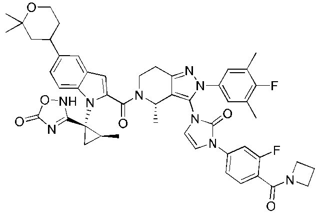 | 912.6 | ¹H NMR (400 MHz, CDCl₃) δ 11.28 (s, 1H), 7.71 - 7.60 (m, 1H), 7.61 - 7.56 (m, 1H), 7.49 (d, *J =* 16.1 Hz, 1H), 7.41 (d, *J* = 8.2 Hz, 1H), 7.25 (d, *J =* 3.1 Hz, 1H), 7.10 (d, *J =* 6.1 Hz, 2H), 6.77 (d, *J =* 3.2 Hz, 1H), 6.68 (d, *J =* 10.6 Hz, 1H), 6.35 (d, *J =* 3.1 Hz, 1H), 5.76 (q, *J =* 6.5 Hz, 1H), 4.47 (dd, *J =* 13.8, 5.0 Hz, 1H), 4.19 (s, 3H), 3.86 (td, *J =* 12.7, 11.7, 4.6 Hz, 2H), 3.59 (t, *J =* 11.4 Hz, 1H), 3.13 (td, *J* = 21.5, 19.5, 10.8 Hz, 1H), 3.02 (s, 2H), 2.36 (p, *J =* 7.8 Hz, 2H), 2.25 (d, *J =* 2.1 Hz, 6H), 1.90 (s, 4H), 1.85 - 1.74 (m, 2H), 1.73 (d, *J =* 4.2 Hz, 1H), 1.70 - 1.58 (m, 2H), 1.51 (d, *J =* 6.8 Hz, 3H), 1.34 (d, *J =* 3.6 Hz, 3H), 1.28 (s, 4H), 1.18 (d, *J =* 5.5 Hz, 2H), 1.06 (d, *J* = 6.1 Hz, 1H). |
| **54** | 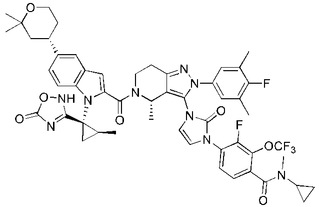 | 1010.8 | ¹H NMR (400 MHz, CDCl₃) δ 11.32 - 11.23 (m, 1H), 7.73 - 7.50 (m, 3H), 7.36 - 7.32 (m, 1H), 7.25 - 7.21 (m, 1H), 7.13 - 7.01 (m, 2H), 6.70 - 6.52 (m, 2H), 6.35 - 6.12 (m, 1H), 5.81 - 5.22 (m, 1H), 4.92 - 4.43 (m, 1H), 3.89 - 3.81 (m, 2H), 3.63 - 3.36 (m, 1H), 3.15 - 3.01 (m, 5H), 2.89 - 2.74 (m, 2H), 2.28 - 2.22 (m, 6H), 1.97 - 1.86 (m, 1H), 1.80 - 1.73 (m, 3H), 1.69 - 1.64 (m, 2H), 1.56 - 1.49 (m, 4H), 1.35 - 1.33 (m, 3H), 1.29 - 1.26 (m, 3H), 1.20 - 1.05 (m, 3H), 0.95 - 0.76 (m, 1H), 0.63 - 0.43 (m, 3H). |
| **55** | 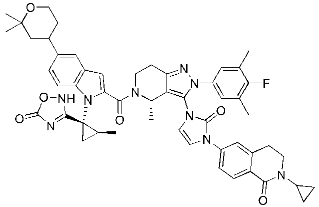 | 920.6 | ¹H NMR (400 MHz, CDCl₃) δ 11.28 (d, *J =* 24.2 Hz, 1H), 8.26 - 8.04 (m, 1H), 7.69 (s, 1H), 7.58 - 7.43 (m, 2H), 7.42 - 7.31 (m, 1H), 7.16 - 6.97 (m, 2H), 6.84 - 6.62 (m, 2H), 6.46 - 6.06 (m, 1H), 6.07 - 5.16 (m, 1H), 4.94 - 4.35 (m, 1H), 3.90 - 3.80 (m, 2H), 3.70 - 3.45 (m, 3H), 3.45 - 3.11 (m, 1H), 3.07 - 2.85 (m, 5H), 2.23 (d, *J =* 16.3 Hz, 6H), 1.92 - 1.89 (m, 1H), 1.88 - 1.82 (m, 4H), 1.80 - 1.76 (m, 2H), 1.76 - 1.61 (m, 4H), 1.34 (s, 3H), 1.30 - 1.26 (m, 3H), 1.19 - 1.05 (m, 3H), 0.85 (d, *J* = 68.5 Hz, 4H). |

### Example 3: Synthesis of Compounds 56a and 56b

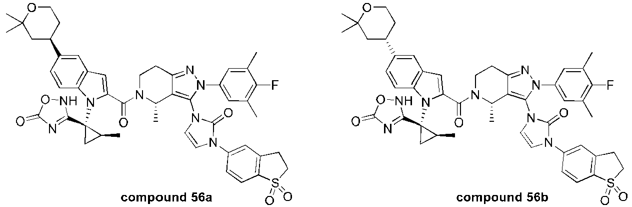

### Compound 56 was synthesized via the following route:

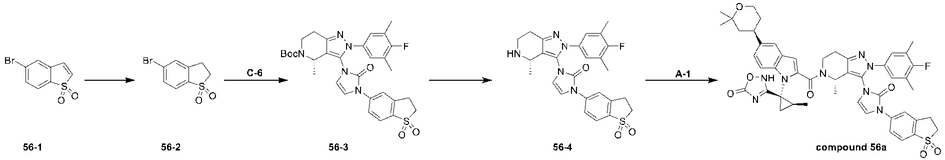

### Step 1: Synthesis of Compound 56-2

Compound 56-1 (1.2 g, 4.90 mmol) was dissolved in ethanol (6 mL), and sodium borohydride (278 mg, 7.35 mmol) was added. The mixture was stirred at room temperature overnight. Saturated ammonium chloride solution (100 mL) was added to quench the reaction, and the mixture was extracted with dichloromethane (100 mL × 3). The combined organic phases were washed with saturated brine (100 mL × 2), dried over anhydrous sodium sulfate, filtered, and concentrated to obtain the crude product. The crude product was purified by silica gel column chromatography (petroleum ether/ethyl acetate = 0% to 10%) to afford the target compound 56-2 (580 mg, 48% yield).

¹H NMR (400 MHz, DMSO-d₆) δ 8.22 (s, 1H), 7.51 (d, J = 8.7 Hz, 1H), 7.43 (dd, J = 8.7, 6.4 Hz, 1H), 4.07 (s, 4H).

### Step 2: Synthesis of compound 56-3

Compound 56-2 (62 mg, 0.25 mmol) was dissolved in N-methylpyrrolidone (2 mL), followed by sequential addition of INT5-4 (110 mg, 0.25 mmol), copper(I) iodide (10 mg, 0.05 mmol), potassium carbonate (104 mg, 0.75 mmol), and N,N'-dimethyl-1,2-cyclohexanediamine (18 mg, 0.13 mmol). The reaction mixture was stirred at 130°C under nitrogen atmosphere for 3 hours. Water (30 mL) was added to dilute the reaction mixture, which was then extracted with ethyl acetate (30 mL × 3). The combined organic phases were washed with saturated brine (30 mL × 2), dried over anhydrous sodium sulfate, filtered, and concentrated to obtain the crude product. The crude product was purified by silica gel column chromatography (petroleum ether/ethyl acetate *=* 10% to 60%) to afford the target compound 56-3 (150 mg, 99% yield).

LC-MS (ESI⁺) m/z: 608.2 (M+H)⁺.

### Step 3: Synthesis of compound 56-4

Compound 56-3 (100 mg, 0.16 mmol) was dissolved in a solution of hydrogen chloride in 1,4-dioxane (4M, 2 mL), and the reaction mixture was stirred at room temperature for 0.5 hours. The reaction mixture was directly concentrated to obtain the crude product 56-4 (70 mg).

LC-MS (ESI⁺) m/z: 508.1 (M+H)⁺.

### Step 4: Synthesis of compound 56

Compound 56-4 (50 mg, 0.12 mmol) was dissolved in N,N-dimethylformamide (1 mL), followed by sequential addition of compound A-1 (67 mg, 0.13 mmol), 2-(7-azabenzotriazolyl)-N,N,N',N'-tetramethyluronium hexafluorophosphate (55 mg, 0.14 mmol), and N,N-diisopropylethylamine (62 mg, 0.48 mmol). The reaction mixture was stirred at room temperature for 1 hour. The reaction mixture was directly purified by reverse-phase chromatography (acetonitrile/0.05% formic acid containing water = 5%-50%) to afford the target compound 56a (46 mg, 42% yield).

LC-MS (ESI⁺) m/z: 901.4 (M+H)⁺;
¹H NMR (400 MHz, CDCl₃) δ 11.26 (d, J = 31.5 Hz, 1H), 7.94 - 7.66 (m, 2H), 7.62 - 7.50 (m, 3H), 7.39 - 7.27 (m, 1H), 7.13 - 6.98 (m, 2H), 6.82 - 6.65 (m, 2H), 6.43 - 6.15 (m, 1H), 5.84 - 5.19 (m, 1H), 4.92 - 4.43 (m, 1H), 3.90 - 3.81 (m, 2H), 3.60 - 3.33 (m, 5H), 3.17 - 2.98 (m, 3H), 2.26 - 2.21 (m, 6H), 1.95 - 1.86 (m, 1H), 1.80 - 1.77 (m, 4H), 1.68 - 1.59 (m, 2H), 1.54 - 1.51 (m, 2H), 1.36 - 1.33 (m, 3H), 1.30 - 1.23 (m, 4H), 1.19 - 1.05 (m, 3H).

Compound 56b was synthesized from compound A-2 and compound 56-4 according to the synthetic method of 56a.

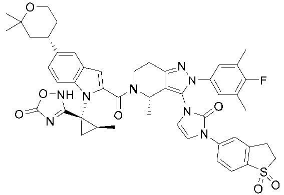

LC-MS (ESI⁺) m/z: 901.3 (M+H)⁺;
¹H NMR (400 MHz, CDCl₃) δ 11.27 (d, J = 32.1 Hz, 1H), 7.99 - 7.65 (m, 2H), 7.63 - 7.44 (m, 3H), 7.40 - 7.27 (m, 1H), 7.14 - 6.97 (m, 2H), 6.83 - 6.63 (m, 2H), 6.41 - 6.16 (m, 1H), 5.81 - 5.19 (m, 1H), 4.91 - 4.40 (m, 1H), 3.91 - 3.78 (m, 2H), 3.63 - 3.28 (m, 5H), 3.22 - 2.94 (m, 3H), 2.23 (dd, J = 15.6, 1.8 Hz, 6H), 1.94 - 1.83 (m, 1H), 1.76 - 1.68 (m, 4H), 1.65 - 1.57 (m, 2H), 1.53 - 1.50 (m, 2H), 1.34 (s, 3H), 1.31 - 1.21 (m, 4H), 1.19 - 1.03 (m, 3H).

### Compounds 57 to 62 were prepared according to the preparation method of Example 3:

| **Compound No.** | **Structure** | **LC-MS (ESI⁺) m/z: (M+H)⁺** | **¹H NMR (400 MHz)** |
|---|---|---|---|
| **57** | 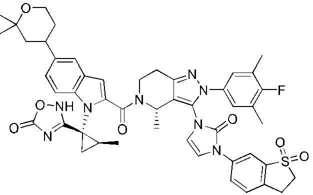 | 901.2 | ¹H NMR (400 MHz, CDCl₃) δ 11.28 (d, *J =* 24.5 Hz, 1H), 8.17 - 7.95 (m, 1H), 7.83 - 7.73 (m, 1H), 7.62 - 7.49 (m, 3H), 7.34 - 7.24 (m, 3H), 7.12 - 6.99 (m, 2H), 6.81 - 6.63 (m, 2H), 6.39 - 6.14 (m, 1H), 5.80 - 5.26 (m, 1H), 4.87 - 4.46 (m, 1H), 3.90 - 3.81 (m, 2H), 3.60 - 3.54 (m, 2H), 3.52 - 3.32 (m, 3H), 3.19 - 3.09 (m, 1H), 3.06 - 2.97 (m, 2H), 2.27 - 2.20 (m, 6H), 1.92 - 1.87 (m, 1H), 1.78 - 1.76 (m, 1H), 1.74 - 1.72 (m, 1H), 1.69 - 1.68 (m, 1H), 1.63 - 1.61 (m, 1H), 1.53 - 1.49 (m, 3H), 1.35 - 1.32 (m, 3H), 1.29 - 1.25 (m, 3H), 1.20 - 1.03 (m, 3H). |
| **58** | 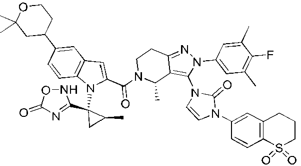 | 915.6 | ¹H NMR (400 MHz, CDCl₃) δ 11.63 - 10.95 (m, 1H), 8.10 - 7.62 (m, 2H), 7.60 - 7.36 (m, 3H), 7.35 - 7.23 (m, 1H), 7.16 - 6.95 (m, 2H), 6.86 - 6.56 (m, 2H), 6.47 - 6.00 (m, 1H), 5.92 - 4.35 (m, 3H), 3.98 - 3.74 (m, 2H), 3.68 - 3.51 (m, 1H), 3.44 - 3.29 (m, 2H), 3.27 - 2.83 (m, 5H), 2.63 - 2.37 (m, 2H), 2.32 - 2.12 (m, 6H), 1.90 *(t, J=* 5.0 Hz, 1H), 1.81 - 1.70 (m,3H), 1.71 - 1.57 (m, 2H), 1.53 - 1.46 (m, 3H), 1.34 (s, 3H), 1.30 - 1.25 (m, 3H), 1.22 - 1.02 (m, 3H). |
| **59** | 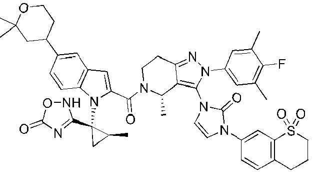 | 915.6 | ¹H NMR (400 MHz, CDCl₃) δ 11.35 - 11.23 (m, 1H), 8.10 - 7.63 (m, 2H), 7.61 - 7.33 (m, 3H), 7.25 - 6.99 (m, 3H), 6.82 - 6.64 (m, 2H), 6.38 - 6.08 (m, 1H), 5.81 - 5.23 (m, 1H), 4.93 - 4.41 (m, 1H), 3.90 - 3.80 (m, 2H), 3.57 - 3.16 (m, 3H), 3.07 - 2.99 (m, 3H), 2.58 - 2.47 (m, 2H), 2.28 - 2.21 (m, 6H), 2.02 - 1.85 (m, 1H), 1.79 - 1.67 (m, 4H), 1.65 - 1.61 (m, 4H), 1.54 - 1.49 (m, 3H), 1.35 - 1.32 (m, 3H), 1.29 - 1.26 (m, 3H), 1.20 - 1.04 (m, 3H). |
| **60** | 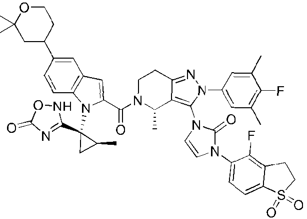 | 919.2 | ¹H NMR (400 MHz, CDCl₃) δ 11.26 *(d, J= 25.7 Hz,* 1H), 8.02 - 7.71 (m, 1H), 7.67 - 7.42 (m, 3H), 7.15 - 7.00 (m, 2H), 6.71 - 6.53 (m, 2H), 6.40 - 6.14 (m, 1H), 5.79 - 5.22 (m, 1H), 4.91 - 4.35 (m, 1H), 3.92 - 3.79 (m, 2H), 3.64 - 3.34 (m, 5H), 3.20 - 2.97 (m, 3H), 2.29 - 2.23 (m, 7H), 1.93 - 1.72 (m, 4H), 1.69 - 1.58 (m, 2H), 1.57 - 1.44 (m, 4H), 1.35 (s, 3H), 1.29 (s, 3H), 1.20 - 1.04 (m, 3H). |
| **61** | 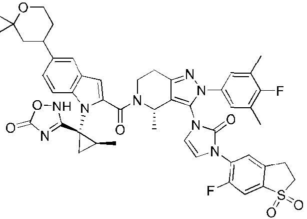 | 919.7 | ¹H NMR (400 MHz, CDCl₃) δ 11.34 (s, 1H), 8.41 - 7.72 (m, 1H), 7.66 - 7.45 (m, 3H), 7.23 - 6.96 (m, 2H), 6.94 - 6.46 (m, 2H), 6.41 - 5.86 (m, 1H), 5.83 - 5.19 (m, 1H), 5.13 - 4.07 (m, 1H), 4.05 - 3.74 (m, 2H), 3.63 - 3.27 (m, 5H), 3.21 - 2.93 (m, 3H), 2.32 - 2.17 (m, 6H), 1.92 (d, *J=* 4.7 Hz, 1H), 1.81 - 1.72 (m, 3H), 1.69 - 1.59 (m, 2H), 1.54 (d, *J* = 6.6 Hz, 3H), 1.42 (d, *J =* 6.5 Hz, 2H), 1.34 (s, 3H), 1.28 (s, 3H), 1.21 - 1.05 (m, 3H). |
| **62** | 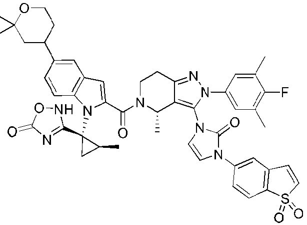 | 899.5 | ¹H NMR (400 MHz, CDCl₃) δ 11.41 - 11.14 (m, 1H), 8.00 - 7.56 (m, 4H), 7.52 - 7.38 (m, 1H), 7.29 - 7.27 (m, 1H), 7.11 - 6.99 (m, 2H), 6.93 - 6.50 (m, 3H), 6.47 - 6.14 (m, 1H), 5.89 - 5.21 (m, 1H), 4.91 - 3.91 (m, 1H), 3.90 - 3.73 (m, 2H), 3.64 - 3.35 (m, 1H), 3.20 - 2.97 (m, 3H), 2.28 - 2.19 (m, 6H), 1.92 - 1.89 (m, 2H), 1.74 - 1.72 (m, 1H), 1.68 - 1.60 (m, 2H), 1.54 - 1.50 (m, 3H), 1.36 - 1.25 (m, 9H), 1.19 - 1.04 (m, 3H). |
| **62a** | 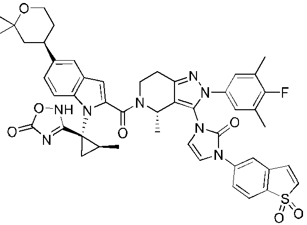 | 899.6 | ¹H NMR (400 MHz, CDCl₃) δ 11.36 - 11.20 (m, 1H), 8.07 - 7.30 (m, 6H), 7.11 - 7.00 (m, 2H), 6.82 - 6.64 (m, 3H), 6.41 - 6.17 (m, 1H), 5.77 - 5.24 (m, 1H), 4.87 - 4.46 (m, 1H), 3.93 - 2.98 (m, 7H), 2.27 - 2.20 (m, 6H), 1.92 - 1.73 (m, 4H), 1.53 - 1.49 (m, 3H), 1.36 - 1.25 (m, 9H), 1.18 - 1.05 (m, 3H). |
| **62b** | 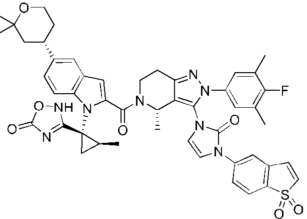 | 899.6 | ¹H NMR (400 MHz, CDCl₃) δ 8.01 - 7.28 (m, 6H), 7.11 - 6.99 (m, 2H), 6.86 - 6.62 (m, 3H), 6.44 - 6.17 (m, 1H), 5.83 - 5.34 (m, 1H), 4.85 - 4.45 (m, 1H), 3.94 - 2.98 (m, 7H), 2.28 - 2.20 (m, 6H), 1.90 - 1.71 (m, 4H), 1.66 - 1.56 (m, 3H), 1.35 - 1.25 (m, 9H), 1.20 - 1.13 (m, 3H). |

### Example 4:

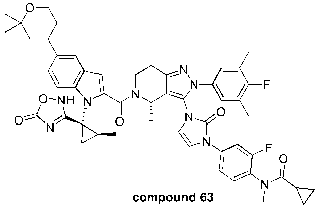

### Compound 63 was synthesized via the following route:

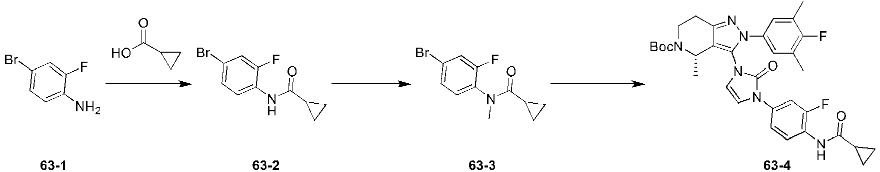

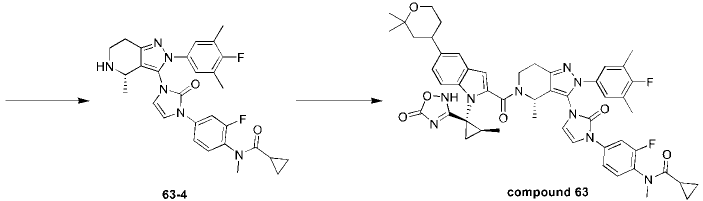

### Step 1: Synthesis of compound 63-2

Compound 63-1 (1.5 g, 7.89 mmol) and cyclopropanecarboxylic acid (815.52 mg, 9.47 mmol) were dissolved in N,N-dimethylformamide (10 mL), followed by sequential addition of 2-(7-azabenzotriazolyl)-N,N,N',N'-tetramethyluronium hexafluorophosphate (3.60 g, 9.47 mmol) and N,N-diisopropylethylamine (3.06 g, 23.68 mmol). The reaction mixture was stirred at room temperature for 1 hour. Water (80 mL) was added to dilute the reaction mixture, which was then extracted with ethyl acetate (60 mL × 3). The organic phases were washed with saturated brine (80 mL × 3), dried over anhydrous sodium sulfate, filtered, and concentrated to obtain the crude product. The crude product was purified by silica gel column chromatography (petroleum ether/ethyl acetate = 9:1 to 4:1) to afford the target compound (1 g, 49% yield).

LC-MS (ESI⁺) m/z: 257.9 (M+H)⁺;
¹H NMR (400 MHz, CDCl₃) δ 8.16 (t, J = 8.5 Hz, 1H), 7.46 (s, 1H), 7.21 - 7.17 (m, 2H), 1.51 - 1.47 (m, 1H), 1.05 - 1.02 (m, 2H), 0.85 - 0.81 (m, 2H).

### Step 2: Synthesis of compound 63-3

Compound 63-2 (500 mg, 1.94 mmol) was dissolved in N,N-dimethylformamide (5 mL), and the reaction solution was placed at 0°C. Sodium hydride (55 mg, 2.32 mmol) was added in portions, followed by stirring for 30 minutes. Methyl iodide (412 mg, 2.91 mmol) was then slowly added dropwise, and the reaction solution was stirred at room temperature under nitrogen atmosphere for 1 hour. Ice water (50 mL) was added to quench the reaction, and the mixture was extracted with ethyl acetate (50 mL×3). The combined organic phases were washed with saturated brine (50 mL×3), dried over anhydrous sodium sulfate, filtered, and concentrated to obtain the target compound 63-3 (300 mg, yield 57%).

LC-MS (ESI⁺) m/z: 272.0 (M+H)⁺;
¹H NMR(400 MHz, CDCl₃) δ 7.75 (s, 1H), 7.66 (d, J = 8.4 Hz, 1H), 7.57 - 7.53 (m, 1H), 3.03 (s, 3H), 2.77 - 2.72 (m, 1H), 0.62 (s, 2H), 0.41 (s, 2H).

### Step 3: Synthesis of compound 63-4

Compound C-6 (180 mg, 0.41 mmol) and compound 63-3 (134 mg, 0.49 mmol) were dissolved in N-methylpyrrolidone (3 mL), followed by sequential addition of N,N'-dimethyl-1,2-cyclohexanediamine (24 mg, 0.17 mmol), potassium carbonate (171 mg, 1.24 mmol), and copper(I) iodide (16 mg, 0.084 mmol) to the reaction solution. The reaction solution was stirred at 130°C under nitrogen atmosphere for 16 hours. After cooling to room temperature, water (20 mL) was added to dilute the reaction solution, and the mixture was extracted with ethyl acetate (25 mL×3). The combined organic phases were washed with saturated brine (20 mL×3), dried over anhydrous sodium sulfate, filtered, and concentrated to obtain the crude product. The crude product was purified by silica gel column chromatography (petroleum ether/ethyl acetate = 4:5) to obtain the target compound 63-4 (236 mg, yield 92%).

¹H NMR (400 MHz, CDCl₃) δ 7.62 - 7.56 (m, 1H), 7.45 - 7.39 (m, 2H), 7.10 - 7.04 (m, 2H), 6.75 - 6.68 (m, 1H), 6.40 - 6.28 (m, 1H), 3.29 - 3.23 (m, 3H), 2.86 - 2.74 (m, 2H), 2.24 - 2.20 (m, 6H), 1.49 (s, 9H), 1.31 - 1.27 (m, 4H), 1.10 - 1.00 (m, 3H), 0.91 - 0.81 (m, 4H).

### Step 4: Synthesis of compound 63-5

4M hydrogen chloride in 1,4-dioxane solution (2 mL) was added to 63-4 (231 mg, 0.37 mmol) in 1,4-dioxane (4 mL), and the mixture was stirred at room temperature for 2 hours. After the reaction was complete, the solution was directly concentrated to obtain the target compound (192 mg, yield 99%).

LC-MS (ESI⁺) m/z: 533.3 (M+H)⁺.

### Step 5: Synthesis of compound 63

Compound 63-5 (176 mg, 0.33 mmol) and compound A (90 mg, 0.22 mmol) were dissolved in N,N-dimethylformamide (2 mL), followed by sequential addition of 2-(7-azabenzotriazolyl)-N,N,N',N'-tetramethyluronium hexafluorophosphate (125 mg, 0.33 mmol) and N,N-diisopropylethylamine (85 mg, 0.66 mmol). The reaction solution was stirred at room temperature for 16 hours. Water (30 mL) was added to dilute the reaction solution, and the mixture was extracted with ethyl acetate (30 mL×3).The organic phase was washed with saturated brine (30 mL×3), dried over anhydrous sodium sulfate, filtered, and concentrated to obtain the crude product. The crude product was purified by reverse-phase chromatography (acetonitrile/0.05% formic acid containing water) to obtain the target compound 63 (102 mg, yield 50%).

LC-MS (ESI⁺) m/z: 926.8 (M+H)⁺;
¹H NMR (400 MHz, CDCl₃) δ 11.34 - 11.20 (m, 1H), 7.73 - 7.54 (m, 2H), 7.54 - 7.49 (m, 1H), 7.48 - 7.35 (m, 2H), 7.30 - 7.26 (m, 1H), 7.13 - 7.00 (m, 2H), 6.79 - 6.58 (m, 2H), 6.39 - 6.14 (m, 1H), 5.79 - 5.22 (m, 1H), 4.95 - 4.37 (m, 1H), 3.91 - 3.80 (m, 2H), 3.69 - 3.46 (m, 1H), 3.28 - 3.25 (m, 2H), 3.24 - 3.16 (m, 1H), 3.16 - 2.92 (m, 3H), 2.28 - 2.20 (m, 6H), 1.94 - 1.87 (m, 1H), 1.82 - 1.67 (m, 7H), 1.66 - 1.59 (m, 2H), 1.59 - 1.54 (m, 1H), 1.38 - 1.32 (m, 4H), 1.29 - 1.25 (m, 3H), 1.19 - 1.16 (m, 2H), 1.08 - 1.02 (m, 2H), 0.73 - 0.57 (m, 2H).

### Compounds 63a to 77 were prepared according to the preparation method of Example 4.

| **Compound No.** | **Structure** | **LC-MS (ESI⁺) m/z: (M+H)⁺** | **¹H NMR (400 MHz)** |
|---|---|---|---|
| **63a** | 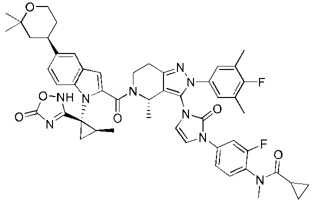 | 926.6 | ¹H NMR (400 MHz, CDCl₃) δ 11.31 - 11.21 (m, 1H), 7.62 - 7.26 (m, 6H), 7.13 - 7.01 (m, 2H), 6.79 - 6.65 (m, 2H), 6.59 - 6.16 (m, 1H), 5.81 - 5.24 (m, 1H), 4.87 - 4.44 (m, 1H), 3.89 - 3.78 (m, 2H), 3.69 - 3.45 (m, 1H), 3.31 - 3.10 (m, 4H), 3.05 - 2.97 (m, 2H), 2.27 - 2.22 (m, 6H), 1.91 - 1.71 (m, 4H), 1.58 - 1.51 (m, 7H), 1.34 - 1.27 (m, 6H), 1.19 - 1.07 (m, 3H), 1.05 - 0.96 (m, 2H), 0.71 - 0.60 (m, 2H). |
| **63b** | 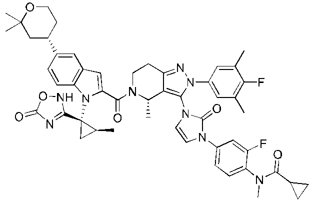 | 926.6 | ¹H NMR (400 MHz, CDCl₃) δ 11.30 - 11.22 (m, 1H), 7.67 - 7.27 (m, 6H), 7.12 - 7.00 (m, 2H), 6.80 - 6.64 (m, 2H), 6.59 - 6.15 (m, 1H), 5.79 - 5.18 (m, 1H), 4.90 - 4.42 (m, 1H), 3.90 - 3.81 (m, 2H), 3.64 - 3.43 (m, 1H), 3.29 - 3.10 (m, 4H), 3.05 - 2.98 (m, 2H), 2.27 - 2.22 (m, 6H), 1.90 - 1.72 (m, 4H), 1.57 - 1.51 (m, 7H), 1.34 - 1.26 (m, 6H), 1.20 - 1.09 (m, 3H), 1.06 - 1.00 (m, 2H), 0.71 - 0.61 (m, 2H). |
| **64** | 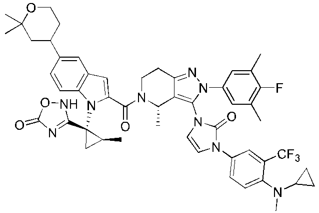 | 948.3 | ¹H NMR (400 MHz, CDCl₃) δ 11.37 - 11.19 (m, 1H), 7.83 - 7.66 (m, 1H), 7.60 - 7.44 (m, 3H), 7.26 - 7.23 (m, 1H), 7.14 - 7.00 (m, 2H), 6.72 - 6.54 (m, 2H), 6.35 - 6.05 (m, 1H), 5.79 - 5.23 (m, 1H), 4.93 - 4.38 (m, 1H), 3.94 - 3.76 (m, 2H), 3.68 - 3.25 (m, 1H), 3.20 - 2.95 (m, 3H), 2.83 - 2.74 (m, 3H), 2.52 - 2.41 (m, 1H), 2.28 - 2.20 (m, 6H), 1.92 - 1.72 (m, 4H), 1.70 - 1.53 (m, 6H), 1.52 - 1.50 (m, 1H), 1.35 - 1.32 (m, 3H), 1.29 - 1.26 (m, 3H), 1.20 - 1.04 (m, 3H), 0.60 - 0.40 (m, 4H). |
| **65** | 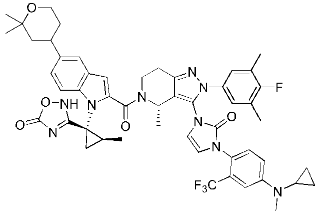 | 948.3 | ¹H NMR (400 MHz, CDCl₃) δ 11.39 - 11.26 (m, 1H), 7.61 - 7.50 (m, 2H), 7.32 - 7.27 (m, 1H), 7.25 - 7.23 (m, 1H), 7.22 - 7.18 (m, 1H), 7.16 - 7.14 (m, 1H), 7.11 - 6.99 (m, 1H), 6.72 - 6.67 (m, 1H), 6.44 - 6.26 (m, 1H), 6.24 - 5.98 (m, 1H), 5.79 - 5.25 (m, 1H), 4.92 - 4.29 (m, 1H), 3.90 - 3.78 (m, 2H), 3.64 - 3.26 (m, 1H), 3.16 - 2.96 (m, 6H), 2.58 - 2.46 (m, 1H), 2.30 - 2.25 (m, 6H), 1.93 - 1.89 (m, 1H), 1.80 - 1.72 (m, 4H), 1.68 - 1.59 (m, 2H), 1.56 - 1.43 (m, 4H), 1.34 (s, 3H), 1.28 (s, 3H), 1.22 - 1.05 (m, 3H), 0.94 - 0.88 (m, 2H), 0.74 - 0.65 (m, 2H). |
| **66** | 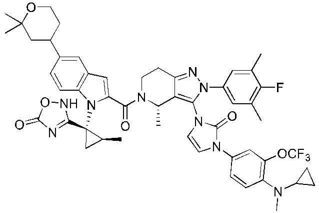 | 964.6 | ¹H NMR (400 MHz, CDCl₃) δ 11.34 - 11.23 (m, 1H), 7.60 - 7.54 (m, 1H), 7.52 - 7.47 (m, 1H), 7.45 - 7.35 (m, 2H), 7.25 - 7.22 (m, 1H), 7.13 -7.00 (m, 2H), 6.70 - 6.67 (m, 1H), 6.66 - 6.49 (m, 1H), 6.30 - 6.03 (m, 1H), 5.77 - 5.23 (m, 1H), 4.90 - 4.40 (m, 1H), 3.90 - 3.79 (m, 2H), 3.63 - 3.35 (m, 1H), 3.20 - 3.07 (m, 1H), 3.05 - 2.92 (m, 5H), 2.54 - 2.41 (m, 1H), 2.27 - 2.21 (m, 6H), 1.92 - 1.82 (m, 1H), 1.80 - 1.67 (m, 4H), 1.66 - 1.60 (m, 2H), 1.56 1.48 (m, 4H), 1.35 - 1.32 (m, 3H), 1.29 - 1.26 (m, 3H), 1.20 - 1.04 (m, 3H), 0.78 - 0.66 (m, 2H), 0.56 - 0.42 (m, 2H). |
| **67** | 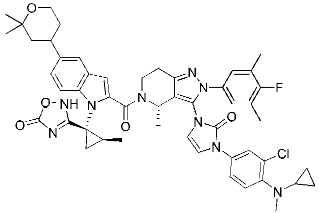 | 914.2 | ¹H NMR (400 MHz, CDCl₃) δ 11.35 - 11.23 (m, 1H), 7.61 - 7.55 (m, 1H), 7.53 - 7.46 (m, 2H), 7.44 - 7.37 (m, 1H), 7.25 - 7.19 (m, 1H), 7.13 - 7.01 (m, 2H), 6.71 - 6.67 (m, 1H), 6.66 - 6.49 (m, 1H), 6.30 - 6.04 (m, 1H), 5.78 - 5.23 (m, 1H), 4.90 - 4.41 (m, 1H), 3.90 - 3.79 (m, 2H), 3.65 - 3.34 (m, 1H), 3.19 - 2.97 (m, 3H), 2.94 - 2.88 (m, 3H), 2.55 - 2.42 (m, 1H), 2.27 - 2.21 (m, 6H), 1.92 - 1.72 (m, 4H), 1.70 - 1.53 (m, 6H), 1.51 (s, 1H), 1.35 - 1.32 (m, 3H), 1.29 - 1.26 (m, 3H), 1.20 - 1.05 (m, 3H), 0.72 - 0.63 (m, 2H), 0.53 - 0.41 (m, 2H). |
| **67a** | 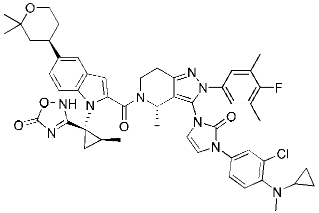 | 914.5 | ¹H NMR (400 MHz, CDCl₃) δ 11.32 - 11.26 (m, 1H), 7.61 - 7.57 (m, 1H), 7.56 - 7.52 (m, 1H), 7.52 - 7.45 (m, 2H), 7.25 - 7.18 (m, 1H), 7.13 - 7.03 (m, 2H), 6.70 - 6.68 (m, 1H), 6.67 - 6.50 (m, 1H), 6.30 - 6.05 (m, 1H), 5.78 - 5.23 (m, 1H), 4.85 - 4.31 (m, 1H), 3.88 - 3.81 (m, 2H), 3.66 - 3.51 (m, 1H), 3.17 - 3.07 (m, 1H), 3.02 - 2.89 (m, 5H), 2.64 - 2.53 (m, 1H), 2.27 - 2.22 (m, 6H), 1.94 - 1.87 (m, 1H), 1.83 - 1.72 (m, 4H), 1.68 - 1.62 (m, 2H), 1.55 - 1.50 (m, 4H), 1.34 - 1.32 (m, 3H), 1.29 - 1.27 (m, 3H), 1.20 - 1.07 (m, 3H), 0.72 - 0.51 (m, 4H). |
| **67b** | 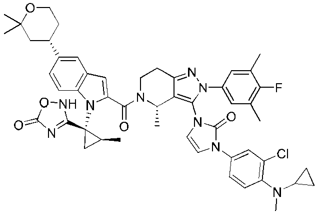 | 914.6 | ¹H NMR (400 MHz, CDCl₃) δ 7.65 - 7.53 (m, 1H), 7.52 - 7.49 (m, 1H), 7.48 - 7.31 (m, 2H), 7.25 - 7.19 (m, 1H), 7.14 - 7.01 (m, 2H), 6.71 - 6.67 (m, 1H), 6.66 - 6.47 (m, 1H), 6.31 - 6.02 (m, 1H), 5.87 - 5.14 (m, 1H), 4.90 - 4.28 (m, 1H), 3.93 - 3.77 (m, 2H), 3.64 - 3.36 (m, 1H), 3.21 - 3.06 (m, 1H), 3.05 - 2.85 (m, 5H), 2.52 - 2.43 (m, 1H), 2.28 - 2.20 (m, 6H), 1.97 - 1.83 (m, 1H), 1.81 - 1.68 (m, 4H), 1.66 - 1.60 (m, 2H), 1.55 - 1.49 (m, 4H), 1.36 - 1.32 (m, 3H), 1.29 - 1.26 (m, 3H), 1.20 - 1.05 (m, 3H), 0.73 - 0.64 (m, 2H), 0.50 - 0.40 (m, 2H). |
| **68** | 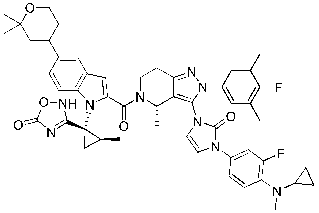 | 898.1 | ¹H NMR (400 MHz, CDCl₃) δ 11.33 - 11.23 (m, 1H), 7.60 - 7.55 (m, 1H), 7.52 - 7.38 (m, 2H), 7.25 - 6.99 (m, 4H), 6.70 - 6.51 (m, 2H), 6.31 - 6.05 (m, 1H), 5.78 - 5.22 (m, 1H), 4.90 - 4.41 (m, 1H), 3.90 - 3.79 (m, 2H), 3.63 - 3.34 (m, 1H), 3.18 - 2.97 (m, 6H), 2.60 - 2.44 (m, 1H), 2.27 - 2.21 (m, 6H), 1.91 - 1.72 (m, 4H), 1.67 - 1.55 (m, 6H), 1.51 (s, 1H), 1.35 - 1.32 (m, 3H), 1.29 - 1.26 (m, 3H), 1.19 - 1.05 (m, 3H), 0.81 - 0.63 (m, 4H). |
| **69** | 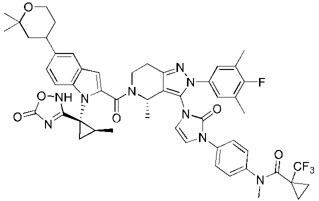 | 976.4 | ¹H NMR (400 MHz, CDCl₃) δ 11.33 - 11.23 (m, 1H), 7.70 - 7.67 (m, 1H), 7.61 - 7.56 (m, 1H), 7.53 - 7.48 (m, 1H), 7.41 - 7.33 (m, 2H), 7.16 - 7.01 (m, 2H), 6.77 - 6.60 (m, 2H), 6.38 - 6.10 (m, 1H), 5.81 - 5.22 (m, 1H), 4.93 - 4.42 (m, 1H), 3.92 - 3.81 (m, 2H), 3.61 - 3.36 (m, 4H), 3.17 - 2.97 (m, 3H), 2.28 - 2.20 (m, 6H), 2.02 - 1.84 (m, 5H), 1.81 - 1.58 (m, 6H), 1.35 - 1.32 (m, 3H), 1.30 - 1.24 (m, 5H), 1.23 - 1.05 (m, 6H). |
| **70** | 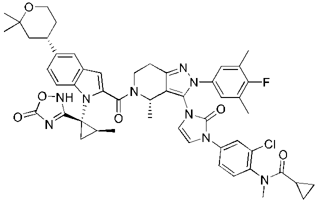 | 942.4 | ¹H NMR (400 MHz, CDCl₃) δ 11.26 (d, *J =* 24.9 Hz, 1H), 7.64 - 7.33 (m, 4H), 7.30 - 7.26 (m, 2H), 7.13 - 7.01 (m, 2H), 6.79 - 6.60 (m, 2H), 6.40 - 6.09 (m, 1H), 5.81 - 5.21 (m, 1H), 4.91 - 4.38 (m, 1H), 3.92 - 3.79 (m, 2H), 3.62 - 3.37 (m, 1H), 3.27 - 3.10 (m, 4H), 3.08 - 2.95 (m, 3H), 2.35 - 2.20 (m, 8H), 1.94 - 1.74 (m, 7H), 1.74 - 1.69 (m, 2H), 1.68 - 1.63 (m, 2H), 1.52 - 1.50 (m, 1H), 1.36 - 1.32 (m, 3H), 1.30 - 1.24 (m, 3H), 1.20 - 1.05 (m, 3H). |
| **71** | 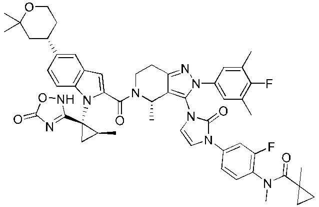 | 940.4 | ¹H NMR (400 MHz, CDCl₃) δ 11.33 - 11.23 (m, 1H), 7.64 - 7.55 (m, 2H), 7.54 - 7.43 (m, 2H), 7.41 - 7.35 (m, 1H), 7.25 - 7.18 (m, 1H), 7.13 - 7.01 (m, 2H), 6.78 - 6.60 (m, 2H), 6.38 - 6.13 (m, 1H), 5.79 - 5.24 (m, 1H), 4.88 - 4.45 (m, 1H), 3.91 - 3.81 (m, 2H), 3.63 - 3.3 9 (m, 1H), 3.30 - 3.22 (m, 3H), 3.21 - 3.11 (m, 1H), 3.05 - 2.98 (m, 2H), 2.27 - 2.21 (m, 6H), 1.93 - 1.85 (m, 2H), 1.82 - 1.72 (m, 4H), 1.69 - 1.61 (m, 2H), 1.59 - 1.54 (m, 1H), 1.52 - 1.46 (m, 2H), 1.36 - 1.32 (m, 3H), 1.29 - 1.25 (m, 3H), 1.19 - 1.17 (m, 2H), 1.12 - 1.04 (m, 5H), 0.52 - 0.43 (m, 2H). |
| **72** | 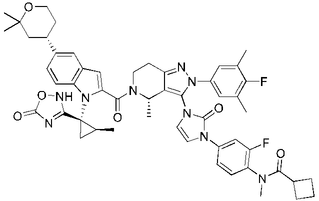 | 940.8 | ¹H NMR (400 MHz, CDCl₃) δ 11.33 - 11.16 (m, 1H), 7.83 - 7.35 (m, 5H), 7.34 - 7.27 (m, 1H), 7.13 - 7.01 (m, 2H), 6.77 - 6.60 (m, 2H), 6.39 - 6.14 (m, 1H), 5.78 - 5.20 (m, 1H), 4.93 - 4.36 (m, 1H), 3.92 - 3.79 (m, 2H), 3.62 - 3.38 (m, 1H), 3.26 - 2.99 (m, 6H), 2.28 - 2.21 (m, 6H), 1.92 - 1.79 (m, 2H), 1.79 - 1.73 (m, 3H), 1.71 - 1.59 (m, 3H), 1.57 - 1.51 (m, 3H), 1.35 - 1.31 (m, 3H), 1.30 - 1.21 (m, 4H), 1.19 - 0.55 (m, 8H). |
| **73** | 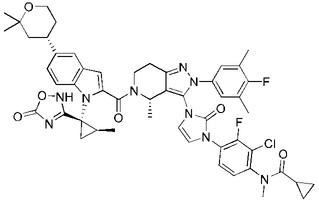 | 960.3 | ¹H NMR (400 MHz, CDCl₃) δ 11.33 - 11.21 (m, 1H), 7.67 - 7.54 (m, 2H), 7.53 - 7.50 (m, 1H), 7.36 - 7.31 (m, 1H), 7.14 - 7.03 (m, 2H), 6.75 - 6.60 (m, 2H), 6.39 - 6.14 (m, 1H), 5.81 - 5.24 (m, 1H), 4.95 - 4.38 (m, 1H), 3.91 - 3.81 (m, 2H), 3.66 - 3.48 (m, 1H), 3.27 - 3.18 (m, 3H), 3.15 - 2.96 (m, 3H), 2.30 - 2.23 (m, 6H), 1.95 - 1.87 (m, 1H), 1.81 - 1.70 (m, 4H), 1.64 1.55 (m, 6H), 1.54 - 1.52 (m, 2H), 1.35 - 1.32 (m, 3H), 1.30 - 1.27 (m, 3H), 1.20 - 1.18 (m, 2H), 1.09 - 1.03 (m, 2H), 0.74 - 0.59 (m, 2H). |
| **74** | 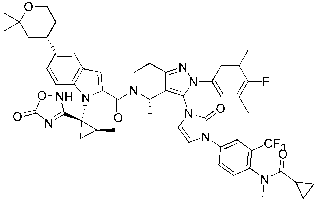 | 976.6 | ¹H NMR (400 MHz, CDCl₃) δ 11.35 - 11.17 (m, 1H), 8.04 - 7.70 (m, 2H), 7.60 - 7.36 (m, 3H), 7.26 - 7.23 (m, 1H), 7.14 - 7.00 (m, 2H), 6.83 - 6.65 (m, 2H), 6.51 - 6.12 (m, 1H), 5.79 - 5.22 (m, 1H), 4.96 - 4.36 (m, 1H), 3.89 - 3.39 (m, 3H), 3.25 - 3.17 (m, 3H), 3.17 - 2.93 (m, 3H), 2.27 - 2.21 (m, 6H), 1.94 - 1.73 (m, 4H), 1.66 - 1.58 (m, 3H), 1.53 - 1.50 (m, 3H), 1.35 - 1.32 (m, 3H), 1.29 - 1.25 (m, 3H), 1.19 - 1.16 (m, 2H), 1.12 - 0.89 (m, 4H), 0.68 - 0.52 (m, 2H). |
| **75** | 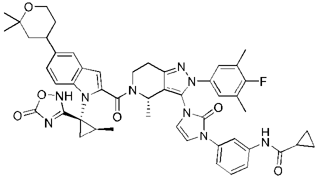 | 867.7 | ¹H NMR (400 MHz, DMSO-d₆) δ 11.74 (s, 1H), 10.54 - 10.14 (m, 1H), 8.03 - 7.76 (m, 1H), 7.64 - 7.44 (m, 2H), 7.42 - 7.32 (m, 2H), 7.30 - 7.16 (m, 3H), 7.15 - 7.04 (m, 2H), 6.99 - 6.68 (m, 2H), 5.84 - 5.10 (m, 1H), 4.91 - 4.22 (m, 1H), 3.81 - 3.43 (m, 3H), 3.27 - 3.05 (m, 1H), 3.03 - 2.72 (m, 2H), 2.24 - 2.16 (m, 6H), 1.85 - 1.63 (m, 5H), 1.62 - 1.47 (m, 3H), 1.42 - 1.34 (m, 2H), 1.33 - 1.25 (m, 4H), 1.25 - 1.00 (m, 6H), 0.84 - 0.74 (m, 4H). |
| **76** | 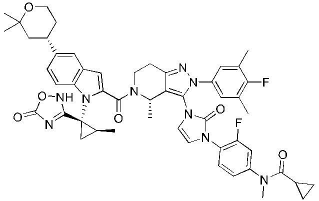 | 926.5 | ¹H NMR (400 MHz, CDCl₃) δ 11.34 - 11.21 (m, 1H), 7.69 - 7.27 (m, 4H), 7.25 - 7.20 (m, 2H), 7.13 - 7.03 (m, 2H), 6.71 - 6.50 (m, 2H), 6.35 - 6.10 (m, 1H), 5.86 - 5.17 (m, 1H), 4.92 - 4.36 (m, 1H), 3.91 - 3.56 (m, 3H), 3.34 - 3.27 (m, 3H), 3.19 - 2.99 (m, 3H), 2.29 - 2.23 (m, 6H), 1.93 - 1.73 (m, 4H), 1.61 - 1.47 (m, 6H), 1.42 - 1.32 (m, 4H), 1.29 - 1.26 (m, 3H), 1.21 - 1.18 (m, 2H), 1.16 - 0.98 (m, 3H), 0.75 - 0.67 (m, 2H). |
| **77** | 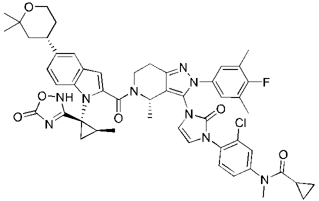 | 942.3 | ¹H NMR (400 MHz, CDCl₃) δ 11.33 - 11.24 (m, 1H), 7.61 - 7.55 (m, 1H), 7.55 - 7.49 (m, 3H), 7.37 - 7.33 (m, 1H), 7.30 - 7.27 (m, 2H), 7.16 - 7.04 (m, 2H), 7.00 - 6.69 (m, 1H), 6.58 - 6.41 (m, 1H), 6.36 - 6.12 (m, 1H), 5.79 - 5.22 (m, 1H), 4.92 - 4.40 (m, 1H), 3.90 - 3.55 (m, 3H), 3.33 - 3.27 (m, 3H), 3.17 - 2.98 (m, 3H), 2.29 - 2.24 (m, 6H), 1.94 - 1.71 (m, 6H), 1.68 - 1.61 (m, 4H), 1.53 - 1.51 (m, 1H), 1.35 - 1.34 (m, 3H), 1.29 - 1.27 (m, 3H), 1.21 - 1.19 (m, 2H), 1.11 - 0.93 (m, 3H), 0.76 - 0.68 (m, 2H). |

### Example 5:

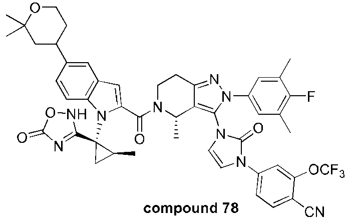

### Compound 78 was synthesized via the following route:

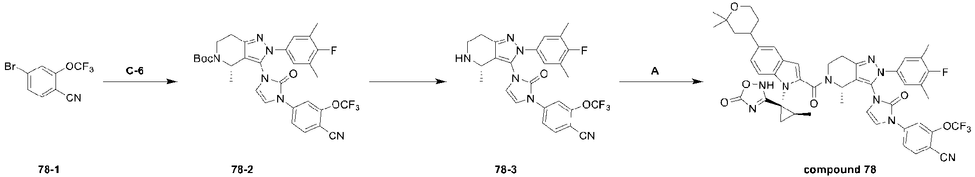

### Step 1: Synthesis of compound 78-2

Compound 78-1 (72 mg, 0.27 mmol) was dissolved in N-methylpyrrolidone (2 mL), followed by addition of compound C-6 (120 mg, 0.27 mmol), N,N'-dimethyl-1,2-cyclohexanediamine (19 mg, 0.14 mmol), copper(I) iodide (10 mg, 0.05 mmol), and potassium carbonate (110 mg, 0.81 mmol) to the reaction solution. The reaction solution was stirred at 130°C under nitrogen atmosphere for 3 hours. Water (30 mL) was added to dilute the reaction solution, and the mixture was extracted with ethyl acetate (50 mL×3).The combined organic phases were washed with saturated brine (30 mL×2), dried over anhydrous sodium sulfate, filtered, and concentrated to obtain the crude product. The crude product was purified by silica gel column chromatography (petroleum ether/ethyl acetate = 1:2) to obtain the target compound 78-2 (130 mg, yield 76%).

LC-MS (ESI⁺) m/z: 627.4 (M+H)⁺.

### Step 2: Synthesis of compound 78-3

Compound 78-2 (120 mg, 0.19 mmol) was dissolved in dichloromethane (5 mL), followed by addition of hydrogen chloride in dioxane solution (4M, 4 mL). The reaction mixture was stirred at room temperature for 30 minutes. The reaction mixture was concentrated, then redissolved in tetrahydrofuran (5 mL), and saturated potassium carbonate aqueous solution (5 mL) was added. The mixture was stirred at room temperature for 30 minutes. Water (20 mL) was added to dilute the above solution, which was then extracted with ethyl acetate (60 mL×3). The combined organic phases were washed with saturated brine (20 mL×2) and water (20 mL× 1), dried over anhydrous sodium sulfate, filtered, and concentrated to obtain product 78-3 (90 mg, 89%).

LC-MS (ESI⁺) m/z: 527.4 (M+H)⁺;
¹H NMR (400 MHz, CDCl₃) δ 7.89 (s, 1H), 7.77 (d, J = 8.6 Hz, 1H), 7.70 (dd, J = 8.6, 2.0 Hz, 1H), 7.03 (d, J = 6.2 Hz, 2H), 6.73 (d, J = 3.3 Hz, 1H), 6.34 (d, J = 3.2 Hz, 1H), 4.11 - 4.05 (m, 1H), 3.37 - 3.35 (m, 1H), 3.12 - 3.01 (m, 1H), 2.80 - 2.74 (m, 2H), 2.20 (d, J = 2.1 Hz, 6H), 1.24 (d, J = 7.1 Hz, 3H).

### Step 3: Synthesis of Compound 78

Compound 78-3 (63 mg, 0.12 mmol) was dissolved in N,N-dimethylformamide (2 mL), followed by sequential addition of Compound A (50 mg, 0.12 mmol), 2-(7-azabenzotriazolyl)-N,N,N',N'-tetramethyluronium hexafluorophosphate (55 mg, 0.14 mmol), and N,N-diisopropylethylamine (47 mg, 0.36 mmol) to the reaction mixture. The reaction mixture was stirred at room temperature for 16 hours. The reaction mixture was directly purified by reverse-phase chromatography (acetonitrile/0.05% formic acid containing water = 5%-60%) to obtain the target compound 78 (59 mg, 52%).

LC-MS (ESI⁺) m/z: 920.4 (M+H)⁺;
¹H NMR (400 MHz, CDCl₃) δ 11.39 - 11.03 (m, 1H), 7.92 - 7.26 (m, 6H), 7.13 - 6.96 (m, 2H), 6.85 - 6.63 (m, 2H), 6.46 - 6.20 (m, 1H), 5.79 - 5.19 (m, 1H), 5.03 - 4.32 (m, 1H), 4.14 - 3.66 (m, 2H), 3.65 - 3.36 (m, 1H), 3.27 - 2.85 (m, 3H), 2.44 - 2.06 (m, 6H), 1.79 - 1.49 (m, 10H), 1.38 - 1.25 (m, 6H), 1.19 - 1.04 (m, 3H).

Compounds 79 to 97were prepared according to the preparation method of Example 5.

| **Compound No.** | **Structure** | **LC-MS (ESI⁺) m/z: (M+H)⁺** | **¹H NMR (400 MHz)** |
|---|---|---|---|
| **79** | 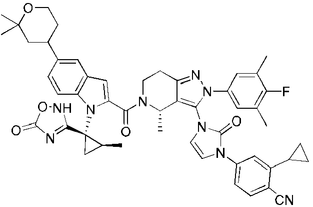 | 876.7 | ¹H NMR (400 MHz, CDCl₃) δ 11.30 - 11.22 (m, 1H), 7.70 - 7.56 (m, 2H), 7.52 - 7.46 (m, 2H), 7.34 - 7.30 (m, 1H), 7.28 - 7.26 (m, 1H), 7.13 - 7.04 (m, 2H), 6.76 - 6.59 (m, 2H), 6.38 - 6.12 (m, 1H), 5.76 - 5.20 (m, 1H), 4.87 - 4.43 (m, 1H), 3.89 - 3.80 (m, 2H), 3.63 - 3.39 (m, 1H), 3.15 - 2.98 (m, 3H), 2.36 - 2.29 (m, 1H), 2.28 - 2.19 (m, 7H), 1.95 - 1.86 (m, 1H), 1.81 - 1.74 (m, 3H), 1.73 - 1.70 (m, 3H), 1.65 - 1.58 (m, 2H), 1.36 - 1.33 (m, 3H), 1.30 - 1.27 (m, 3H), 1.23 - 1.16 (m, 4H), 1.10 - 1.03 (m, 1H), 0.89 - 0.67 (m, 2H). |
| **80** | 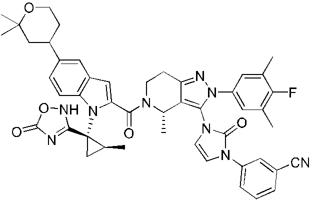 | 836.4 | ¹H NMR (400 MHz, DMSO-d₆) δ 8.21 *(d, J=* 2.6 Hz, 1H), 8.14 (s, 2H), 7.76 (s, 1H), 7.70 (s, 1H), 7.46 (s, 2H), 7.37 (d, *J=* 7.1 Hz, 2H), 7.16 (s, 3H), 7.10 - 7.04 (m, 1H), 4.38 (s, 1H), 3.72 (s, 2H), 2.99 (s, 2H), 2.20 (d, *J =* 0.8 Hz, 6H), 2.16 (s, 1H), 1.68 (s, 4H), 1.60 - 1.47 (m, 4H), 1.27 (s, 6H), 1.24 (s, 5H), 1.17 (s, 3H). |
| **81** | 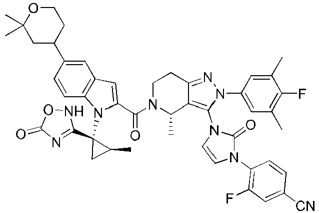 | 854.4 | ¹H NMR (400 MHz, CDCl₃) δ 11.26 (d, *J =* 27.0 Hz, 1H), 7.91 (t, *J =* 7.9 Hz, 1H), 7.65 - 7.41 (m, 4H), 7.37 - 7.27 (m, 1H), 7.25 - 7.24 (m, 1H), 7.15 - 6.97 (m, 2H), 6.78 - 6.56 (m, 2H), 6.42 - 6.11 (m, 1H), 5.83 - 5.18 (m, 1H), 4.94 - 4.27 (m, 1H), 3.95 - 3.75 (m, 2H), 3.68 - 3.34 (m, 1H), 3.22 - 2.92 (m, 3H), 2.33 - 2.13 (m, 6H), 1.95 - 1.71 (m, 4H), 1.65 - 1.59 (m, 2H), 1.53 (d, *J* = 6.4 Hz, 4H), 1.31 (d, *J =* 23.6 Hz, 6H), 1.19 (d, *J =* 5.6 Hz, 2H), 1.09 - 0.93 (m, 1H). |
| **81a** | 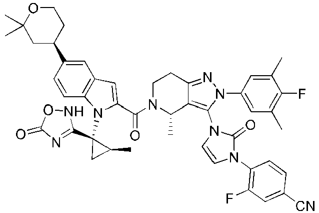 | 854.5 | ¹H NMR (400 MHz, CDCl₃) δ 11.29 (s, 1H), 7.91 (t, *J* = 8.0 Hz, 1H), 7.66 - 7.55 (m, 3H), 7.51 (d, *J* = 1.6 Hz, 1H), 7.27 (d, *J =* 1.8 Hz, 1H), 7.10 (d, *J* = 6.1 Hz, 2H), 6.73 (t, *J* = 3.2 Hz, 1H), 6.70 (s, 1H), 6.36 (d, *J=* 3.3 Hz, 1H), 5.76 (q, *J* = 6.6 Hz, 1H), 4.48 (dd, *J=* 13.8, 5.1 Hz, 1H), 3.92 - 3.78 (m, 2H), 3.64 - 3.53 (m, 1H), 3.25 - 3.09 (m, 1H), 3.10 - 2.94 (m, 2H), 2.26 (d, *J* = 2.1 Hz, 6H), 1.94 - 1.87 (m, 1H), 1.78 (dd, *J* = 9.6, 4.1 Hz, 3H), 1.74 - 1.71 (m, 1H), 1.68 - 1.58 (m, 2H), 1.53 (d, *J* = 6.5 Hz, 3H), 1.34 (s, 3H), 1.29 (d, *J=* 2.4 Hz, 3H), 1.18 (d, *J =* 5.5 Hz, 2H), 1.05 (d, *J* = 6.1 Hz, 1H). |
| **81b** | 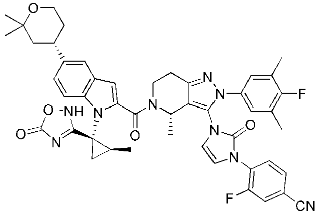 | 854.5 | ¹H NMR (400 MHz, CDCl₃) δ 11.29 (s, 1H), 7.91 (t, *J =* 7.9 Hz, 1H), 7.63 - 7.54 (m, 3H), 7.51 (s, 1H), 7.27 (s, 1H), 7.10 (d, *J =* 6.1 Hz, 2H), 6.76 - 6.65 (m, 2H), 6.36 (d, *J* = 3.2 Hz, 1H), 5.76 (q, *J=* 6.6 Hz, 1H), 4.48 (dd, *J =* 13.8, 5.1 Hz, 1H), 3.87 (s, 2H), 3.59 (t, *J* 1= 11.5 Hz, 1H), 3.21 - 3.08 (m, 1H), 3.01 (d, *J* = 15.9 Hz, 2H), 2.24 (dd, J= 14.5, 2.1 Hz, 6H), 1.95 - 1.88 (m, 1H), 1.83 - 1.75 (m, 3H), 1.73 (d, *J = 3.8* Hz, 1H), 1.70 - 1.58 (m, 2H), 1.53 (d, *J =* 6.6 Hz, 3H), 1.35 (d, *J =* 3.5 Hz, 3H), 1.28 (s, 3H), 1.19 (d,*J =* 5.6 Hz, 2H), 1.05 (d, *J* = 6.1 Hz, 1H). |
| **82a** | 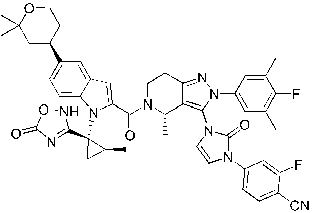 | 854.5 | ¹H NMR (400 MHz, CDCl₃) δ 11.28 (s, 1H), 7.80 - 7.69 (m, 1H), 7.58 (d, *J= 8.6* Hz, 1H), 7.52 - 7.49 (m, 1H), 7.27 (d, *J=* 1.6 Hz, 1H), 7.25 (s, 1H), 7.09 (d, *J=* 6.1 Hz, 2H), 6.81 (d, *J* = 2.8 Hz, 1H), 6.70 (s, 1H), 6.44 - 6.39 (m, 1H), 5.74 (d, *J* = 6.7 Hz, 1H), 4.48 (dd, *J =* 13.6, 4.9 Hz, 1H), 3.86 (q, *J =* 3.3 Hz, 2H), 3.65 - 3.52 (m, 1H), 3.14 (td, *J =* 14.5, 12.5, 5.1 Hz, 1H), 3.02 (s, 2H), 2.23 (dd, *J =* 14.5, 2.1 Hz, 6H), 1.90 (s, 1H), 1.77 (d, *J* = 4.6 Hz, 3H), 1.73 (d, *J* = 3.6 Hz, 1H), 1.68 - 1.62 (m, 1H), 1.58 (q, *J* = 8.6, 7.7 Hz, 1H), 1.55 - 1.51 (m, 1H), 1.50 (d, *J =* 6.5 Hz, 3H), 1.34 (d, *J =* 2.0 Hz, 3H), 1.28 (s, 3H), 1.17 (d, *J =* 5.4 Hz, 2H), 1.05 (d, *J =* 6.2 Hz, 1H). |
| **82b** | 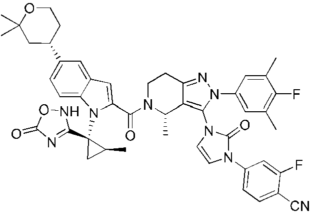 | 854.5 | ¹H NMR (400 MHz, CDCl₃) δ 11.28 (s, 1H), 7.80 - 7.69 (m, 2H), 7.58 (d, *J=* 8.6 Hz, 2H), 7.51 *(d, J=* 1.6 Hz, 1H), 7.27 (s, 1H), 7.09 (d, *J =* 6.1 Hz, 2H), 6.81 *(d, J=* 3.3 Hz, 1H), 6.70 (s, 1H), 6.41 (d, *J* = 3.3 Hz, 1H), 5.74 (q, *J* = 6.6 Hz, 1H), 4.48 (dd, *J* = 13.8, 5.1 Hz, 1H), 3.84 (qd, J = 12.0, 11.5, 6.3 Hz, 2H), 3.59 (td, *J=* 13.1, 3.9 Hz, 1H), 3.15 (ddd, J= 17.5, 12.5, 5.3 Hz, 1H), 3.06 - 2.98 (m, 2H), 2.24 (d, *J =* 2.1 Hz, 6H), 1.96 - 1.87 (m, 1H), 1.77 (dt, *J =* 8.6, 3.6 Hz, 2H), 1.72 (d, *J =* 3.9 Hz, 1H), 1.65 (d, J= 12.7 Hz, 1H), 1.58 (q, *J=* 8.1, 7.5 Hz, 1H), 1.54 - 1.51 (m, 1H), 1.50 (d, *J* = 6.6 Hz, 3H), 1.34 (s, 3H), 1.27 (d, *J* = 5.3 Hz, 3H), 1.17 (d, *J* = 5.5 Hz, 2H), 1.05 (d, *J*= 6.2 Hz, 1H). |
| **83** | 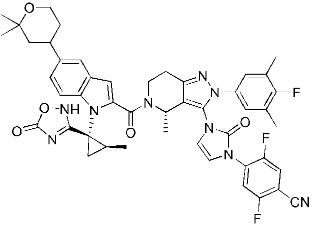 | 872.4 | ¹H NMR (400 MHz, CDCl₃) δ 11.24 (d, *J* = 20.2 Hz, 1H), 7.84 (s, 1H), 7.63 - 7.43 (m, 3H), 7.14 - 6.94 (m, 2H), 6.86 - 6.63 (m, 2H), 6.46 - 6.13 (m, 1H), 5.83 - 5.15 (m, 1H), 4.93 - 4.35 (m, 1H), 3.98 - 3.76 (m, 2H), 3.69 - 3.27 (m, 1H), 3.20 - 2.88 (m, 3H), 2.24 (d, *J=* 14.1 Hz, 6H), 1.97 - 1.70 (m, 4H), 1.69 - 1.60 (m, 2H), 1.56 - 1.42 (m, 5H), 1.31 (d, *J =* 23.7 Hz, 6H), 1.12 (d, *J=* 51.3 Hz, 3H). |
| **84** | 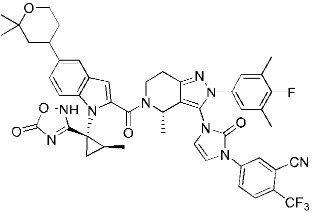 | 904.5 | ¹H NMR (400 MHz, CDCl₃) δ 11.25 (d, *J =* 24.4 Hz, 1H), 8.22 - 7.92 (m, 2H), 7.92 - 7.62 (m, 1H), 7.59 (d, *J =* 8.1 Hz, 1H), 7.49 (d, *J =* 20.9 Hz, 1H), 7.29 - 7.27 (m, 1H), 7.12 - 6.95 (m, 2H), 6.86 - 6.63 (m, 2H), 6.34 (d, *J =* 85.3 Hz, 1H), 5.85 - 5.18 (m, 1H), 5.08 - 4.28 (m, 1H), 3.99 - 3.76 (m, 2H), 3.68 - 3.32 (m, 1H), 3.23 - 2.90 (m, 3H), 2.24 (d, *J* = 14.8 Hz, 6H), 1.91 - 1.72 (m, 4H), 1.63 - 1.47 (m, 6H), 1.35 - 1.25 (m, 6H), 1.19 - 1.04 (m, 3H). |
| **85** | 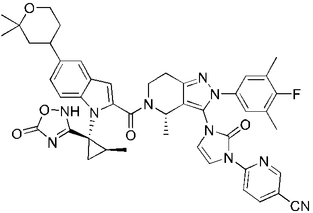 | 837.5 | ¹H NMR (400 MHz, CDCl₃) δ 11.30 (s, 1H), 8.73 - 8.65 (m, 2H), 8.11 - 8.00 (m, 1H), 7.59 (dd, *J* = 10.0, 3.8 Hz, 1H), 7.51 (d, *J* = 1.5 Hz, 1H), 7.30 - 7.22 (m, 1H), 7.08 (d, *J* = 6.1 Hz, 2H), 6.99 *(d, J=* 6.1 Hz, 1H), 6.70 (s, 1H), 6.35 (d, *J* = 3.4 Hz, 1H), 5.78 (q, *J = 6.6* Hz, 1H), 4.49 (dd, *J* = 13.9, 5.1 Hz, 1H), 3.92 - 3.80 (m, 2H), 3.57 (td, *J=* 13.2, 3.8 Hz, 1H), 3.22 - 3.02 (m, 1H), 3.02 (s, 2H), 2.24 (d, *J* = 2.1 Hz, 6H), 1.89 (d, *J* = 5.4 Hz, 1H), 1.82 - 1.74 (m, 2H), 1.73 (d, *J* = 3.8 Hz, 2H), 1.64 (dd, *J* = 15.1, 10.6 Hz, 1H), 1.56 (d, *J* = 6.7 Hz, 1H), 1.54 - 1.51 (m, 1H), 1.47 (d, *J* = 6.7 Hz, 3H), 1.34 (d, *J* = 4.2 Hz, 3H), 1.27 (d, *J* = 6.6 Hz, 3H), 1.16 (d, *J* = 5.4 Hz, 2H), 1.06 (d, *J=* 6.2 Hz, 1H). |
| **86** | 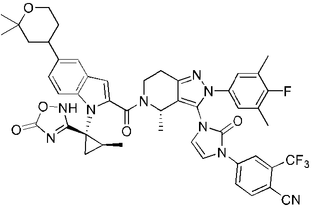 | 904.4 | ¹H NMR (400 MHz, CDCl₃) δ 11.31 - 11.20 (m, 1H), 8.13 - 7.96 (m, 2H), 7.95 - 7.73 (m, 1H), 7.61 - 7.57 (m, 1H), 7.52 - 7.44 (m, 1H), 7.30 - 7.26 (m, 1H), 7.11 - 6.98 (m, 2H), 6.88 - 6.64 (m, 2H), 6.50 - 6.22 (m, 1H), 5.83 - 5.18 (m, 1H), 4.89 - 4.46 (m, 1H), 3.90 - 3.81 (m, 2H), 3.63 - 3.39 (m, 1H), 3.18 - 2.99 (m, 3H), 2.27 - 2.21 (m, 6H), 1.93 - 1.87 (m, 1H), 1.79 - 1.74 (m, 4H), 1.66 - 1.57 (m, 2H), 1.53 - 1.49 (m, 3H), 1.36 - 1.33 (m, 3H), 1.31 - 1.27 (m, 3H), 1.18 - 1.04 (m, 3H). |
| **87** | 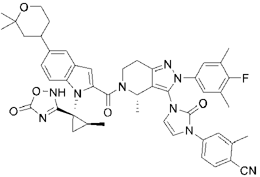 | 850.5 | ¹H NMR (400 MHz, CDCl₃) δ 11.32 - 11.21 (m, 1H), 7.72 - 7.26 (m, 6H), 7.13 - 6.99 (m, 2H), 6.79 - 6.62 (m, 2H), 6.40 - 6.14 (m, 1H), 5.78 - 5.23 (m, 1H), 4.93 - 4.36 (m, 1H), 3.91 - 3.80 (m, 2H), 3.68 - 3.31 (m, 1H), 3.21 - 2.98 (m, 3H), 2.63 - 2.48 (m, 3H), 2.26 - 2.20 (m, 6H), 1.91 - 1.72 (m, 4H), 1.67 - 1.64 (m, 1H), 1.54 - 1.48 (m, 4H), 1.35 - 1.28 (m, 6H), 1.27 - 1.25 (m, 1H), 1.18 - 1.04 (m, 3H). |
| **88** | 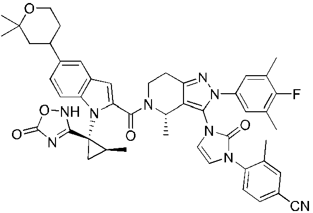 | 850.8 | ¹H NMR (400 MHz, CDCl₃) δ 11.24 - 11.15 (m, 1H), 7.58 - 7.43 (m, 4H), 7.39 - 7.20 (m, 2H), 7.07 - 6.95 (m, 2H), 6.63 - 6.59 (m, 1H), 6.43 - 6.09 (m, 2H), 5.70 - 5.34 (m, 1H), 4.81 - 4.37 (m, 1H), 3.83 - 3.74 (m, 2H), 3.56 - 3.28 (m, 1H), 3.11 - 2.92 (m, 3H), 2.23 - 2.16 (m, 9H), 1.82 - 1.68 (m, 4H), 1.58 - 1.57 (m, 1H), 1.49 - 1.46 (m, 4H), 1.28 - 1.26 (m, 3H), 1.22 - 1.20 (m, 3H), 1.19 - 1.18 (m, 1H), 1.13 - 0.97 (m, 3H). |
| **89** | 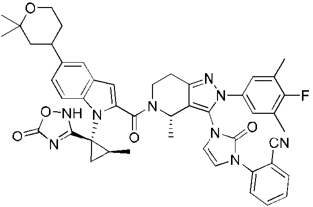 | 836.5 | ¹H NMR (400 MHz, CDCl₃) δ 11.33 - 11.26 (m, 1H), 7.82 - 7.68 (m, 2H), 7.66 - 7.49 (m, 4H), 7.25 - 7.22 (m, 1H), 7.15 - 7.03 (m, 2H), 6.75 - 6.61 (m, 2H), 6.37 - 6.12 (m, 1H), 5.83 - 5.33 (m, 1H), 4.93 - 4.42 (m, 1H), 3.90 - 3.81 (m, 2H), 3.61 - 2.97 (m, 4H), 2.29 - 2.24 (m, 6H), 2.05 - 1.88 (m, 1H), 1.82 - 1.71 (m, 4H), 1.68 - 1.64 (m, 2H), 1.54 - 1.49 (m, 2H), 1.35 - 1.33 (m, 3H), 1.29 - 1.28 (m, 3H), 1.26 - 1.25 (m, 1H), 1.20 - 1.06 (m, 3H). |
| **90** | 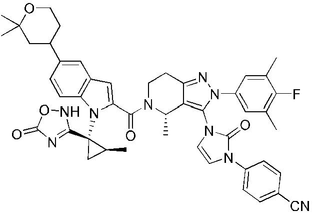 | 836.5 | ¹H NMR (400 MHz, CDCl₃) δ 11.33 - 11.19 (m, 1H), 7.84 - 7.76 (m, 3H), 7.60 - 7.45 (m, 3H), 7.29 - 7.27 (m, 1H), 7.16 - 6.98 (m, 2H), 6.81 - 6.63 (m, 2H), 6.43 - 6.11 (m, 1H), 5.81 - 5.22 (m, 1H), 4.95 - 4.27 (m, 1H), 3.91 - 3.80 (m, 2H), 3.77 - 3.26 (m, 1H), 3.21 - 2.94 (m, 3H), 2.27 - 2.20 (m, 6H), 1.92 - 1.72 (m, 4H), 1.69 - 1.60 (m, 2H), 1.54 - 1.49 (m, 4H), 1.36 - 1.33 (m, 3H), 1.30 - 1.27 (m, 3H), 1.19 - 1.04 (m, 3H). |
| **91** | 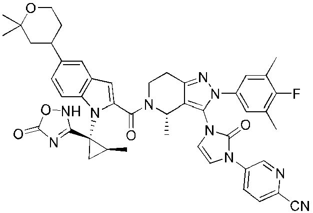 | 837.5 | ¹H NMR (400 MHz, CDCl₃) δ 11.29 - 11.20 (m, 1H), 8.96 - 8.69 (m, 1H), 8.49 - 7.94 (m, 1H), 7.85 - 7.27 (m, 4H), 7.11 - 6.98 (m, 2H), 6.88 - 6.63 (m, 2H), 6.48 - 6.22 (m, 1H), 5.82 - 5.21 (m, 1H), 4.89 - 4.46 (m, 1H), 3.90 - 3.82 (m, 2H), 3.66 - 3.37 (m, 1H), 3.17 - 2.98 (m, 3H), 2.26 - 2.21 (m, 6H), 1.94 - 1.87 (m, 1H), 1.78 - 1.72 (m, 3H), 1.68 - 1.66 (m, 1H), 1.65 - 1.63 (m, 2H), 1.53 - 1.50 (m, 3H), 1.35 - 1.33 (m, 3H), 1.29 - 1.28 (m, 3H), 1.18 - 1.04 (m, 3H). |
| **92** | 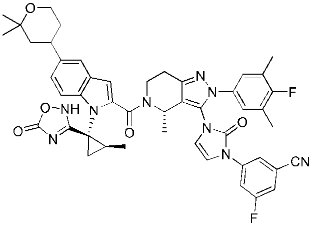 | 854.5 | ¹H NMR (400 MHz, CDCl₃) δ 11.30 - 11.21 (m, 1H), 7.83 - 7.47 (m, 4H), 7.31 - 7.27 (m, 1H), 7.26 - 7.20 (m, 1H), 7.13 - 6.99 (m, 2H), 6.78 - 6.61 (m, 2H), 6.44 - 6.18 (m, 1H), 5.85 - 4.39 (m, 2H), 3.90 - 3.79 (m, 2H), 3.68 - 3.35 (m, 1H), 3.18 - 2.97 (m, 3H), 2.27 - 2.21 (m, 6H), 1.93 - 1.83 (m, 1H), 1.80 - 1.72 (m, 3H), 1.69 - 1.66 (m, 1H), 1.66 - 1.64 (m, 2H), 1.53 - 1.50 (m, 3H), 1.35 - 1.32 (m, 3H), 1.30 - 1.26 (m, 3H), 1.19 - 1.05 (m, 3H). |
| **93** | 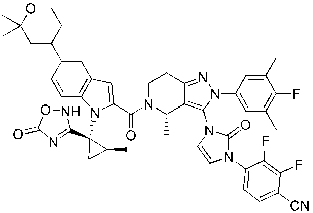 | 872.4 | ¹H NMR (400 MHz, CDCl₃) δ 11.29 - 11.20 (m, 1H), 8.02 - 7.67 (m, 1H), 7.61 - 7.57 (m, 1H), 7.56 - 7.49 (m, 2H), 7.29 - 7.27 (m, 1H), 7.10 - 6.99 (m, 2H), 6.75 - 6.62 (m, 2H), 6.39 - 6.16 (m, 1H), 5.78 - 5.22 (m, 1H), 4.88 - 4.46 (m, 1H), 3.89 - 3.82 (m, 2H), 3.62 - 3.37 (m, 1H), 3.15 - 2.99 (m, 3H), 2.27 - 2.23 (m, 6H), 1.94 - 1.88 (m, 1H), 1.80 - 1.75 (m, 3H), 1.64 - 1.60 (m, 3H), 1.54 - 1.51 (m, 3H), 1.35 - 1.34 (m, 3H), 1.29 - 1.28 (m, 3H), 1.19 - 1.04 (m, 3H). |
| **94** | 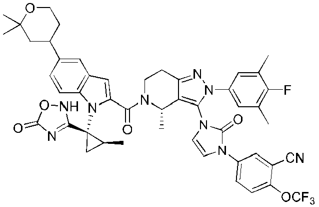 | 920.3 | ¹H NMR (400 MHz, CDCl₃) δ 11.39 - 11.18 (m, 1H), 8.04 - 7.66 (m, 2H), 7.61 - 7.56 (m, 1H), 7.54 - 7.31 (m, 2H), 7.12 - 6.98 (m, 2H), 6.80 - 6.60 (m, 2H), 6.48 - 6.14 (m, 1H), 5.84 - 5.16 (m, 1H), 4.92 - 4.27 (m, 1H), 3.91 - 3.78 (m, 2H), 3.71 - 3.33 (m, 1H), 3.19 - 2.96 (m, 3H), 2.28 - 2.20 (m, 6H), 2.07 - 1.87 (m, 1H), 1.84 - 1.64 (m, 5H), 1.59 - 1.56 (m, 1H), 1.54 - 1.47 (m, 4H), 1.35 - 1.32 (m, 3H), 1.29 - 1.25 (m, 3H), 1.19 - 1.05 (m, 3H). |
| **95** | 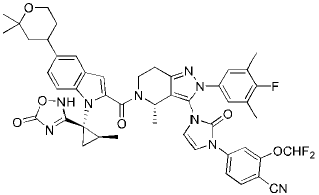 | 902.5 | ¹H NMR (400 MHz, CDCl₃) δ 11.30 - 11.20 (m, 1H), 7.80 - 7.77 (m, 1H), 7.72 -7.30 (m, 4H), 7.11 - 6.98 (m, 2H), 6.91 - 6.76 (m, 1H), 6.72 - 6.50 (m, 2H), 6.44 - 6.18 (m, 1H), 5.77 - 5.22 (m, 1H), 4.87 - 4.32 (m, 1H), 3.92 - 3.54 (m, 3H), 3.42 - 3.00 (m, 3H), 2.25 - 2.20 (m, 6H), 2.03 - 1.89 (m, 1H), 1.77 - 1.65 (m, 6H), 1.54 - 1.48 (m, 4H), 1.34 - 1.27 (m, 6H), 1.18 - 1.03 (m, 3H). |
| **96** | 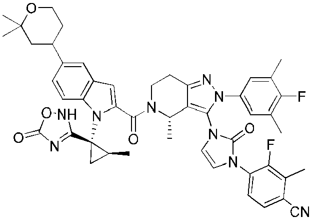 | 868.3 | ¹H NMR (400 MHz, CDCl₃) δ 11.35 - 11.18 (m, 1H), 7.66 - 7.28 (m, 4H), 7.26 - 7.24 (m, 1H), 7.13 - 7.00 (m, 2H), 6.74 - 6.53 (m, 2H), 6.38 - 6.12 (m, 1H), 5.80 - 5.19 (m, 1H), 4.90 - 4.42 (m, 1H), 3.91 - 3.35 (m, 3H), 3.20 - 2.95 (m, 3H), 2.59 - 2.44 (m, 3H), 2.29 - 2.21 (m, 6H), 1.94 - 1.67 (m, 5H), 1.65 - 1.57 (m, 3H), 1.56 - 1.54 (m, 2H), 1.36 - 1.27 (m, 6H), 1.21 - 1.03 (m, 3H). |
| **97** | 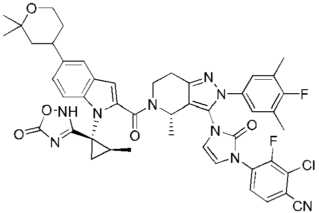 | 888.4 | ¹H NMR (400 MHz, CDCl₃) δ 11.30 - 11.17 (m, 1H), 7.84 - 7.60 (m, 2H), 7.58 - 7.38 (m, 2H), 7.29 - 7.27 (m, 1H), 7.10 - 6.99 (m, 2H), 6.75 - 6.60 (m, 2H), 6.45 - 6.12 (m, 1H), 5.88 - 5.22 (m, 1H), 4.87 - 4.45 (m, 1H), 3.90 - 3.81 (m, 2H), 3.62 - 2.99 (m, 4H), 2.28 - 2.23 (m, 6H), 1.93 - 1.71 (m, 5H), 1.54 - 1.51 (m, 3H), 1.34 (s, 3H), 1.28 (s, 3H), 1.26 - 1.04 (m, 5H). |

### Biological Test Evaluation

### I. cAMP Level Determination in Human GLP-1R Stably Transfected HEK293T Cells

### 1. Experimental Equipment

| **Equipment Name** | **Manufacturer** | **Model** |
|---|---|---|
| Microplate Reader | Molecula Devices | Spectramax i3 |
| Desktop Centrifuge | BECKMAN | Avanti J15R |
| Mini Vortex Mixer | Titan | VM-M1 |
| A2 Type Class II Biosafety Cabinet-Double | ESCO | AC2-658-CN |
| Incubator | Thermo | thermofisher 371 |
| Water Bath | Yiheng | DK-8AX |
| Microscope | Leica | Dmi 1 |
| Cell Counter | BIO-RAD | / |

### 2. Experimental Materials

| Reagent / consumable material | Manufacturer/Source | Catalog No. |
|---|---|---|
| Human GLP-1R Stably Transfected HEK293T Cell | Wuhan Huamei | / |
| GLP-1(7-37) acetate | MCE | HY-P0055A |
| DMEM Medium | GIBCO | C11995500BT |
| IBMX | MCE | HY-12318 |
| CAMP - GS HIRANGE KIT | CISBIO | 62AM6PEC |
| 384-well White Shallow Plate | Corning | 4513 |
| Distilled Water | Watsons | / |
| Trypsin | GIBCO | 25200-056 |
| PBS | GIBCO | C10010500BT |
| FBS | EXCELL | FND500 |
| DMSO | Shanghai Sangon | A503039-0250 |
| Trypan Blue Stain | GIBCO | 15250-061 |
| Cell counting plate | Neubauer | TS105-YA0810 |

### 3. Experimental Method

Human GLP-1R HEK293T stably transfected cells were digested with trypsin, terminated with DMEM total (without p/s), centrifuged and washed twice with 1X Stimulation buffer, then resuspended in 1X stimulation buffer and counted. The cells were then prepared as a 1 Million/mL cell suspension in stimulation buffer. Compound powder was first prepared as a 10 mM stock solution in DMSO, then diluted to 2 mM with DMSO, and further prepared as 2X compound in 1X stimulation buffer (Cisbio kit buffer with freshly added IBMX at a final concentration of 0.5 mM). The top dose final concentration was 2000 nM, with a final DMSO concentration of 0.2%, diluted in a 1:3 gradient for 11 gradients.

According to the layout, 5 µl of compound was added to each well; Min wells contained 100 nM GLP-1, and Max wells contained 0.2% DMSO in 1X stimulation buffer. 5 µl of cell suspension was added to each well, centrifuged at 300 rpm at room temperature for 1 min, then incubated at 37°C for 30 min. 2X cAMP standards were prepared in 1X Stimulation buffer, with a top final concentration of 2.8 µM, diluted in a 1:3 gradient for 12 doses. According to the layout, 10 µl was added to each well. Finally, d2 and antibody were diluted 20-fold in 1X Detection buffer (Cisbio kit buffer), and 5 µl of each was added to each well according to the layout. After centrifugation at 300 rpm at room temperature for 1 min, the plate was incubated at room temperature for 2 hr.

### 4. Data Analysis

Stimulation% was calculated based on Min and Max readings (665nm/615nm ratio) using the formula: 100*(ratio Max well - ratio test well)/(ratio Max well - ratio Min well). The cAMP concentration in each test well was derived from the cAMP standard curve, and the data was analyzed using GraphPad Prism 9 to plot four-parameter fit curves and calculate EC₅₀ values using the formula: Y=Bottom + (Top-Bottom)/(1+10^((LogEC₅₀-X)*HillSlope)). Results are shown in Table 1.

**Table 1: cAMP Level Determination in Human GLP-1R Stably Transfected HEK293T Cells**

| **Compound No.** | **EC₅₀ (nM)** |
|---|---|
| **1a** | 35.52 |
| **1b** | 17.53 |
| **2** | 56.48 |
| **3** | 26.52 |
| **4** | 34.32 |
| **5** | 7.69 |
| **6** | 5.16 |
| **7** | 6.12 |
| **8** | 4.75 |
| **9** | 4.85 |
| **10** | 8.55 |
| **11** | 5.03 |
| **12** | 9.58 |
| **14** | 5.61 |
| **15** | 7.27 |
| **16** | 10.03 |
| **17** | 8.97 |
| **19** | 16.19 |
| **21** | 14.39 |
| **22** | 50.66 |
| **23** | 13.78 |
| **24** | 54.17 |
| **25** | 11.79 |
| **26** | 11.47 |
| **29** | 102.40 |
| **30** | 205.90 |
| **31** | 15.07 |
| **32** | 79.73 |
| **34** | 7.44 |
| **35** | 19.98 |
| **36** | 67.78 |
| **37** | 18.79 |
| **38** | 18.09 |
| **39** | 11.31 |
| **40** | 154.80 |
| **43** | 13.15 |
| **44** | 16.93 |
| **45** | 41.03 |
| **46** | 77.88 |
| **47** | 65.10 |
| **50** | 11.57 |
| **51** | 8.76 |
| **52** | 11.02 |
| **53** | 25.53 |
| **54** | 4.48 |
| **55** | 8.07 |
| **56a** | 241.20 |
| **56b** | 5.59 |
| **57** | 15.78 |
| **58** | 9.93 |
| **59** | 15.04 |
| **60** | 10.62 |
| **61** | 10.82 |
| **62** | 13.52 |
| **62a** | 222.60 |
| **62b** | 4.53 |
| **63** | 8.69 |
| **63a** | 145.50 |
| **63b** | 6.40 |
| **64** | 29.01 |
| **65** | 647.60 |
| **66** | 67.78 |
| **67** | 67.78 |
| **67a** | 51.40 |
| **67b** | 11.24 |
| **68** | 17.98 |
| **69** | 10.33 |
| **70** | 3.55 |
| **71** | 4.01 |
| **72** | 9.71 |
| **73** | 6.25 |
| **74** | 3.25 |
| **75** | 12.95 |
| **76** | 8.18 |
| **77** | 6.19 |
| **78** | 20.28 |
| **79** | 17.77 |
| **80** | 11.51 |
| **81** | 11.03 |
| **81a** | 386.7 |
| **81b** | 3.92 |
| **82a** | 458.60 |
| **82b** | 6.40 |
| **83** | 12.85 |
| **84** | 9.87 |
| **85** | 50.21 |
| **86** | 13.79 |
| **87** | 17.63 |
| **88** | 205.00 |
| **89** | 200.60 |
| **90** | 15.07 |
| **91** | 38.41 |
| **92** | 43.47 |
| **93** | 15.08 |
| **94** | 21.59 |
| **95** | 17.63 |
| **96** | 7.37 |
| **97** | 7.84 |
| **Compound a** | 7.32 |
| **Compound b** | 11.29 |

### II. Pharmacokinetic Analysis of Compounds in Mouse Plasma

### 1. Experimental Materials

Male C57BL/6J mice, approximately 30 g, 6-9 weeks old, gavage oral administration and intravenous injection: 3 mice per compound, animals from Shanghai Medicilon Inc.

### 2. Experimental Method

The pharmacokinetic characteristics of the compounds were tested in mice after oral gavage (PO) or intravenous injection (IV) using a standard protocol. The test compounds were prepared as clear solutions with the vehicle: 10% (v/v) DMSO + 10% (v/v) Solutol + 80% (v/v) Saline. Three mice were administered a single oral gavage dose of 5 mg/kg, and blood samples were collected at 0.25, 0.5, 1, 2, 4, 6, 8, and 24 hours post-dose via submandibular vein or other appropriate methods. Each sample collected was approximately 0.03 mL, anticoagulated with sodium heparin, and placed on ice after collection. Additionally, three mice were administered a single intravenous injection of 1 mg/kg of the test compound, and blood samples were collected at 0.083, 0.25, 0.5, 1, 2, 4, 8, and 24 hours post-dose via submandibular vein or other appropriate methods. Each sample collected was approximately 0.03 mL, anticoagulated with sodium heparin, and placed on ice after collection. Plasma was separated by centrifugation within 1 hour (centrifugation conditions: 6800g, 6 minutes, 2-8°C), and the concentration of the compounds of the present invention was analyzed by LC-MS/MS method.

Prior to analysis, all samples were stored at -80°C, and pharmacokinetic parameters were calculated using Phoenix WinNonlin. The pharmacokinetic parameter results are shown in Table 2.

**Table 2: Pharmacokinetic Parameters**

| **Compound** | **Dose (mg/kg)** | **Tmax (h)** | **Cmax (ng/mL)** | **AUC(0-t) (h*ng/mL)** | **T_{1/2 (h)}** | **Cl (mL/min/kg)** | **F (%)** |
|---|---|---|---|---|---|---|---|
| **5** | 5 | 1.33 | 3882.65 | 36661.38 | 3.11 | 2.38 | 105.86 |
| **7** | 5 | 1.33 | 3116.62 | 34661.78 | 3.41 | 1.94 | 81.76 |
| **11** | 5 | 3 | 3173.20 | 35203.44 | 4.27 | 2.02 | 87.27 |
| Compound **A** | 5 | 1 | 2550.58 | 16300.88 | 2.51 | 4.29 | 85.15 |
| Compound **B** | 5 | 1 | 2084 | 6902.89 | 0.89 | 7.95 | 66.45 |

As can be seen, the compounds of the present invention have longer half-lives in mouse blood compared to the positive control compounds, with significantly increased exposure, suggesting the potential for improved efficacy.

All documents mentioned in the present invention are incorporated herein by reference as if each document is cited individually as a reference. Furthermore, it should be understood that after reading the above teachings of the present invention, those skilled in the art can make various modifications or alterations to the present invention, and these equivalent forms also fall within the scope defined by the appended claims.

## Claims

1. A compound of formula (I), a stereoisomer, a tautomer, or a pharmaceutically acceptable salt thereof, 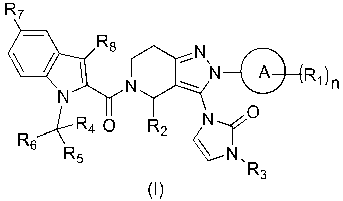 wherein,
ring A is selected from a group consisting of a 3-12 membered heterocyclyl, C₆₋₁₀ aryl, and 5-10 membered heteroaryl;
R₁ is selected from a group consisting of cyano, halogen, C₁₋₁₀ alkyl, C₃₋₁₂ cycloalkyl, 3-12 membered heterocyclyl, halogenated C₁₋₁₀ alkyl, halogenated C₃₋₁₂ cycloalkyl, halogenated 3-12 membered heterocyclyl, halogenated C₁₋₁₀ alkoxy, halogenated C₁₋₁₀ alkylthio, C₁₋₆ alkyl-substituted C₃₋₁₂ cycloalkyl, and C₁₋₆ alkyl-substituted 3-12 membered heterocyclyl;
R₂ is selected from a group consisting of hydrogen, cyano, C₁₋₁₀ alkyl, halogenated C₁₋₁₀ alkyl, C₁₋₁₀ alkoxy, halogenated C₁₋₁₀ alkoxy, and -NR₁₂R₁₃;
R₃ is selected from a group consisting of C₃₋₁₂ cycloalkyl, 3-12 membered heterocyclyl, C₆₋₁₀ aryl, and 5-10 membered heteroaryl, wherein above groups are optionally further substituted with one or more substituents selected from a group consisting of halogen, cyano, nitro, azido, C₁₋₁₀ alkyl, halogenated C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, -SF₅, -S(O)ᵣR₉, -O-R₁₀, -C(O)OR₁₀, - C(O)R₁₁, -O-C(O)R₁₁, -NR₁₂R₁₃, -C(=NR₁₂)R₁₁, -N(R₁₂)-C(=NR₁₃)R₁₁, -C(O)NR₁₂R₁₃, and - N(R₁₂)-C(O)R₁₁;
R₄ and R₅ are each independently selected from a group consisting of hydrogen, halogen, hydroxyl, C₁₋₁₀ alkyl, halogenated C₁₋₁₀ alkyl, C₁₋₁₀ alkoxy, and halogenated C₁₋₁₀ alkoxy, or R₄ and R₅ together with a carbon atom to which they are directly attached form a C₃₋₁₂ cycloalkyl, wherein said C₃₋₁₂ cycloalkyl is optionally further substituted with one or more substituents selected from a group consisting of hydrogen, halogen, amino, hydroxyl, C₁₋₁₀ alkyl, halogenated C₁₋₁₀ alkyl, C₁₋₁₀ alkoxy, and halogenated C₁₋₁₀ alkoxy;
R₆ is 5-10 membered heteroaryl;
R₇ is 3-12 membered heterocyclyl or 5-10 membered heteroaryl, wherein above groups are optionally substituted with one or more substituents selected from a group consisting of halogen, hydroxyl, cyano, nitro, C₁₋₁₀ alkyl, halogenated C₁₋₁₀ alkyl, C₁₋₁₀ alkoxy, halogenated C₁₋₁₀ alkoxy, and -NR₁₂R₁₃, or any two substituents on the same carbon atom of said 3-12 membered heterocyclyl together with a carbon atom to which they are attached form a C₃₋₆ cycloalkyl, wherein said C₃₋₆ cycloalkyl is optionally further substituted with one or more substituents selected from a group consisting of halogen, cyano, C₁₋₆ alkyl, halogenated C₁₋₆ alkyl, and C₁₋₆ alkoxy;
R₈ is selected from a group consisting of hydrogen, cyano, C₃₋₁₂ cycloalkyl, 3-12 membered heterocyclyl, C₁₋₁₀ alkyl, halogenated C₁₋₁₀ alkyl, C₁₋₁₀ alkoxy, halogenated C₁₋₁₀ alkoxy, and - NR₁₂R₁₃;
each R₉ is independently selected from a group consisting of hydrogen, deuterium, hydroxyl, C₁₋₁₀ alkyl, C₂₋₁₀ alkenyl, C₃₋₁₂ cycloalkyl, 3-12 membered heterocyclyl, C₆₋₁₀ aryl, 5-10 membered heteroaryl, and -NR₁₂R₁₃, wherein above groups are independently optionally further substituted with one or more substituents selected from a group consisting of deuterium, halogen, hydroxyl, oxo, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocycloxy, C₆₋₁₀ aryl, C₆₋₁₀ aryloxy, 5-10 membered heteroaryl, 5-10 membered heteroaryloxy, and -NR₁₂R₁₃;
each R₁₀ is independently selected from a group consisting of hydrogen, deuterium, C₁₋₁₀ alkyl, C₂₋₁₀ alkenyl, C₃₋₁₂ cycloalkyl, 3-12 membered heterocyclyl, C₆₋₁₀ aryl, and 5-10 membered heteroaryl, wherein above groups are independently optionally further substituted with one or more substituents selected from a group consisting of deuterium, halogen, hydroxyl, oxo, cyano, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocycloxy, C₆₋₁₀ aryl, C₆₋₁₀ aryloxy, 5-10 membered heteroaryl, 5-10 membered heteroaryloxy, and -NR₁₂R₁₃;
each R₁₁ is selected from a group consisting of hydrogen, deuterium, hydroxyl, C₁₋₁₀ alkyl, C₁₋₁₀ alkoxy, C₂₋₁₀ alkenyl, C₂₋₁₀ alkynyl, C₃₋₁₂ cycloalkyl, C₃₋₁₂ cycloalkoxy, 3-12 membered heterocyclyl, 3-12 membered heterocycloxy, C₆₋₁₀ aryl, C₆₋₁₀ aryloxy, 5-10 membered heteroaryl, 5-10 membered heteroaryloxy, and -NR₁₂R₁₃, wherein above groups are independently optionally further substituted with one or more substituents selected from a group consisting of deuterium, halogen, hydroxyl, cyano, C₁₋₁₀ alkyl, halogenated C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocycloxy, C₆₋₁₀ aryl, C₆₋₁₀ aryloxy, 5-10 membered heteroaryl, 5-10 membered heteroaryloxy, and -NR₁₂R₁₃;
each R₁₂ and each R₁₃ are independently selected from a group consisting of hydrogen, deuterium, hydroxyl, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, sulfinyl, sulfonyl, methanesulfonyl, isopropylsulfonyl, cyclopropylsulfonyl, p-toluenesulfonyl, aminosulfonyl, dimethylaminosulfonyl, amino, mono-C₁₋₁₀ alkylamino, di-C₁₋₁₀ alkylamino, and C₁₋₁₀ alkanoyl, wherein above groups are independently optionally further substituted with one or more substituents selected from a group consisting of deuterium, halogen, hydroxyl, C₁₋₁₀ alkyl, C₂₋₁₀ alkenyl, C₂₋₁₀ alkynyl, halogenated C₁₋₁₀ alkyl, deuterium-substituted C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocycloxy, C₆₋₁₀ aryl, C₆₋₁₀ aryloxy, 5-10 membered heteroaryl, 5-10 membered heteroaryloxy, amino, mono-C₁₋₁₀ alkylamino, di-C₁₋₁₀ alkylamino, and C₁₋₁₀ alkanoyl;
or R₁₂ and R₁₃ together with a nitrogen atom to which they are directly attached form a 4-10 membered heterocyclyl or 4-10 membered heteroaryl, wherein said 4-10 membered heterocyclyl or 4-10 membered heteroaryl is optionally further substituted with one or more substituents selected from a group consisting of deuterium, halogen, hydroxyl, C₁₋₁₀ alkyl, C₂₋₁₀ alkenyl, C₂₋₁₀ alkynyl, halogenated C₁₋₁₀ alkyl, deuterium-substituted C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocycloxy, C₆₋₁₀ aryl, C₆₋₁₀ aryloxy, 5-10 membered heteroaryl, 5-10 membered heteroaryloxy, amino, mono-C₁₋₁₀ alkylamino, di-C₁₋₁₀ alkylamino, and C₁₋₁₀ alkanoyl;
with the proviso that when A is selected from a group consisting of C₆₋₁₀ aryl and 5-10 membered heteroaryl, n is not equal to 0, at least one R₁ is selected from a group consisting of cyano, C₃₋₁₂ cycloalkyl, 3-12 membered heterocyclyl, halogenated C₃₋₁₂ cycloalkyl, halogenated 3-12 membered heterocyclyl, halogenated C₁₋₁₀ alkoxy, C₁₋₆ alkyl-substituted C₃₋₁₂ cycloalkyl, and C₁₋₆ alkyl-substituted 3-12 membered heterocyclyl,
or when A is selected from a group consisting of C₆₋₁₀ aryl and 5-10 membered heteroaryl, n is not equal to 0, and R₁ is only selected from halogen and/or C₁₋₁₀ alkyl and/or halogenated C₁₋₁₀ alkyl, and R₃ is selected from a group consisting of C₆₋₈ aryl and 5-6 membered heteroaryl, R₃ is substituted with at least one substituent selected from a group consisting of cyano, -NR₁₂R₁₃, - C(O)NR₁₂R₁₃, and -N(R₁₂)-C(O)R₁₁, wherein in said -NR₁₂R₁₃ and -C(O)NR₁₂R₁₃, R₁₂ or R₁₃ is selected from a group consisting of C₃₋₁₂ cycloalkyl, wherein in said -N(R₁₂)-C(O)R₁₁, R₁₁ or R₁₂ is C₃₋₁₂ cycloalkyl, wherein said C₃₋₁₂ cycloalkyl is independently optionally further substituted with one or more substituents selected from a group consisting of deuterium, halogen, hydroxyl, cyano, C₁₋₁₀ alkyl, halogenated C₁₋₁₀ alkyl, and C₁₋₁₀ alkoxy;
each r is independently 0, 1, or 2;
n is 0, 1, 2, 3, or 4.

2. The compound of claim 1, stereoisomer, tautomer, or pharmaceutically acceptable salt thereof, wherein ring A is selected from a group consisting of 3-6 membered heterocyclyl, C₆₋₈ aryl, and 5-8 membered heteroaryl;
R₁ is selected from a group consisting of cyano, halogen, C₁₋₆ alkyl, C₃₋₆ cycloalkyl, 3-6 membered heterocyclyl, halogenated C₁₋₆ alkyl, halogenated C₃₋₆ cycloalkyl, halogenated 3-6 membered heterocyclyl, halogenated C₁₋₆ alkoxy, halogenated C₁₋₆ alkylthio, C₁₋₆ alkyl-substituted C₃₋₆ cycloalkyl, and C₁₋₆ alkyl-substituted 3-6 membered heterocyclyl;
R₂ is selected from a group consisting of hydrogen, C₁₋₆ alkyl, halogenated C₁₋₆ alkyl, C₁₋₆ alkoxy, and halogenated C₁₋₆ alkoxy;
R₃ is selected from a group consisting of 5-10 membered heteroaryl and C₆₋₁₀ aryl, wherein above groups are optionally further substituted with one or more substituents selected from a group consisting of halogen, cyano, nitro, hydroxyl, C₁₋₆ alkyl, halogenated C₁₋₆ alkyl, C₃₋₆ cycloalkyl, C₁₋₆ alkoxy, halogenated C₁₋₆ alkoxy, -S(O)ᵣR₉, -C(O)NR₁₂R₁₃, -N(R₁₂)-C(O)R₁₁, and -NR₁₂R₁₃;
R₄ and R₅ together with a carbon atom to which they are directly attached form a C₃₋₆ cycloalkyl, wherein said C₃₋₆ cycloalkyl is optionally further substituted with one or more substituents selected from a group consisting of hydrogen, halogen, amino, hydroxyl, C₁₋₆ alkyl, and halogenated C₁₋₆ alkyl;
R₆ is 5-6 membered heteroaryl;
R₇ is 3-6 membered heterocyclyl, wherein said 3-6 membered heterocyclyl is optionally substituted with one or more substituents selected from a group consisting of halogen, hydroxyl, cyano, nitro, C₁₋₆ alkyl, and halogenated C₁₋₆ alkyl, or any two substituents on the same carbon atom of said 3-6 membered heterocyclyl together with a carbon atom to which they are attached form a C₃₋₆ cycloalkyl, wherein said C₃₋₆ cycloalkyl is optionally further substituted with one or more substituents selected from a group consisting of halogen, cyano, C₁₋₆ alkyl, halogenated C₁₋₆ alkyl, and C₁₋₆ alkoxy;
R₈ is selected from a group consisting of hydrogen, cyano, C₃₋₆ cycloalkyl, 3-6 membered heterocyclyl, C₁₋₆ alkyl, halogenated C₁₋₆ alkyl, C₁₋₆ alkoxy, halogenated C₁₋₆ alkoxy, and -NR₁₂R₁₃
R₁₁, R₁₂, and R₁₃ are each independently selected from a group consisting of hydrogen, deuterium, C₁₋₆ alkyl, and C₃₋₆ cycloalkyl, wherein said C₃₋₆ cycloalkyl is optionally further substituted with one or more substituents selected from a group consisting of halogen, halogenated C₁₋₆ alkyl, and C₁₋₆ alkyl;
with the proviso that when A is selected from a group consisting of C₆₋₈ aryl and 5-8 membered heteroaryl, n is not equal to 0, at least one R₁ is selected from a group consisting of cyano, C₃₋₆ cycloalkyl, 3-6 membered heterocyclyl, halogenated C₃₋₆ cycloalkyl, halogenated 3-6 membered heterocyclyl, halogenated C₁₋₆ alkoxy, C₁₋₆ alkyl-substituted C₃₋₆ cycloalkyl, and C₁₋₆ alkyl-substituted 3-6 membered heterocyclyl;
or when A is selected from a group consisting of C₆₋₈ aryl and 5-8 membered heteroaryl, n is not equal to 0, and R₁ is only selected from halogen and/or C₁₋₆ alkyl and/or halogenated C₁₋₆ alkyl, and R₃ is selected from a group consisting of C₆₋₈ aryl and 5-6 membered heteroaryl, R₃ is substituted with at least one substituent selected from a group consisting of cyano, -NR₁₂R₁₃, - C(O)NR₁₂R₁₃, and -N(R₁₂)-C(O)R₁₁, wherein in said -NR₁₂R₁₃ and -C(O)NR₁₂R₁₃, R₁₂ or R₁₃ is C₃₋₁₂ cycloalkyl, wherein in said -N(R₁₂)-C(O)R₁₁, R₁₁ or R₁₂ is C₃₋₆ cycloalkyl, wherein said C₃₋₆ cycloalkyl is independently optionally further substituted with one or more substituents selected from a group consisting of deuterium, halogen, halogenated C₁₋₆ alkyl, and C₁₋₆ alkyl.

3. The compound of any one of claims 1-2, stereoisomer, tautomer, or pharmaceutically acceptable salt thereof, wherein ring A is selected from a group consisting of phenyl, pyridinyl, and pyrimidinyl; R₂ is selected from a group consisting of methyl, ethyl, and isopropyl; R₄ and R₅ together with a carbon atom to which they are directly attached form a cyclopropyl or cyclobutyl, wherein said cyclopropyl or cyclobutyl is optionally further substituted with one or more substituents selected from a group consisting of methyl, ethyl, and isopropyl;
R₆ is

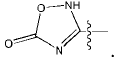

4. The compound of any one of claims 1-3, stereoisomer, tautomer, or pharmaceutically acceptable salt thereof, wherein the compound has the following formula: 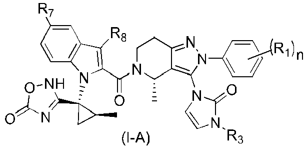
wherein R₃ is selected from a group consisting of 5-10 membered heteroaryl and C₆₋₁₀ aryl, wherein above groups are optionally further substituted with one or more substituents selected from a group consisting of halogen, cyano, nitro, hydroxyl, C₁₋₆ alkyl, halogenated C₁₋₆ alkyl, C₃₋₆ cycloalkyl, C₁₋₆ alkoxy, halogenated C₁₋₆ alkoxy, -S(O)ᵣR₉, -C(O)NR₁₂R₁₃, -N(R₁₂)-C(O)R₁₁, and -NR₁₂R₁₃;
R₁₁, R₁₂, and R₁₃ are each independently selected from a group consisting of hydrogen, deuterium, C₁₋₆ alkyl, and C₃₋₆ cycloalkyl, wherein said C₃₋₆ cycloalkyl is optionally further substituted with one or more substituents selected from a group consisting of halogen, halogenated C₁₋₆ alkyl, and C₁₋₆ alkyl;
n is 1, 2, 3, or 4;
R₁, R₇, and R₈ are as defined in any one of claims 1-3.

5. The compound of any one of claims 1-4, stereoisomer, tautomer, or pharmaceutically acceptable salt thereof, wherein R₃ is selected from phenyl, pyridinyl, pyrimidinyl, pyrazinyl, pyridazinyl, or the following groups: 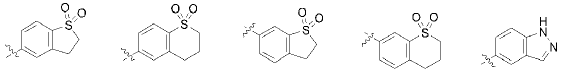 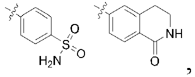
wherein above groups are optionally further substituted with one or more substituents selected from a group consisting of cyano, nitro, hydroxyl, halogen, C₁₋₆ alkyl, halogenated C₁₋₆ alkyl, C₃₋₆ cycloalkyl, C₁₋₆ alkoxy, halogenated C₁₋₆ alkoxy, -C(O)NR₁₂R₁₃, -N(R₁₂)-C(O)R₁₁, and -NR₁₂R₁₃;
R₁₁, R₁₂, and R₁₃ are as defined in any one of claims 1-4.

6. The compound of any one of claims 1-5, stereoisomer, tautomer, or pharmaceutically acceptable salt thereof, wherein the compound has the following formula: 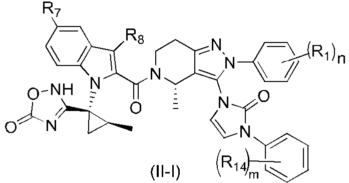 wherein n is 1, 2, 3, or 4;
m is 0, 1, 2, 3, 4, 5, or 6;
R₁₄ is selected from a group consisting of hydrogen, cyano, hydroxyl, nitro, halogen, C₁₋₆ alkyl, halogenated C₁₋₆ alkyl, C₃₋₆ cycloalkyl, C₁₋₆ alkoxy, halogenated C₁₋₆ alkoxy, -C(O)NR₁₂R₁₃, -N(R₁₂)-C(O)R₁₁, and -NR₁₂R₁₃;
R₁₁, R₁₂, and R₁₃ are each independently selected from a group consisting of hydrogen, deuterium, C₁₋₆ alkyl, and C₃₋₆ cycloalkyl, wherein said C₃₋₆ cycloalkyl is optionally further substituted with one or more substituents selected from a group consisting of halogen, halogenated C₁₋₆ alkyl, and C₁₋₆ alkyl;
R₁, R₇, and R₈ are as defined in any one of claims 1-5.

7. The compound of any one of claims 1-6, stereoisomer, tautomer, or pharmaceutically acceptable salt thereof, wherein \R₇ is 5-6 membered heterocyclyl, wherein said 5-6 membered heterocyclyl is optionally substituted with one or more substituents selected from a group consisting of halogen, hydroxyl, cyano, nitro, C₁₋₆ alkyl, and halogenated C₁₋₆ alkyl, or any two substituents on the same carbon atom of said 5-6 membered heterocyclyl together with a carbon atom to which they are attached form a C₃₋₆ cycloalkyl, wherein said C₃₋₆ cycloalkyl is optionally further substituted with one or more substituents selected from a group consisting of halogen, cyano, C₁₋₆ alkyl, halogenated C₁₋₆ alkyl, and C₁₋₆ alkoxy.

8. The compound of any one of claims 1-7, stereoisomer, tautomer, or pharmaceutically acceptable salt thereof, wherein R₇ is tetrahydropyranyl, wherein said tetrahydropyranyl is optionally substituted with one or more substituents selected from a group consisting of methyl, ethyl, isopropyl, fluoromethyl, difluoromethyl, and trifluoromethyl, or any two substituents on the same carbon atom of said tetrahydropyranyl together with a carbon atom to which they are attached form a cyclopropyl, cyclobutyl, or cyclopentyl, wherein the formed ring is optionally further substituted with one or more substituents selected from a group consisting of fluoro, chloro, bromo, cyano, methyl, ethyl, isopropyl, fluoromethyl, difluoromethyl, trifluoromethyl, methoxy, and ethoxy.

9. The compound of claim 6, stereoisomer, tautomer, or pharmaceutically acceptable salt thereof, wherein R₁₄ is selected from a group consisting of hydrogen, cyano, nitro, hydroxyl, fluoro, chloro, bromo, methyl, ethyl, propyl, isopropyl, fluoromethyl, difluoromethyl, trifluoromethyl, methoxy, ethoxy, isopropoxy, fluoromethoxy, difluoromethoxy, trifluoromethoxy, fluoroethoxy, difluoroethoxy, trifluoroethoxy, cyclopropyl, cyclobutyl, cyclopentyl, cyclohexyl, -C(O)NR₁₂R₁₃, -N(R₁₂)-C(O)R₁₁, and -NR₁₂R₁₃;
R₁₁, R₁₂, and R₁₃ are each independently selected from a group consisting of hydrogen, deuterium, methyl, ethyl, propyl, cyclopropyl, cyclobutyl, cyclopentyl, and bicyclo[1.1.1]pentanyl, wherein above groups are optionally substituted with one or more substituents selected from a group consisting of fluoro, chloro, bromo, fluoromethyl, difluoromethyl, trifluoromethyl, methyl, ethyl, propyl, and isopropyl;
or R₁₄ is selected from a group consisting of the following groups:

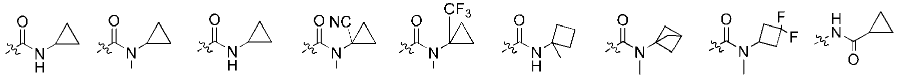

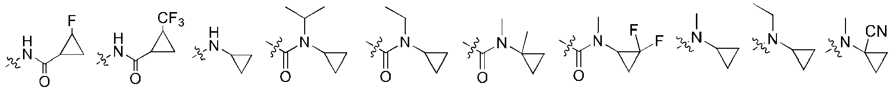

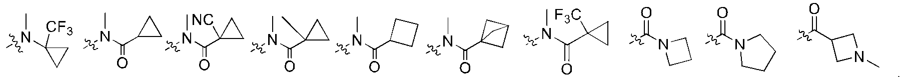

10. The compound of any one of claims 1-9, stereoisomer, tautomer, or pharmaceutically acceptable salt thereof, wherein the compound is selected from: 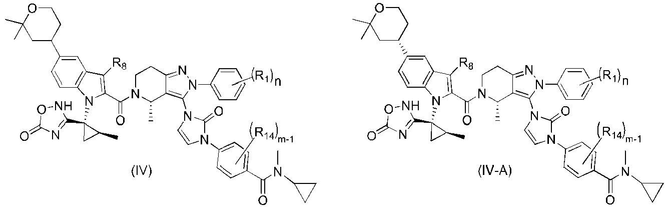
wherein R₁₄ is selected from a group consisting of hydrogen, cyano, hydroxyl, nitro, halogen, C₁₋₆ alkyl, halogenated C₁₋₆ alkyl, C₃₋₆ cycloalkyl, C₁₋₆ alkoxy, halogenated C₁₋₆ alkoxy, - C(O)NR₁₂R₁₃, -N(R₁₂)-C(O)R₁₁, and -NR₁₂R₁₃;
m is 1, 2, 3, or 4;
n is 1, 2, 3, or 4;
R₁ and R₈ are as defined in any one of claims 1-9.

11. The compound of any one of claims 1-10, stereoisomer, tautomer, or pharmaceutically acceptable salt thereof, wherein R₁ is selected from a group consisting of cyano, halogen, C₁₋₆ alkyl, halogenated C₁₋₆ alkyl, C₃₋₆ cycloalkyl, 3-6 membered heterocyclyl, halogenated C₃₋₆ cycloalkyl, halogenated 3-6 membered heterocyclyl, halogenated C₁₋₆ alkoxy, halogenated C₁₋₆ alkylthio, C₁₋₆ alkyl-substituted C₃₋₆ cycloalkyl, and C₁₋₆ alkyl-substituted 3-6 membered heterocyclyl;
R₈ is selected from a group consisting of hydrogen, cyano, C₃₋₆ cycloalkyl, 3-6 membered heterocyclyl, C₁₋₆ alkyl, halogenated C₁₋₆ alkyl, halogenated C₁₋₆ alkoxy, and -NR₁₂R₁₃.

12. The compound of any one of claims 1-11, stereoisomer, tautomer, or pharmaceutically acceptable salt thereof, wherein R₁ is selected from a group consisting of cyano, fluoro, chloro, bromo, methyl, ethyl, isopropyl, fluoromethyl, difluoromethyl, trifluoromethyl, fluoromethoxy, difluoromethoxy, trifluoromethoxy, and trifluoromethylthio, or is selected from a group consisting of the following groups: 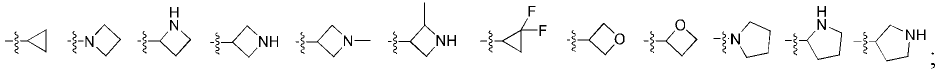 R₈ is selected from a group consisting of hydrogen, cyano, amino, cyclopropyl, cyclobutyl, cyclopentyl, methyl, ethyl, isopropyl, fluoromethyl, difluoromethyl, trifluoromethyl, fluoromethoxy, difluoromethoxy, trifluoromethoxy, trifluoromethylthio, azetidinyl, and methylamino.

13. The compound of any one of claims 1-12, stereoisomer, tautomer, or pharmaceutically acceptable salt thereof, wherein the compound is selected from: 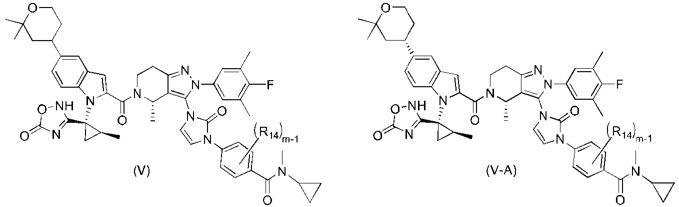
wherein R₁₄ is selected from a group consisting of hydrogen, cyano, hydroxyl, nitro, halogen, C₁₋₆ alkyl, halogenated C₁₋₆ alkyl, C₃₋₆ cycloalkyl, C₁₋₆ alkoxy, halogenated C₁₋₆ alkoxy, - C(O)NR₁₂R₁₃, -N(R₁₂)-C(O)R₁₁, and -NR₁₂R₁₃;
m is 1, 2, 3, or 4.

14. The compound of any one of claims 10-13, stereoisomer, tautomer, or pharmaceutically acceptable salt thereof, wherein R₁₄ is selected from a group consisting of hydrogen, cyano, nitro, hydroxyl, fluoro, chloro, bromo, methyl, ethyl, propyl, isopropyl, fluoromethyl, difluoromethyl, trifluoromethyl, methoxy, ethoxy, isopropoxy, fluoromethoxy, difluoromethoxy, trifluoromethoxy, fluoroethoxy, difluoroethoxy, trifluoroethoxy, cyclopropyl, cyclobutyl, cyclopentyl, cyclohexyl, - C(0)NR₁₂R₁₃, -N(R₁₂)-C(O)R₁₁, and -NR₁₂R₁₃, wherein R₁₁, R₁₂, and R₁₃ are each independently selected from a group consisting of hydrogen, deuterium, methyl, ethyl, propyl, cyclopropyl, cyclobutyl, cyclopentyl, and bicyclo[1.1.1]pentanyl, wherein above groups are optionally substituted with one or more substituents selected from a group consisting of fluoro, chloro, bromo, fluoromethyl, difluoromethyl, trifluoromethyl, methyl, ethyl, propyl, and isopropyl;
preferably, R₁₄ is selected from a group consisting of hydrogen, cyano, fluoro, chloro, bromo, C₁₋₆ alkyl, halogenated C₁₋₆ alkyl, and halogenated C₁₋₆ alkoxy;
more preferably, R₁₄ is selected from a group consisting of hydrogen, cyano, fluoro, chloro, methyl, ethyl, propyl, trifluoromethyl, and trifluoromethoxy.

15. The compound of any one of claims 1-14, stereoisomer, tautomer, or pharmaceutically acceptable salt thereof, wherein the compound is selected from a group consisting of the following compounds:

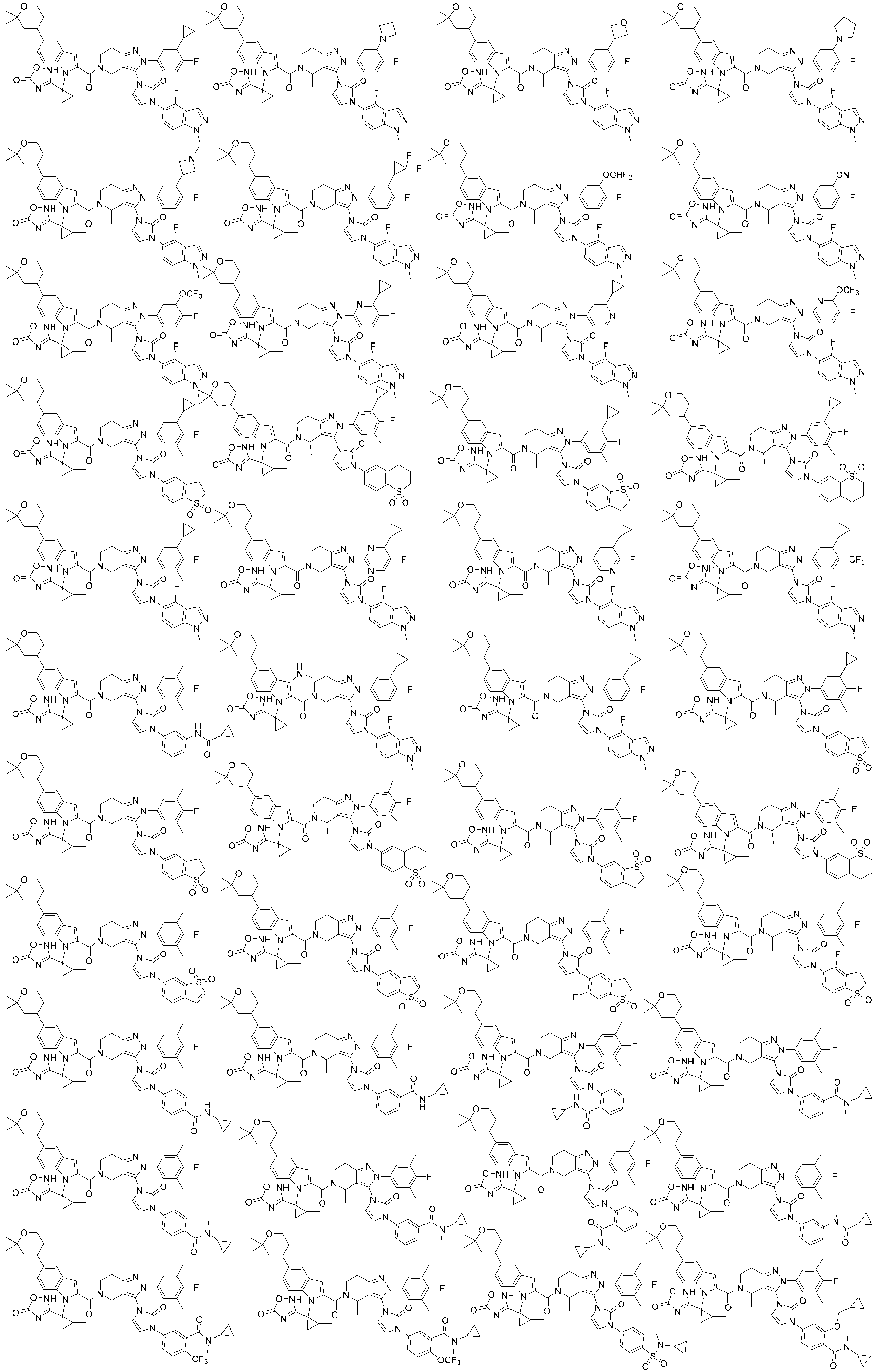

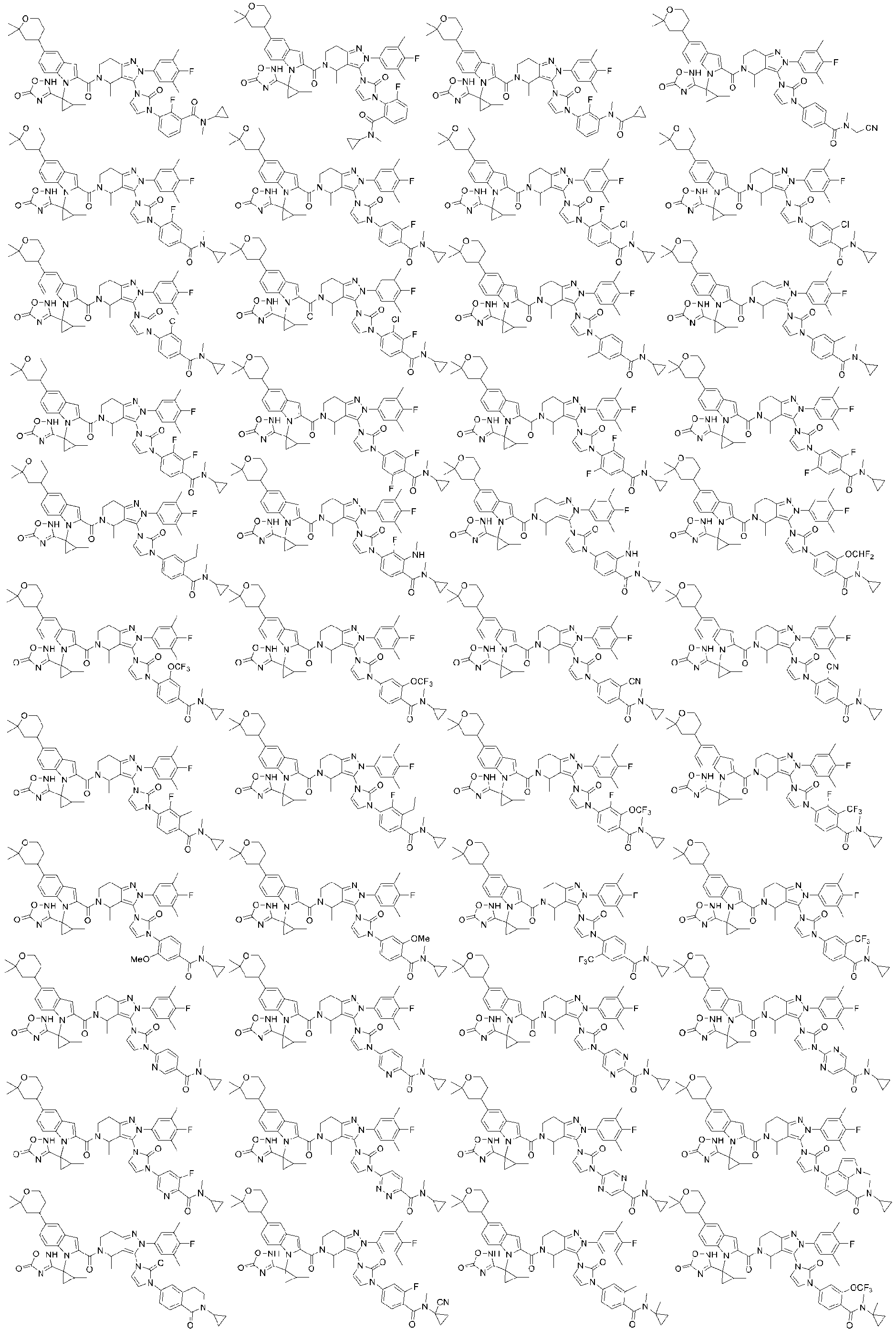

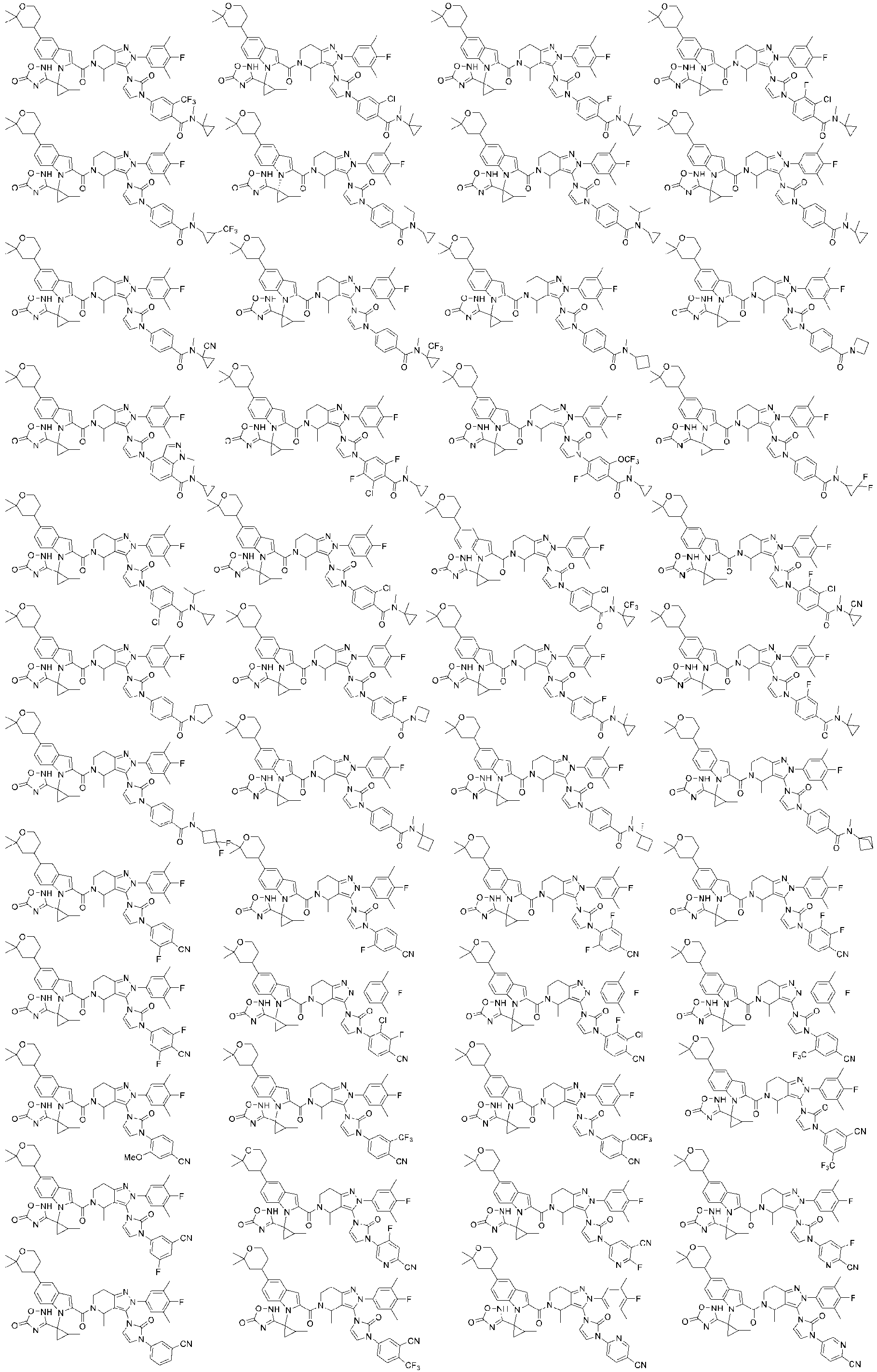

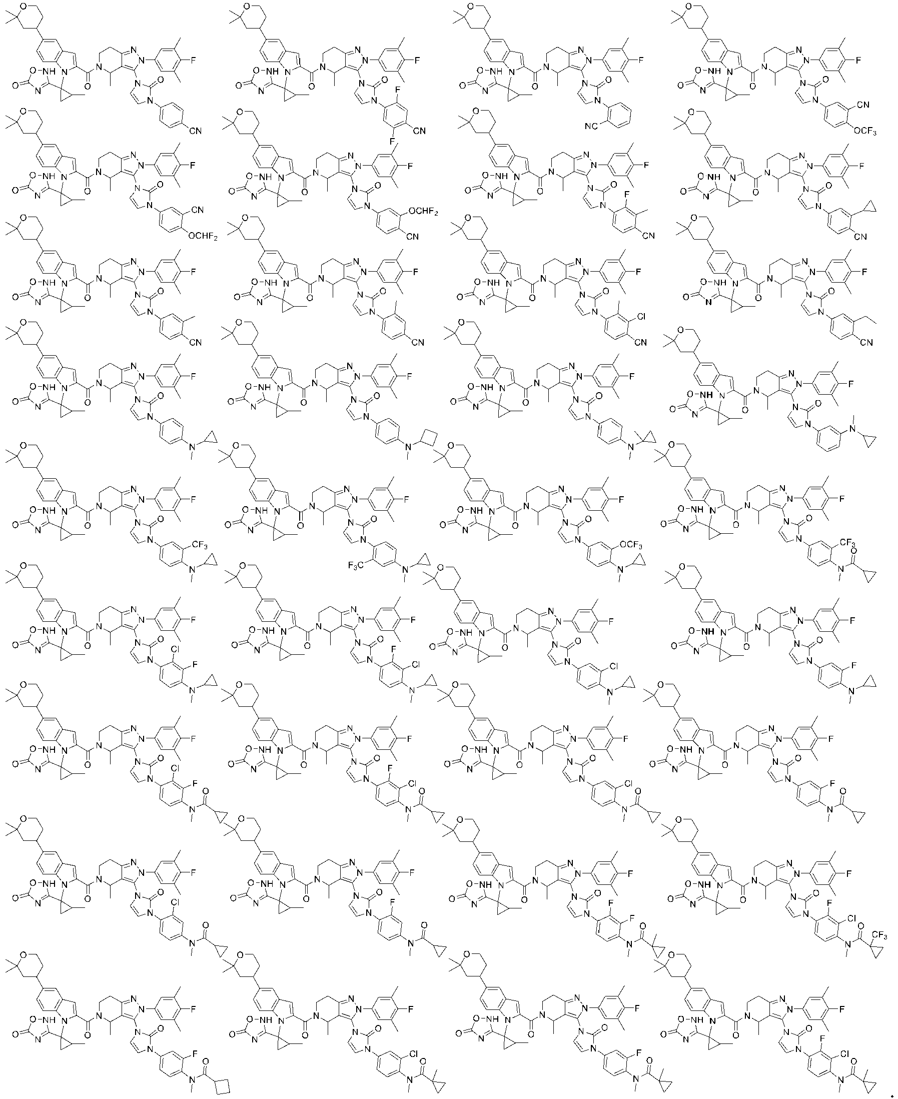

16. The compound of any one of claims 1-15, stereoisomer, tautomer, or a pharmaceutically acceptable salt thereof, wherein the compound is selected from a group consisting of the following compounds:

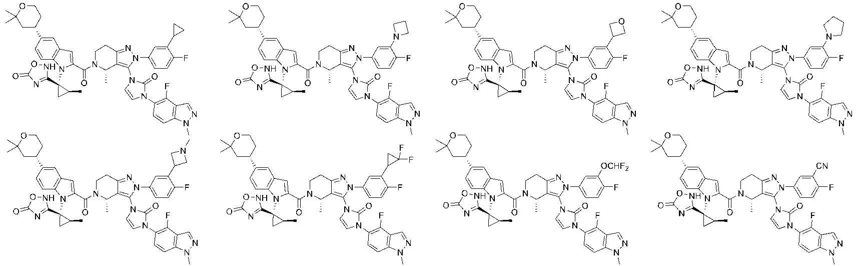

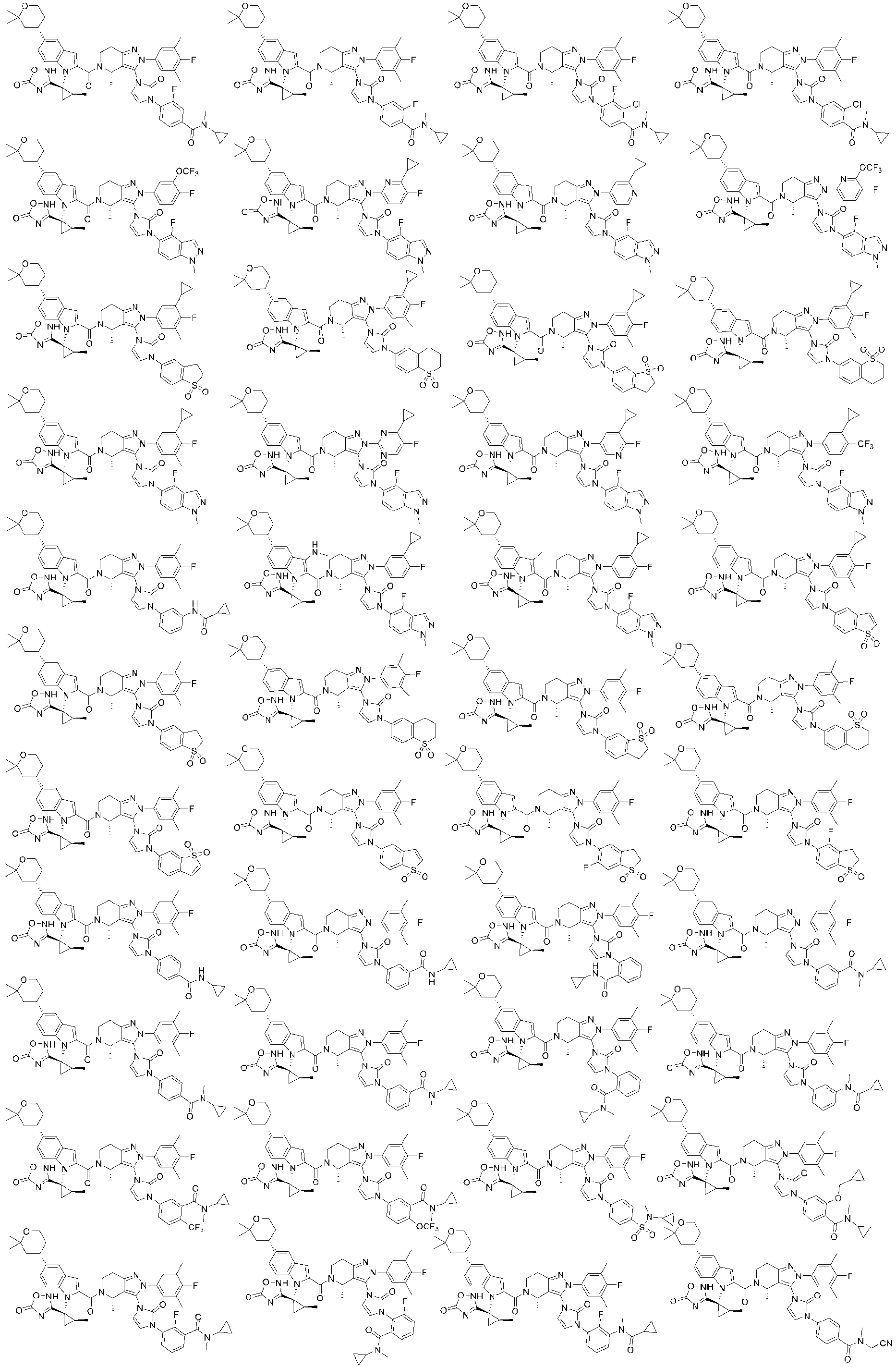

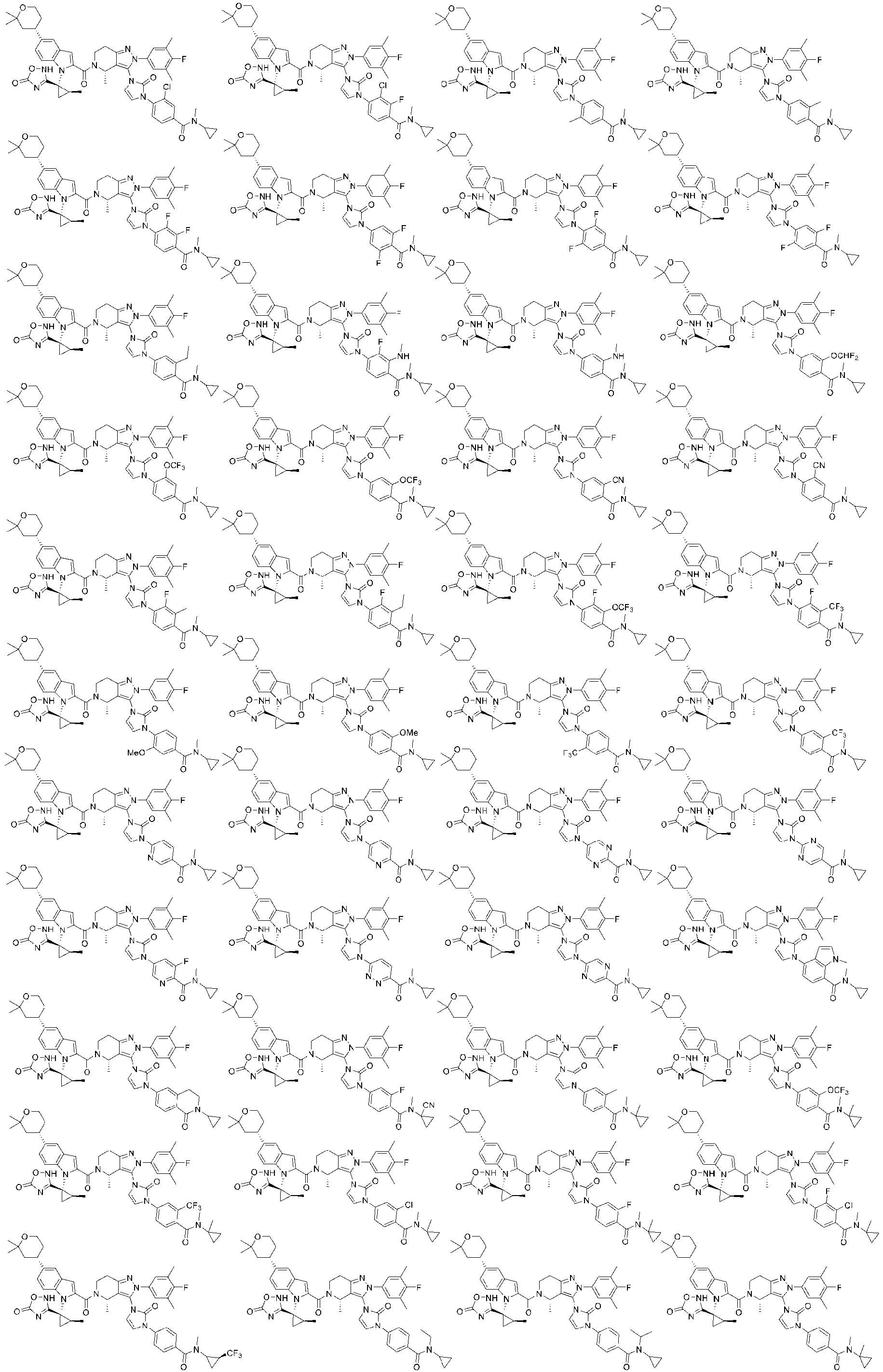

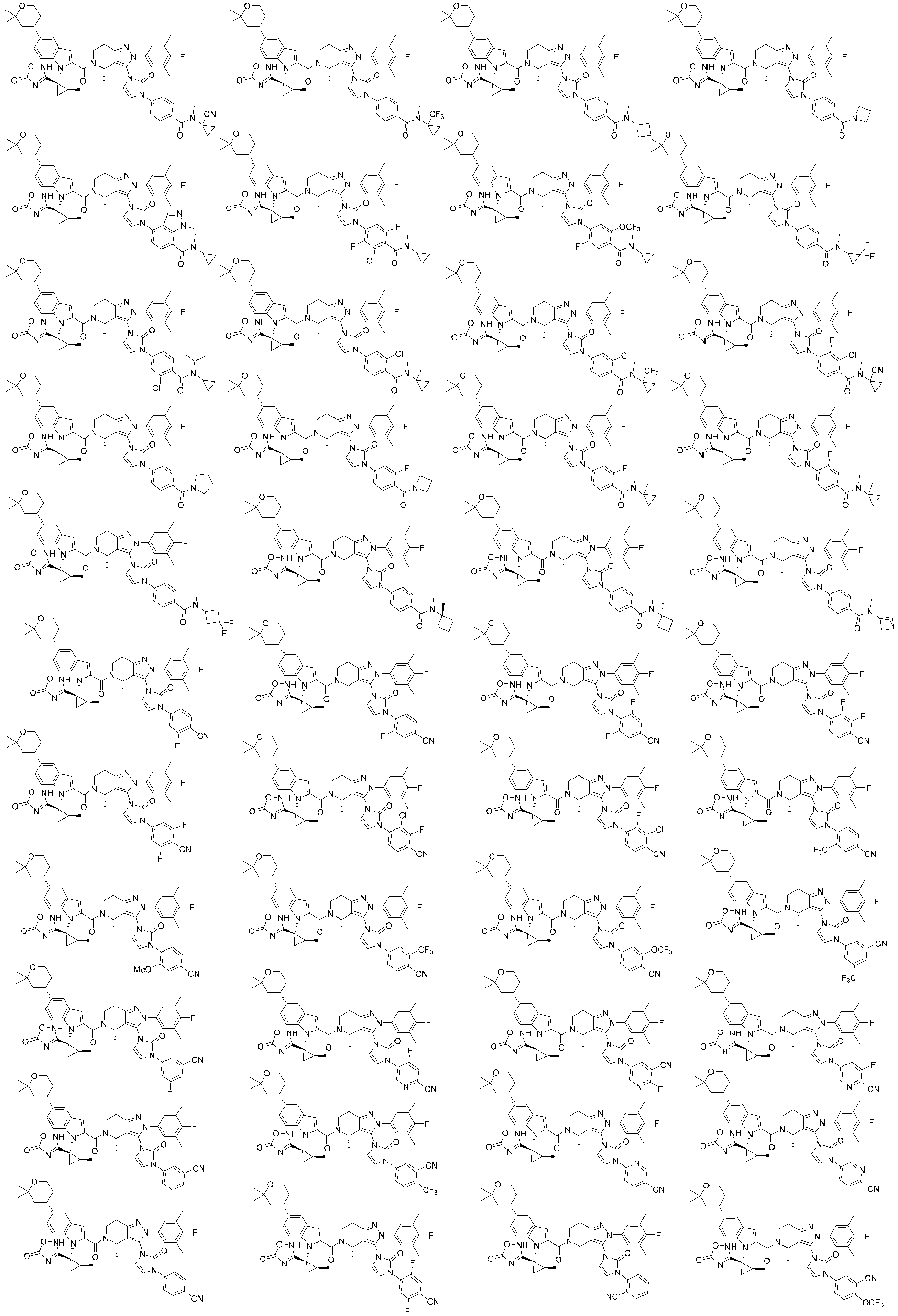

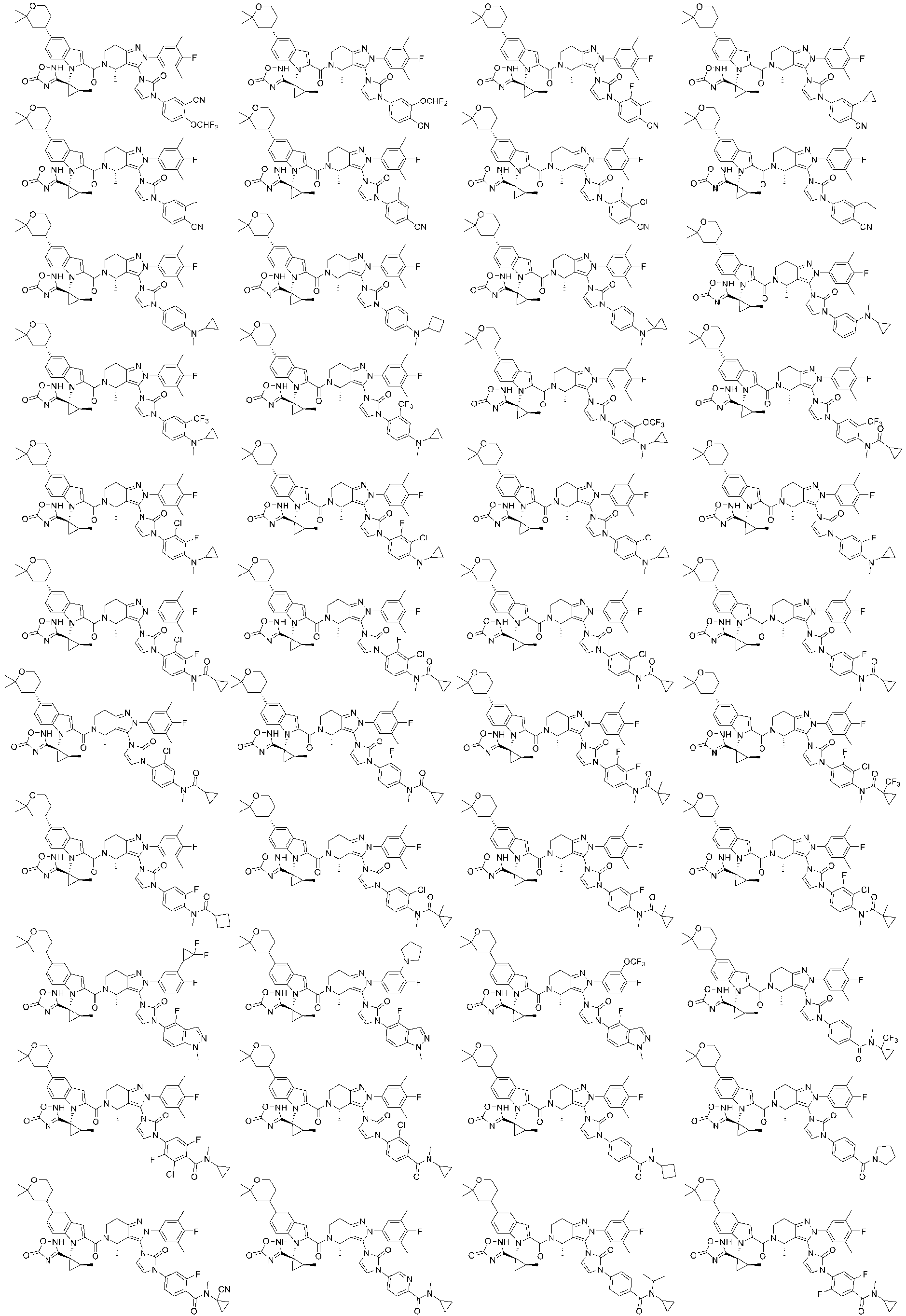

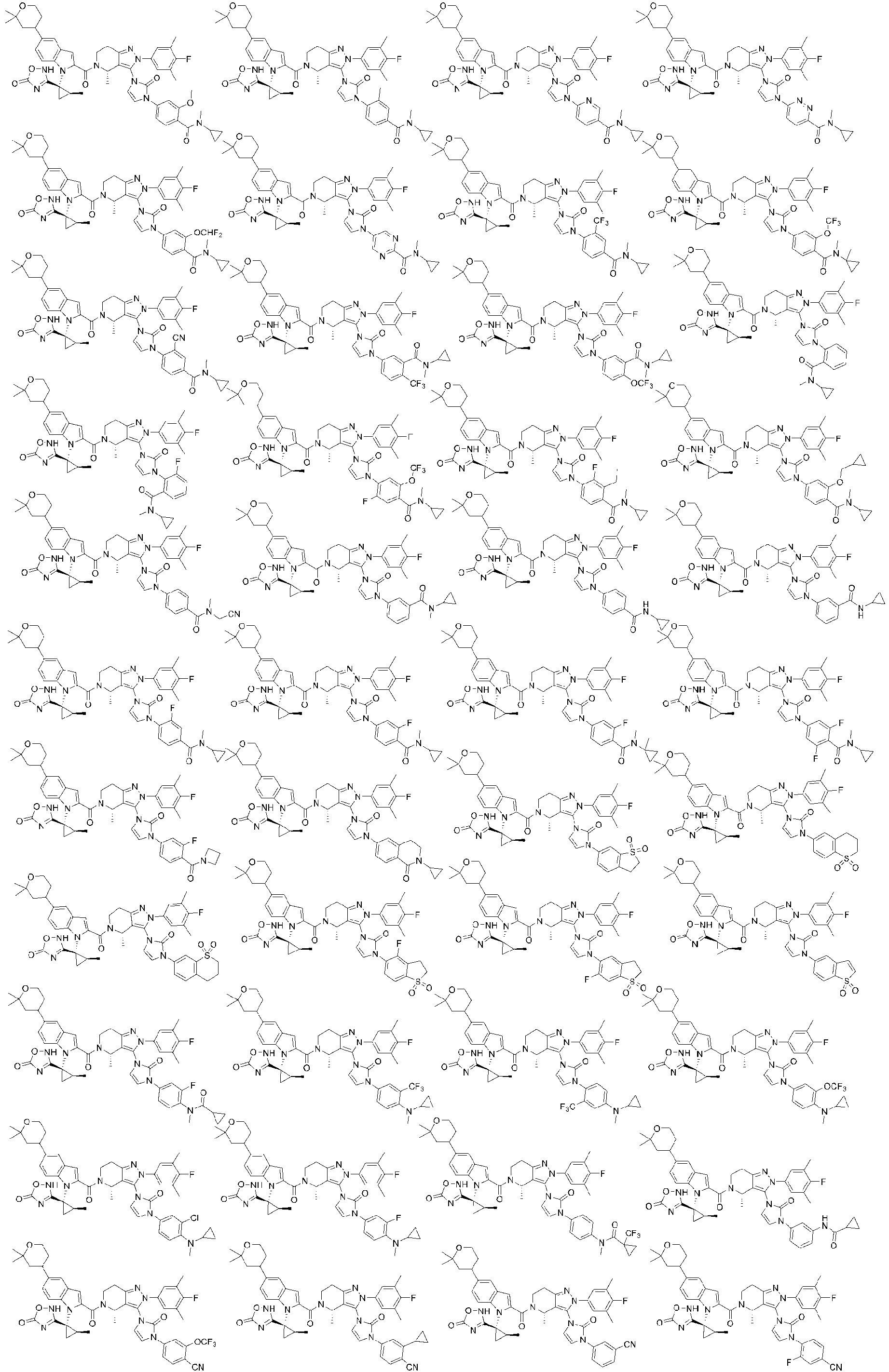

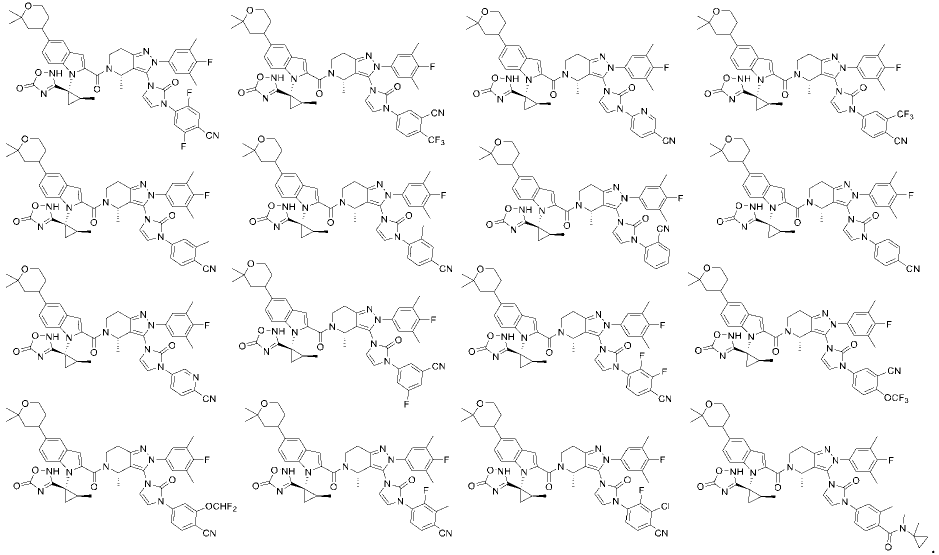

17. A method of preparing the compound of any one of claims 1-16, stereoisomer, tautomer, or pharmaceutically acceptable salt thereof, comprising: 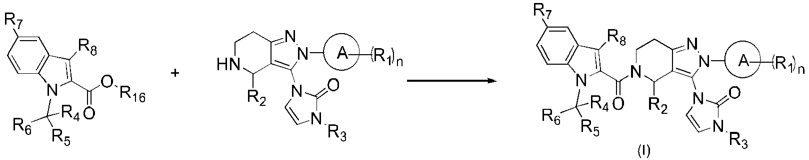 wherein R₁₆ is H;
R₁, R₂, R₃, R₄, R₅, R₆, R₇, R₈, R₉, and n are as defined in any one of claims 1-16.

18. A pharmaceutical composition comprising the compound of any one of claims 1-16, stereoisomer, tautomer, or pharmaceutically acceptable salt thereof, and a pharmaceutically acceptable carrier.

19. Use of the compound of any one of claims 1-16, stereoisomer, tautomer, or pharmaceutically acceptable salt thereof, or the pharmaceutical composition of claim 18 in the preparation of a medicament for treating and/or preventing a disease mediated by a GLP-1 receptor agonist.

20. Use of the compound of any one of claims 1-16, stereoisomer, tautomer, or pharmaceutically acceptable salt thereof, or the pharmaceutical composition of claim 18 in the preparation of a medicament for preventing and/or treating diabetes, hyperglycemia, insulin resistance, glucose intolerance, diabetic nephropathy, diabetic neuropathy, diabetic retinopathy, adipocyte dysfunction, obesity, dyslipidemia, and hyperinsulinemia.
